# EUROPEAN PATENT SPECIFICATION

(11) **EP 2 998 408 B1**
(45) Date of publication and mention of the grant of the patent: **08.01.2020**
(21) Application number: 14797267.3
(22) Date of filing: 19.05.2014
(51) Int. Cl.: C12Q 1/68, C12M 1/00, C12N 15/09

(54) **METHOD FOR OBTAINING EPIGENETIC CELL INFORMATION, METHOD FOR DETERMINING CELL CHARACTERISTICS, METHOD FOR ASSESSING DRUG SENSITIVITY OR SELECTING DRUG OR IMMUNOTHERAPEUTIC AGENT VARIETY, DISEASE DIAGNOSIS METHOD, SELF-REPLICATING VECTOR, ASSAY KIT, AND ANALYSIS DEVICE**
VERFAHREN ZUR GEWINNUNG EPIGENETISCHER ZELLINFORMATIONEN, VERFAHREN ZUR BESTIMMUNG VON ZELLEIGENSCHAFTEN, VERFAHREN ZUR BEURTEILUNG EINER ARZNEIMITTELEMPFINDLICHKEIT ODER ZUR AUSWAHL VON VARIANTEN VON ARZNEIMITTELN ODER IMMUNTHERAPEUTISCHEN MITTELN, KRANKHEITSDIAGNOSEVERFAHREN, SELBSTREPLIZIERENDE VEKTOREN, TESTSATZ UND ANALYSEVORRICHTUNG
PROCÉDÉ D'OBTENTION D'INFORMATIONS DE CELLULE ÉPI-GÉNÉTIQUE, PROCÉDÉ DE DÉTERMINATION DES CARACTÉRISTIQUES CELLULAIRES, PROCÉDÉ D'ÉVALUATION DE LA SENSIBILITÉ À UN MÉDICAMENT OU DE SÉLECTION D'UNE VARIÉTÉ DE MÉDICAMENTS OU D'AGENTS IMMUNOTHÉRAPEUTIQUES, MÉTHODE DE DIAGNOSTIC DE MALADIE, VECTEUR POUVANT SE RÉPLIQUER, NÉCESSAIRE DE DOSAGE ET DISPOSITIF D'ANALYSE

(30) Priority: 17.05.2013 JP 2013105481
(43) Date of publication of application: 23.03.2016
(73) Proprietor: Toshiba Medical Systems Corporation, Otawara-shi, Tochigi 324-8550 (JP)
(72) Inventor: YOSHIMURA, Seiko, Tokyo 105-8001 (JP); ISHIHARA, Mitsuko, Tokyo 105-8001 (JP); AKAHOSHI, Eiichi, Tokyo 105-8001 (JP); SAKURAI, Yasuo, Otawara-shi Tochigi 324-8550 (JP)
(74) Representative: Moreland, David
(86) International application number: PCT/JP2014/063214
(87) International publication number: WO 2014/185549

(56) References cited:
- JP-A- 2013 042 721
- MAUBACH G ET AL: "GFAP promoter directs lacZ expression specifically in a rat hepatic stellate cell line", WORLD JOURNAL OF GASTROENTEROLOGY, WJG PRESS, CN, vol. 12, no. 5, 7 February 2006 (2006-02-07), pages 723-730, XP003005519, ISSN: 1007-9327
- Invitrogen: "pcDNA (TM) 4/TO Expression vector designed for use with the T-REx (TM) System", , 16 December 2011 (2011-12-16), pages 1-21, XP055323865, Retrieved from the Internet: URL:https://tools.thermofisher.com/content /sfs/manuals/pcdna4to_man.pdf [retrieved on 2016-11-29]
- Clontech: "pMetLuc2-Reporter Vector Information", , 3 September 2010 (2010-09-03), XP055323597, Retrieved from the Internet: URL:http://www.clontech.com/xxclt_ibcGetAt tachment.jsp?cItemId=45226 [retrieved on 2016-11-28]
- KHURSHEED IQBAL ET AL: "Cytoplasmic injection of circular plasmids allows targeted expression in mammalian embryos", BIOTECHNIQUES RAPID DISPATCHES, vol. 47, no. 5, 1 November 2009 (2009-11-01), pages 959-968, XP055325005, US ISSN: 0736-6205, DOI: 10.2144/000113270
- BARRETO G. ET AL.: 'Gadd45a promotes epigenetic gene activation by repair-mediated DNA demethylation' NATURE vol. 445, no. 7128, 2007, pages 671 - 675, XP002571281
- MAJUMDER S. ET AL.: 'Role of DNA methyltransferases in regulation of human ribosomal RNA gene transcription' J. BIOL. CHEM. vol. 281, no. 31, 2006, pages 22062 - 22072, XP055295386
- HASSE A. ET AL.: 'De novo methylation of transfected CAT gene plasmid constructs in F9 mouse embryonal carcinoma cells' BIOCHIM. BIOPHYS. ACTA vol. 1131, no. 1, 1992, pages 16 - 22, XP023467595
- WISCHNEWSKI F. ET AL.: 'Promoter demethylation and histone acetylation mediate gene expression of MAGE-A1, -A2, -A3, and -A12 in human cancer cells' MOL. CANCER RES. vol. 4, no. 5, 2006, pages 339 - 349, XP055295385
- TAMURA Y. ET AL.: 'Epigenetic aberration of the human REELIN gene in psychiatric disorders' MOL. PSYCHIATRY vol. 12, no. 6, 2007, pages 593 - 600, XP055295384
- MAHON M.: 'Vectors bicistronically linking a gene of interest to the SV 40 large T antigen in combination with the SV 40 origin of replication enhance transient protein expression and luciferase reporter activity' BIOTECHNIQUES vol. 51, no. 2, 2011, pages 119 - 126, XP055295383
- HSIEH C.: 'Evidence that protein binding specifies sites of DNA demethylation' MOL. CELL . BIOL. vol. 19, no. 1, 1999, pages 46 - 56, XP055295382
- SHERF B. ET AL.: 'Dual-luciferase reporter assay: an advanced co-reporter technology integrating firefly and Renilla luciferase assays' PROMEGA NOTES MAGAZINE vol. 57, 1996, page 02, XP002937069
- COLLAS P.: 'Modulation of plasmid DNA methylation and expression in zebrafish embryos' NUCLEIC ACIDS RES. vol. 26, no. 19, 1998, pages 4454 - 4461, XP055295381
- ESCHER G.: 'Demethylation using the epigenetic modifier, 5-azacytidine, increases the efficiency of transient transfection of macrophages' J. LIPID RES. vol. 46, no. 2, 2005, pages 356 - 365, XP002392684
- ESCHER GENEVIIVE ET AL: "Demethylation using the epigenetic modifier, 5-azacytidine, increases the efficiency of transient transfection of macrophages", JOURNAL OF LIPID RESEARCH, AMERICAN SOCIETY FOR BIOCHEMISTRY AND MOLECULAR BIOLOGY, INC, US, vol. 46, no. 2, 1 November 2004 (2004-11-01), pages 356-365, XP002392684, ISSN: 0022-2275, DOI: 10.1194/JLR.D400014-JLR200
- Emma M. Smith ET AL: "The potential role of epigenetic therapy in multiple myeloma", British Journal of Haematology, vol. 148, no. 5, 1 March 2010 (2010-03-01) , pages 702-713, XP055051410, ISSN: 0007-1048, DOI: 10.1111/j.1365-2141.2009.07976.x

### SEQUENCE LISTING

<110> Kabushiki Kaisha Toshiba
<120> Method of obtaining epigenetic information of cell, method of determining cell property, method of evaluating drug susceptibility or selecting type of drug or immunotherapeutic agent, method of diagnosing disorder and self-replication vector, assay kit and analyzing device
<130> 14S0091PCT
<150> JP 2013-105481
   <151> 2013/05/17
<160> 31
<170> PatentIn version 3.4
<210> 1
   <211> 1683
   <212> DNA
   <213> murine
<400> 1
   gtctgtaagc tgaagacctg gcagtgctga gctggtcacc ccccaggacc tccttttgtg      60
   cccaacgagt gactcacctt ggcatagaca taatggtcag gggtgggcac gcagcctgct     120
   tcccgctgtg ctccaggcct ccttcgatgc tttccgagaa gtctattgag ctgggagctt     180
   gtactgcacc cggggctgac atcctggcat cctgggataa aagcagccca cggggctgcc     240
   cttgccatat gcctcactgg cggcagagga caaggctcta ttcagcaagt gccctggagt     300
   agacaccaga agcccaagca tgggcagagg aaggcagggg ttggggggag cagagctgtc     360
   tgtgttccag aagcccaagg acacagatgg ctaaggcgcc tgggagggac ctgagtggaa     420
   gagatagatg ggcctgaagt ctcaagcagc aacagcctcc tccccgccat tggtgagggt     480
   ggggtttggt ttcccggacc tacatatccc tcagaggcct ggtgtgtagg aatttaaagg     540
   aggtaaatct cctgagagaa tgaggggtac ccaggaagac ggggtgttac agaaagactc     600
   cagcatgcac agccaactca ctcaaaacta ctctgtcagg ggctgccggg ggccaggctc     660
   ggggtggggg gtgggggggc aaagagaagc tggaccaggg agaaatggcc cactaggctg     720
   gatatgaggc cacagagggg ctcaggaatg aagcctgctg tcttacccta ttaggatctg     780
   cgtgcatacc ttctgctgtg cactctaaac acacagccag aggctcaagt tgaccctgga     840
   gtcacagaga gggctccaac cttagccctc cactcctgaa ctccaggaat gagaagatag     900
   agttggagcg attcagggga gaggactctg ttgagaatgg gggccacagg aaactgtaat     960
   ataggttgat cccggaggaa gggaataggt tcttcaagtt cctagcatct cacaggcccc    1020
   cagagaagga cagagttggg gtggtcctgg cttacaggct ctaagaactg gaagctgatt    1080
   accccaccaa gctgtgcact ctctgtctct gtctctgtct ctgtgtgtgc gcgctcgtgc 1140
   acacttatca cacaaatgtt catgtgtgtg cacatagatg agttgagacc agaggtcaac 1200
   ctcaggcact gttgccttgg ttttctgaga gagcatttct ctctggacct ggaactcgcc 1260
   aattagtgag agccaggaag tctgctgatt ttcactgccc agcactggag tttacaagta 1320
   tgcactgtca acccaggcct tttgtattca ttctgcagct agaacttggg tgggtcttca 1380
   tgcttgacag gcaagcaatt tatggactaa gctgtttcct cggccctctc ttgacccatt 1440
   taccagaaag ggggttcctt gatcaatggc gaagccaggc tggtgttccc aagaaagcct 1500
   tgactctggg tacagtgacc tcagtggggt gagaggagtt ctccccttag ctgggctggg 1560
   gcccagcttc accccctcag gctattcagt gggggtgctt ccaggaagtc aggggcagat 1620
   ttagtccaac ccgttcctcc ataaaggccc tgacatccca ggagccagca gaggcagggc 1680
   agg 1683
<210> 2
   <211> 678
   <212> DNA
   <213> Homo Sapiens1
<400> 2
   gcctggtcaa catggtgaaa ccctgtctct actaaaaata caaaaattag ctgggcgtgg 60
   tggcgcgtgc ctgtaatccc agctactccg gaggctgagg caggagaatc gcttgaaccc 120
   gggagatgga ggctgcagtg ggccgagatc acaccactgc actccagtct gggcgacaga 180
   gactcgtctc caaaaaaaca aaaacaaaat cactgggtca ggggtgtgta ggaatatgac 240
   cccagaggga ctgtaattcc cagtggtgtc aaactctggg tgatcttaaa gggtgaggct 300
   cagaatgtgg cttccaggga caggggtgtc caggtatgtc cctgacgggg gaaaggccag 360
   aacagggtct gcagagagag agtgggggag tgcgggtcgg agcttctgcg cggaccgggg 420
   cggggcacct ctggagggca ggggcctctg gtctctggga ggggagggaa ttgaccaatg 480
   gggagagagc ccatatttgc tctcaggagc ctgcaaattc ctcagggctc agatatccgc 540
   ccctgacacc attcctccct tcccccctcc accggccgcg ggcataaaag gcgccaggtg 600
   agggcctcgc cgctcctccc gcgaatcgca gcttctgaga ccagggttgc tccgtccgtg 660
   ctccgcctcg ccctcgag 678
<210> 3
   <211> 655
   <212> DNA
   <213> Homo Sapiens1
<400> 3
   cttaacctta ctcgccccag tctgtcccga cgtgacttcc tcgaccctct aaagacgtac 60
   agaccagaca cggcggcggc ggcgggagag gggattccct gcgcccccgg acctcagggc 120
   cgctcagatt cctggagagg aagccaagtg tccttctgcc ctcccccggt atcccatcca 180
   aggcgatcag tccagaactg gctctcggaa gcgctcgggc aaagactgcg aagaagaaaa 240
   gacatctggc ggaaacctgt gcgcctgggg cggtggaact cggggaggag agggagggat 300
   cagacaggag agtggggact accccctctg ctcccaaatt ggggcagctt cctgggtttc 360
   cgattttctc atttccgtgg gtaaaaaacc ctgcccccac cgggcttacg caattttttt 420
   aaggggagag gagggaaaaa tttgtggggg gtacgaaaag gcggaaagaa acagtcattt 480
   cgtcacatgg gcttggtttt cagtcttata aaaaggaagg ttctctcggt tagcgaccaa 540
   ttgtcatacg acttgcagtg agcgtcagga gcacgtccag gaactcctca gcagcgcctc 600
   cttcagctcc acagccagac gccctcagac agcaaagcct acccccgcgc tcgag 655
<210> 4
   <211> 222
   <212> DNA
   <213> simian virus 40
<400> 4
   cagacatgat aagatacatt gatgagtttg gacaaaccac aactagaatg cagtgaaaaa 60
   aatgctttat ttgtgaaatt tgtgatgcta ttgctttatt tgtaaccatt ataagctgca 120
   ataaacaagt taacaacaac aattgcattc attttatgtt tcaggttcag ggggaggtgt 180
   gggaggtttt ttaaagcaag taaaacctct acaaatgtgg ta 222
<210> 5
   <211> 228
   <212> DNA
   <213> bovine
<400> 5
   gtttaaaccc gctgatcagc ctcgactgtg ccttctagtt gccagccatc tgttgtttgc 60
   ccctcccccg tgccttcctt gaccctggaa ggtgccactc ccactgtcct ttcctaataa 120
   aatgaggaaa ttgcatcgca ttgtctgagt aggtgtcatt ctattctggg gggtggggtg 180
   gggcaggaca gcaaggggga ggattgggaa gacaatagca ggcatgct 228
<210> 6
   <211> 165
   <212> DNA
   <213> simian virus 40
<400> 6
   gctagcaata aaatatcttt attttcatta catctgtgtg ttggtttttt gtgtgaatcg     60
   atagtactaa catacgctct ccatcaaaac aaaacgaaac aaaacaaact agcaaaatag    120
   gctgtcccca gtgcaagtgc aggtgccaga acatttctcg ctagc                    165
<210> 7
   <211> 2133
   <212> DNA
   <213> simian virus 40
<400> 7
   atggataaag ttttaaacag agaggaatct ttgcagctaa tggaccttct aggtcttgaa 60
   aggagtgcct gggggaatat tcctctgatg agaaaggcat atttaaaaaa atgcaaggag 120
   tttcatcctg ataaaggagg agatgaagaa aaaatgaaga aaatgaatac tctgtacaag 180
   aaaatggaag atggagtaaa atatgctcat caacctgact ttggaggctt ctgggatgca 240
   actgagattc caacctatgg aactgatgaa tgggagcagt ggtggaatgc ctttaatgag 300
   gaaaacctgt tttgctcaga agaaatgcca tctagtgatg atgaggctac tgctgactct 360
   caacattcta ctcctccaaa aaagaagaga aaggtagaag accccaagga ctttccttca 420
   gaattgctaa gttttttgag tcatgctgtg tttagtaata gaactcttgc ttgctttgct 480
   atttacacca caaaggaaaa agctgcactg ctatacaaga aaattatgga aaaatattct 540
   gtaaccttta taagtaggca taacagttat aatcataaca tactgttttt tcttactcca 600
   cacaggcata gagtgtctgc tattaataac tatgctcaaa aattgtgtac ctttagcttt 660
   ttaatttgta aaggggttaa taaggaatat ttgatgtata gtgccttgac tagagatcca 720
   ttttctgtta ttgaggaaag tttgccaggt gggttaaagg agcatgattt taatccagaa 780
   gaagcagagg aaactaaaca agtgtcctgg aagcttgtaa cagagtatgc aatggaaaca 840
   aaatgtgatg atgtgttgtt attgcttggg atgtacttgg aatttcagta cagttttgaa 900
   atgtgtttaa aatgtattaa aaaagaacag cccagccact ataagtacca tgaaaagcat 960
   tatgcaaatg ctgctatatt tgctgacagc aaaaaccaaa aaaccatatg ccaacaggct 1020
   gttgatactg ttttagctaa aaagcgggtt gatagcctac aattaactag agaacaaatg 1080
   ttaacaaaca gatttaatga tcttttggat aggatggata taatgtttgg ttctacaggc 1140
   tctgctgaca tagaagaatg gatggctgga gttgcttggc tacactgttt gttgcccaaa 1200
   atggattcag tggtgtatga ctttttaaaa tgcatggtgt acaacattcc taaaaaaaga 1260
   tactggctgt ttaaaggacc aattgatagt ggtaaaacta cattagcagc tgctttgctt 1320
   gaattatgtg gggggaaagc tttaaatgtt aatttgccct tggacaggct gaactttgag 1380
   ctaggagtag ctattgacca gtttttagta gtttttgagg atgtaaaggg cactggaggg 1440
   gagtccagag atttgccttc aggtcaggga attaataacc tggacaattt aagggattat 1500
   ttggatggca gtgttaaggt aaacttagaa aagaaacacc taaataaaag aactcaaata 1560
   tttccccctg gaatagtcac catgaatgag tacagtgtgc ctaaaacact gcaggccaga 1620
   tttgtaaaac aaatagattt taggcccaga gattatttaa agcattgcct ggaacgcagt 1680
   gagtttttgt tagaaaagag aataattcaa agtggcattg ctttgcttct tatgttaatt   1740
   tggtacagac ctgtggctga gtttgctcaa agtattcaga gcagaattgt ggagtggaaa   1800
   gagagattgg acaaagagtt tagtttgtca gtgtatcaaa aaatgaagtt taatgtggct   1860
   atgggaattg gagttttaga ttggctaaga aacagtgatg atgatgatga agacagccag   1920
   gaaaatgctg ataaaaatga agatggtggg gagaagaaca tggaagactc agggcatgaa   1980
   acaggcattg attcacagtc ccaaggctca tttcaggccc ctcagtcctc acagtctgtt   2040
   catgatcata atcagccata ccacatttgt agaggtttta cttgctttaa aaaacctccc   2100
   acacctcccc ctgaacctga acctgaaaca taa                                2133
<210> 8
   <211> 2133
   <212> DNA
   <213> simian virus 40
<400> 8
   atggataaag ttttaaacag agaggaatct ttgcagctaa tggaccttct aggtcttgaa      60
   aggagtgcct gggggaatat tcctctgatg agaaaggcat atttaaaaaa atgcaaggag     120
   tttcatcctg ataaaggagg agatgaagaa aaaatgaaga aaatgaatac tctgtacaag     180
   aaaatggaag atggagtaaa atatgctcat caacctgact ttggaggctt ctgggatgca     240
   actgagattc caacctatgg aactgatgaa tgggagcagt ggtggaatgc ctttaatgag     300
   gaaaacctgt tttgctcaga agaaatgcca tctagtgatg atgaggctac tgctgactct     360
   caacattcta ctcctccaaa aaagaagaga aaggtagaag accccaagga ctttccttca     420
   gaattgctaa gttttttgag tcatgctgtg tttagtaata gaactcttgc ttgctttgct     480
   atttacacca caaaggaaaa agctgcactg ctatacaaga aaattatgga aaaatattct     540
   gtaaccttta taagtaggca taacagttat aatcataaca tactgttttt tcttactcca     600
   cacaggcata gagtgtctgc tattaataac tatgctcaaa aattgtgtac ctttagcttt     660
   ttaatttgta aaggggttaa taaggaatat ttgatgtata gtgccttgac tagagatcca     720
   ttttctgtta ttgaggaaag tttgccaggt gggttaaagg agcatgattt taatccagaa     780
   gaagcagagg aaactaaaca agtgtcctgg aagcttgtaa cagagtatgc aatggaaaca     840
   aaatgtgatg atgtgttgtt attgcttggg atgtacttgg aatttcagta cagttttgaa     900
   atgtgtttaa aatgtattaa aaaagaacag cccagccact ataagtacca tgaaaagcat     960
   tatgcaaatg ctgctatatt tgctgacagc aaaaaccaaa aaaccatatg ccaacaggct    1020
   gttgatactg ttttagctaa aaagcgggtt gatagcctac aattaactag agaacaaatg    1080
   ttaacaaaca gatttaatga tcttttggat aggatggata taatgtttgg ttctacaggc    1140
   tctgctgaca tagaagaatg gatggctgga gttgcttggc tacactgttt gttgcccaaa    1200
   atggagtcag tggtgtatga ctttttaaaa tgcatggtgt acaacattcc taaaaaaaga   1260
   tactggctgt ttaaaggacc aattgatagt ggtaaaacta cattagcagc tgctttgctt   1320
   gaattatgtg gggggaaagc tttaaatgtt aatttgccct tggacaggct gaactttgag   1380
   ctaggagtag ctattgacca gtttttagta gtttttgagg atgtaaaggg cactggaggg   1440
   gagtccagag atttgccttc aggtcaggga attaataacc tggacaattt aagggattat   1500
   ttggatggca gtgttaaggt aaacttagaa aagaaacacc taaataaaag aactcaaata   1560
   tttccccctg gaatagtcac catgaatgag tacagtgtgc ctaaaacact gcaggccaga   1620
   tttgtaaaac aaatagattt taggcccaga gattatttaa agcattgcct ggaacgcagt   1680
   gagtttttgt tagaaaagag aataattcaa agtggcattg ctttgcttct tatgttaatt   1740
   tggtacagac ctgtggctga gtttgctcaa agtattcaga gcagaattgt ggagtggaaa   1800
   gagagattgg acaaagagtt tagtttgtca gtgtatcaaa aaatgaagtt taatgtggct   1860
   atgggaattg gagttttaga ttggctaaga aacagtgatg atgatgatga agacagccag   1920
   gaaaatgctg ataaaaatga agatggtggg gagaagaaca tggaagactc agggcatgaa   1980
   acaggcattg attcacagtc ccaaggctca tttcaggccc ctcagtcctc acagtctgtt   2040
   catgatcata atcagccata ccacatttgt agaggtttta cttgctttaa aaaacctccc   2100
   acacctcccc ctgaacctga acctgaaaca taa                                2133
<210> 9
   <211> 2133
   <212> DNA
   <213> simian virus 40
<400> 9
   atggataaag ttttaaacag agaggaatct ttgcagctaa tggaccttct aggtcttgaa     60
   aggagtgcct gggggaatat tcctctgatg agaaaggcat atttaaaaaa atgcaaggag    120
   tttcatcctg ataaaggagg agatgaagaa aaaatgaaga aaatgaatac tctgtacaag    180
   aaaatggaag atggagtaaa atatgctcat caacctgact ttggaggctt ctgggatgca    240
   actgagattc caacctatgg aactgatgaa tgggagcagt ggtggaatgc ctttaatgag    300
   gaaaacctgt tttgctcaga agaaatgcca tctagtgatg atgaggctac tgctgactct    360
   caacattcta ctcctccaaa aaagaagaga aaggtagaag accccaagga ctttccttca    420
   gaattgctaa gttttttgag tcatgctgtg tttagtaata gaactcttgc ttgctttgct    480
   atttacacca caaaggaaaa agctgcactg ctatacaaga aaattatgga aaaatattct    540
   gtaaccttta taagtaggca taacagttat aatcataaca tactgttttt tcttactcca    600
   cacaggcata gagtgtctgc tattaataac tatgctcaaa aattgtgtac ctttagcttt    660
   ttaatttgta aaggggttaa taaggaatat ttgatgtata gtgccttgac tagagatcca    720
   ttttctgtta ttgaggaaag tttgccaggt gggttaaagg agcatgattt taatccagaa     780
   gaagcagagg aaactaaaca agtgtcctgg aagcttgtaa cagagtatgc aatggaaaca     840
   aaatgtgatg atgtgttgtt attgcttggg atgtacttgg aatttcagta cagttttgaa     900
   atgtgtttaa aatgtattaa aaaagaacag cccagccact ataagtacca tgaaaagcat     960
   tatgcaaatg ctgctatatt tgctgacagc aaaaaccaaa aaaccatatg ccaacaggct    1020
   gttgatactg ttttagctaa aaagcgggtt gatagcctac aattaactag agaacaaatg    1080
   ttaacaaaca gatttaatga tcttttggat aggatggata taatgtttgg ttctacaggc    1140
   tctgctgaca tagaagaatg gatggctgga gttgcttggc tacactgttt gttgcccaaa    1200
   atggagtcag tggtgtatga ctttttaaaa tgcatggtgt acaacattcc taaaaaaaga    1260
   tactggctgt ttaaaggacc aattgatagt ggtaaaacta cattagcagc tgctttgctt    1320
   gaattatgtg gggggaaagc tttaaatgtt aatttgccct tggacaggct gaactttgag    1380
   ctaggagtag ctattgacca gtttttagta gtttttgagg atgtaaaggg cactggaggg    1440
   gagtccagag atttgccttc aggtcaggga attaataacc tggacaattt aagggattat    1500
   ttggatggca gtgttaaggt aaacttagaa aagaaacacc taaataaaag aactcaaata    1560
   tttccccctg gaatagtcac catgaatgag tacagtgtgc ctaaaacact gcaggccaga    1620
   tttgtaaaac aaatagattt taggcccaaa gattatttaa agcattgcct ggaacgcagt    1680
   gagtttttgt tagaaaagag aataattcaa agtggcattg ctttgcttct tatgttaatt    1740
   tggtacagac ctgtggctga gtttgctcaa agtattcaga gcagaattgt ggagtggaaa    1800
   gagagattgg acaaagagtt tagtttgtca gtgtatcaaa aaatgaagtt taatgtggct    1860
   atgggaattg gagttttaga ttggctaaga aacagtgatg atgatgatga agacagccag    1920
   gaaaatgctg ataaaaatga agatggtggg gagaagaaca tggaagactc agggcatgaa    1980
   acaggcattg attcacagtc ccaaggctca tttcaggccc ctcagtcctc acagtctgtt    2040
   catgatcata atcagccata ccacatttgt agaggtttta cttgctttaa aaaacctccc    2100
   acacctcccc ctgaacctga acctgaaaca taa                                 2133
<210> 10
   <211> 599
   <212> DNA
   <213> encephalomyocarditis virus
<400> 10
   cctctagcgg gatcaattcc gccccccccc cctaacgtta ctggccgaag ccgcttggaa      60
   taaggccggt gtgcgtttgt ctatatgtta ttttccacca tattgccgtc ttttggcaat     120
   gtgagggccc ggaaacctgg ccctgtcttc ttgacgagca ttcctagggg tctttcccct     180
   ctcgccaaag gaatgcaagg tctgttgaat gtcgtgaagg aagcagttcc tctggaagct     240
   tcttgaagac aaacaacgtc tgtagcgacc ctttgcaggc agcggaaccc cccacctggc     300
   gacaggtgcc tctgcggcca aaagccacgt gtataagata cacctgcaaa ggcggcacaa     360
   ccccagtgcc acgttgtgag ttggatagtt gtggaaagag tcaaatggct ctcctcaagc     420
   gtattcaaca aggggctgaa ggatgcccag aaggtacccc attgtatggg atctgatctg     480
   gggcctcggt gcacatgctt tacgtgtgtt tagtcgaggt taaaaaacgt ctaggccccc     540
   cgaaccacgg ggacgtggtt ttcctttgaa aaacacgata ataccatgat tgaacaaga      599
<210> 11
   <211> 30
   <212> DNA
   <213> artificial
<220>
   <223> Primer
<400> 11
   cgacgcgtgt ctgtaagctg aagacctggc 30
<210> 12
   <211> 31
   <212> DNA
   <213> artificial
<220>
   <223> Primer
<400> 12
   aaaagtactc ctgccctgcc tctgctggct c 31
<210> 13
   <211> 21
   <212> DNA
   <213> artificial
<220>
   <223> Primer
<400> 13
   gcctggtcaa catggtgaaa c 21
<210> 14
   <211> 31
   <212> DNA
   <213> artificial
<220>
   <223> Primer
<400> 14
   tgggctagcc caagaagctg gttctgagag g 31
<210> 15
   <211> 37
   <212> DNA
   <213> artificial
<220>
   <223> Primer
<400> 15
   ggggtaccag atggataaag ttttaaacag agaggaa 37
<210> 16
   <211> 37
   <212> DNA
   <213> artificial
<220>
   <223> Primer
<400> 16
   gggaattctt atgtttcagg ttcaggttca gggggag 37
<210> 17
   <211> 25
   <212> DNA
   <213> artificial
<220>
   <223> Primer
<400> 17
   tcagagggga caaaagggga gttgg 25
<210> 18
   <211> 23
   <212> DNA
   <213> artificial
<220>
   <223> Primer
<400> 18
   agaggcccct gccctccaga ggt 23
<210> 19
   <211> 23
   <212> DNA
   <213> artificial
<220>
   <223> Primer
<400> 19
   gcaaattcct cagggctcag ata 23
<210> 20
   <211> 25
   <212> DNA
   <213> artificial
<220>
   <223> Primer
<400> 20
   ccaggcctcc gaaggacgac gtggc 25
<210> 21
   <211> 21
   <212> DNA
   <213> artificial
<220>
   <223> Primer
<400> 21
   taggctgtcc ccagtgcaag t 21
<210> 22
   <211> 22
   <212> DNA
   <213> artificial
<220>
   <223> Primer
<400> 22
   aatgccaagc ttacttagat cg 22
<210> 23
   <211> 23
   <212> DNA
   <213> artificial
<220>
   <223> Primer
<400> 23
   cttaacctta ctcgccccag tct 23
<210> 24
   <211> 35
   <212> DNA
   <213> artificial
<220>
   <223> Primer
<400> 24
   aggctcgagc gcgggggtag gctttgctgt ctgag 35
<210> 25
   <211> 21
   <212> DNA
   <213> artificial
<220>
   <223> Primer
<400> 25
   ggaagccaag tgtccttctg c 21
<210> 26
   <211> 20
   <212> DNA
   <213> artificial
<220>
   <223> Primer
<400> 26
   gggcagggtt ttttacccac 20
<210> 27
   <211> 21
   <212> DNA
   <213> artificial
<220>
   <223> Primer
<400> 27
   agcttcctgg gtttccgatt t 21
<210> 28
   <211> 24
   <212> DNA
   <213> artificial
<220>
   <223> Primer
<400> 28
   gccaggtact cacctgtatg gctg 24
<210> 29
   <211> 22
   <212> DNA
   <213> artificial
<220>
   <223> Primer
<400> 29
   aatgccaagc ttacttagat cg 22
<210> 30
   <211> 32
   <212> DNA
   <213> artificial
<220>
   <223> Primer
<400> 30
   ggggctagcc ttaaccttac tcgccccagt ct 32
<210> 31
   <211> 4
   <212> DNA
   <213> Homo Sapiens1
<400> 31
   ccgg 4

## Description

### Technical Field

Embodiments described herein relate generally to a method of obtaining epigenetic information of a cell, a method of determining characteristics of a cell, and also a self-replicating vector, and an assay kit.

### Background Art

Many factors are involved in expression of a gene. The health condition of a subject is influenced by the presence or absence of such a factor, or a variation in quantity when such a factor is present. Further, when the status of such a factor is observed, it is possible to judge the health condition of the object.

Some factors involved in expression of a gene are accompanied by change in base sequence of DNA which forms a genome, while others are not accompanied by change in base sequence of DNA. Those factors accompanied by change in base sequence of DNA include polymorphism or variation. On the other hand, those not accompanied by change in base sequence of DNA involve a change in connection with epigenetic control. Data regarding such polymorphism, variation and epigenetic control (that is, epigenetic information) are useful in various fields, such as medicine, agriculture and fishing. The epigenetic information includes modification of DNA, chemical modification of histone, control by non-translatable RNA, etc.

For example, it has been recently reported that it is medically useful to acquire epigenetic information, including the presence or absence of modification, such as methylation to a specific base sequence, or variation in the amount of modification, etc.

The information regarding methylation of DNA is an example of useful epigenetic information. Abnormalities in DNA methylation are confirmed early in the multi-stages of the carcinogenic process. Further, a methylation in a target gene cluster is also confirmed in an initial stage of hepatic cancer.

The methylation reaction of genomic DNA is that a methyl group is added to the cytosine on a genome by DNA methyltransferase in a cell. When a genome is replicated at the time of cell division, a methyl group is added to the cytosine in the reproduced genome at the same position as that of the genome before the replication.

Barreto et al. ("Gadd45a promotes epigenetic gene activation by repair-mediated DNA demethylation", Nature, 8 February 2007, vol. 445, p.671-675) discloses that Gadd45a, which is a nuclear protein involved in maintenance of genomic stability, DNA repair and suppression of cell growth, has a key role in active DNA demethylation, and states that active demethylation occurs by DNA repair.

Majumder et al. ("Role of DNA methylation in regulation of human ribosomal RNA gene transcription", J Biol Chem, 2006, vol. 281, p.22602-22072) discloses introducing a vector including an rRNA gene promoter into a DNA methyltransferase mutant in order to investigate a role of DNA methyltransferase in regulating rDNA promoters activity. This document also discloses that there is differential regulation of rDNA expression from methylated and unmethylated rDNA promoters.

Presently, for the detection of methylation of DNA, there are, for example, the bisulfate treatment PCR method, the sequence method and a method which utilizes a reporter vector. For example, as a method utilizing a reporter vector, a screening method for a circadian-rhythm disorder-improving agent, in which the circadian-rhythm control mechanism by DNA methylation of a clock gene is assayed, has been reported. This screening method is a process of screening a circadian-rhythm disorder-improving agent, by producing a stably expressing cell line in which a known target nucleic acid sequence methylated in a cell is integrated in the chromosome and utilizing the methylation reaction taking place simultaneously with the genome replication at the time of cell division.

Maubach et al. ("GFAP promoter directs lacz expression specifically in a rat hepatic stellate cell line", World J Gastroenterol, 7 February 2006, 12(5), p.723-730) discloses that the GFAP promoter is capable of expressing the lacZ reporter specifically when a vector comprising GFAP promoter, a lacZ promoter and a replication initiator sequence is transfected into a rat HSC-T6 cell.

Mahon ("Vectors bicistronicaly linking a gene of interest to the SV40 large T antigen in combination with the SV40 origin of replication enhance transient protein expression and luciferase reporter activity", BioTechniques, August 2011, vo. 51, p.119-126) discloses self-replication of a plasmid in order to increase the sensitivity of an assay thereof. In particular, a vector including an SV40 origin of replication and an internal ribosome entry site (IRES) was used to transfect transfection-resistant CV-1 cells, thereby enhancing expression of red fluorescent protein (RFP) and a luciferase reporter activity.

Clontech ("pMetLuc2-reporter vector information", 3 September 2010) discloses a promoter reporter vector that allows the analysis of promoter function in a cell-based assay. The vector includes a promoter (MCS), Metridia luciferase promoter protein, and an SV40 origin.

Iqbal et al. ("Cytoplasmic injection of circular plasmids allows targeted expression in mammalian embryos", Biotechniques Rapid Dispatches, 1 November 2009, vol. 47(5), p.959-968) discloses testing expression of a reporter gene by introducing a plasmid completely methylated in vitro into murine and bovine zygotes by a microinjection method.

### Prior literatures

### Patent Literatures

Patent literature 1: JP 2009-232761 A
Patent literature 2: JP 2012-60894 A

### Non-patent literatures

Non-patent literature 1: Yoshiura K et al., Proc Nat'l Acad. Sci. USA. 1995, 92, 7416-7419.
Non-patent literature 2: Chen YL et al., Biochem. Biophys. Res. Commun. 2012, 425 (2), 290-296
Non-patent literature 3: Hayatsu H et al., Biochemistry 1970, 9, 2858-2865.
Non-patent literature 4: Gonzalgo ML et al., Nucleic Acids Res. 1997, 25, 2529-2531.
Non-patent literature 5: Herman JG et al., Proc. Nat'l Acad. Sci. USA. 1996, 93, 9821-9826.
Non-patent literature 6: Clark SJ et al., Nucleic Acids Res. 1994, 22, 2990-2997.
Non-patent literature 7: Frommer M et al., Proc. Nat'l Acad. Sci. USA. 1992, 89, 1827-1831.
Non-patent literature 8: Warnecke PM et al., Methods 2002, 27, 101-107.

### Summary

### Technical Problem

One of embodiments described herein aims to provide a method of obtaining epigenetic information of a cell easily.

### Solution to Problem

According to an embodiment, there is provided a method of obtaining epigenetic information of a cell. The method includes introducing a reporter nucleic acid construct in a nuclear of a subject cell, self-replication of the reporter nucleic acid construct, detecting the presence/absence of a signal produced in the subject cell and/or a size thereof, and obtaining epigenetic information of the subject cell based on the result obtained. The reporter nucleic acid construct is characterized by transcribing the state of methylation in a specific sequence on the genome of the subject cell onto a corresponding sequence thereof by substitution of a methyl group during the self-replication in the nuclear of the subject cell, and producing a detectable signal depending on the state of presence of the transcribed methyl group.

### Brief Description of Drawings

FIG. 1 is a diagram showing an example of the self-replicating vector.
FIG. 2 is a diagram showing an example of the self-replicating vector.
FIG. 3 is a diagram showing an example of the self-replicating vector.
FIG. 4 is a diagram showing examples of the self-replicating vector.
FIG. 5 is a diagram showing examples of the self-replicating vector.
FIG. 6 is a diagram showing an example of the self-replicating vector.
FIG. 7 is a diagram showing an example of the self-replicating vector.
FIG. 8 shows a scheme indicating one example of a method of acquires epigenetic information of a cell.
FIG. 9 shows a scheme indicating one example of a method of acquires epigenetic information of a cell.
FIG. 10 shows a scheme indicating one example of a method of acquires epigenetic information of a cell.
FIG. 11 shows a scheme indicating one example of a method of acquires epigenetic information of a cell.
FIG. 12 is diagram showing an example of a method of determining characteristics of a subject cell.
FIG. 13 is a block diagram showing an analytic device configured to perform the procedure of obtaining epigenetic information of a cell.
FIG. 14 is a diagram showing an example of a process of producing a self-replicating vector including a target sequence for methylation.
FIG. 15 is a graph showing experimental results.
FIG. 16 is a diagram showing a CK19 promoter region and a self-replicating vector including a replication initiator sequence.
FIG. 17 is a diagram showing a replication initiator protein gene expressing vector.
FIG. 18 is a diagram showing the position of a CCGG sequence of a CK19 promoter region.
FIG. 19 is a diagram showing experimental results.
FIG. 20 is a diagram showing experimental results.
FIG. 21 is a diagram showing experimental results.
FIG. 22 is a diagram showing experimental results.
FIG. 23 is a diagram showing a self-replicating vector including a COX2 promoter region and a replication initiator sequence.
FIG. 24 is a diagram showing the position of a CCGG sequence of a COX2 promoter region.
FIG. 25 is a diagram showing experimental results.
FIG. 26 is a diagram showing experimental results.
FIG. 27 is a diagram showing experimental results.
FIG. 28 is a diagram showing experimental results.
FIG. 29 is a diagram showing experimental results.
FIG. 30 is a diagram showing experimental results.
FIG. 31 is a diagram showing experimental results.
FIG. 32 is a diagram showing results of a luciferase assay.
FIG. 33 is a diagram showing an example of a method of producing a self-replicating vector including a methylated target sequence.
FIG. 34 is a schematic diagram showing a co-introducion of two kinds of vectors to a cell.
FIG. 35 is a diagram showing an example of a self-replicating vector.
FIG. 36 is a diagram showing experimental results.

### Detailed Description

A method of obtaining epigenetic information of a cell uses a self-replicative reporter nucleic acid construct. The reporter nucleic acid construct transcribes the modification status, such as the methylation status, of a specific sequence in the genome of a subject cell on its own sequence when self-replicating in the nuclear of the subject cell. That is, the reporter nucleic acid construct includes a sequence homologous to the specific sequence to be observed. Further, by the self-replication, the modification status, such as the methylation status, on the specific sequence of the genome is transcribed (copied) on a sequence homologous to that of the reporter nucleic acid construct. The reporter nucleic acid construct produces a detectable signal depending on the states of presence of a functional group, such as a methyl group, copied by the reporter nucleic acid construct. Here, the presence/absence, the size or quantity of a signal varies depending on the state of being of the functional group, such as a methyl group. In this manner, the reporter nucleic acid construct reports the modification status, such as the methylation status, on a specific sequence of a genome.

The reporting facilities of the reporter nucleic acid construct can be obtained for the first time if the reporter nucleic acid construct self-replicates. Therefore, the reporter nucleic acid construct is introduced in the nuclear of a subject cell placed under such conditions that the self-replication of the reporter nucleic acid construct is started. Then, after the time required for the reporter nucleic acid construct introduced in the nuclear of a subject cell to self-replicates elapses, the signal produced by the reporter nucleic acid construct in the subject cell is detected. The signal detection may be performed based on the presence/absence of a signal, or based on the size or quantity of a signal. The result obtained by the detection is the epigenetic information on the subject cell. Here, "the epigenetic information of a cell" may be such information as to whether or not a methyl group is joined to a base which a portion of a specific sequence on a genome of a subject cell or whether or not there has been substitution, or the like. Further, a "detectable signal" may be fluorescence, photogenesis, coloration or the like, or may be presentation of a substance such as protein. Or it suffices if, for example, a signal can be detected by a well-known procedure by itself. Furthermore, the signal changes the presence/absence or the size of its presentation depending on the state of being of the methyl group copied by the reporter nucleic acid construct.

The "reporter nucleic acid construct" is a nucleic acid construct to report epigenetic information of a cell to be examined. An example of correlation between a reporter nucleic acid construct and modification in a specific sequence of a genome will be described. The following is an example in which an item of epigenetic information is information on the methylation in a specific sequence of the genome. TABLE 1 below shows correlation between the reporter activity of a reporter nucleic acid construct and methylation.

**TABLE 1**

| Original modification state of reporter nucleic acid construct | Modification state of specific sequence on genome | Substitution of functional group in self replication | Size of detactable signal | Reporter activity |
|---|---|---|---|---|
| 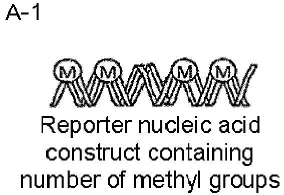 | 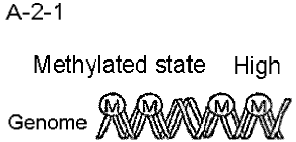 | 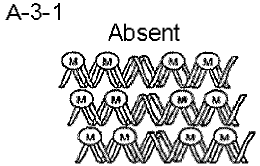 | 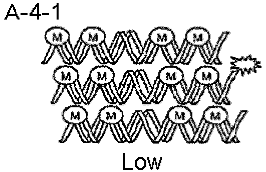 | A-5-1 |
| | | | | Low |
| | 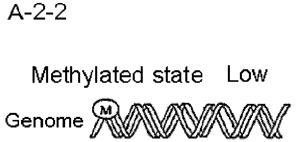 | 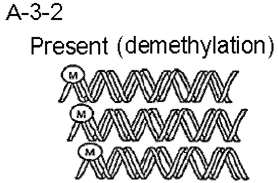 | 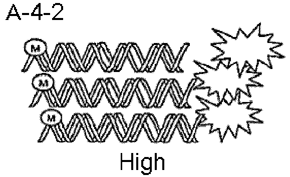 | A-5-2 |
| | | | | High |
| 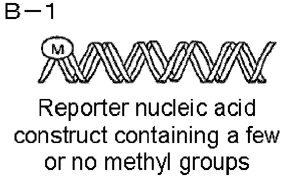 | 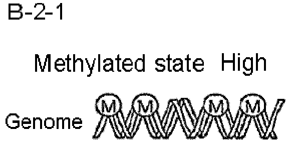 | 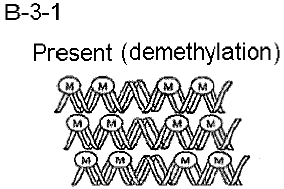 | 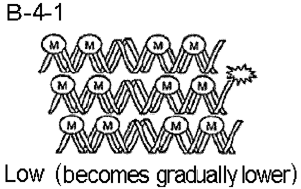 | B-5-1 |
| | | | | Low |
| | 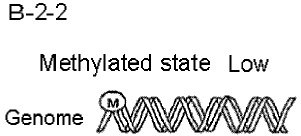 | 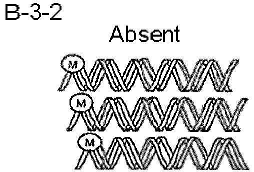 | 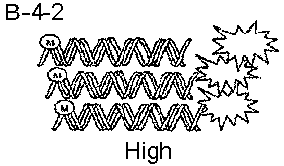 | B-5-2 |
| | | | | High |

The correlation shown in TABLE 1 is an example in which the signal of a reporter nucleic acid construct is increased by demethylation of the sequence corresponding to the reporter nucleic acid construct, and is reduced by methylation.

This reporter nucleic acid construct can be used, for example, to detect cancer. In cancer cells, demethylation occurs in a specific sequence on the genome. The epigenetic information in such a cell is transcribed by the corresponding sequence contained in the reporter nucleic acid construct at the time of the self-replication. For example, if the sequence corresponding to the reporter nucleic acid construct is methylated in advance, demethylation occurs at the time of the self-replication. At this time, the detectable signal from the reporter nucleic acid construct is increased. For example, it is possible to detect cancer by utilizing such a large signal as an index. Here, note that the term "corresponding sequence" refers to a specific sequence on the genome, that is, a sequence homologous to the sequence in which the modification to be detected is located.

For example, a reporter nucleic acid construct containing a great number of methyl groups may be prepared (A-1). Let us suppose here that the specific sequence on the genome contains a great number of methyl groups (A-2-1). In this case, when self-replicated, there are no or few substitutions of functional groups in the construct (A-3-1). Thus, the detected signal is low (A-4-1). Therefore, the reporter activity is relatively low (A-5-1). By contrast, suppose that the specific sequence on the genome does not contain a number of methyl groups (A-2-2). In this case, when self-replication occurs, functional groups are substituted in the construct, and demethylation occurs (A-3-2). Thus, the detected signal is large (A-4-2). Therefore, the reporter activity is relatively high (A-5-2).

Or, for example, a reporter nucleic acid construct containing a few methyl groups is prepared (B-1). Let us suppose here that the specific sequence on the genome contains a great number of methyl groups (B-2-1). In this case, when self-replication occurs, the functional groups in the construct are substituted and methylation occurs (B-3-1). Here, the detected signal is small (B-4-1). Further, in this case, the signal becomes smaller along with time. Therefore, the reporter activity is relatively low (B-5-1). By contrast, suppose that the specific sequence on the genome does not contain a number of methyl groups (B-2-2). In this case, when self-replication occurs, there are no or few substitutions of functional groups in the construct (B-3-2). Here, the detected signal is large (B-4-2). Therefore, the reporter activity is relatively high (B-5-2).

Note that the above-provided explanation on the correlation with the reporter activity of a reporter nucleic acid construct and methylation is only an example, and the embodiments are not limited to this example.

### 1. Reporter Nucleic Acid Construct

The reporter nucleic acid construct may be, for example, a self-replicating vector. The self-replicating vector as reporter nucleic acid constructs will be described with reference to drawings. Note that the same structural members are designated by the same reference symbols, and the explanations therefor will not be repeated. Further, structures may be combined to be utilized in another construct, or two or more constructs may be used at the same time in one analysis.

### First Example Vector

In an example method of obtaining epigenetic information of a cell, a self-replicating vector is used under the conditions which the replication initiator protein is expressed in the cell. The self-replicating vector is also called a "reporter vector".

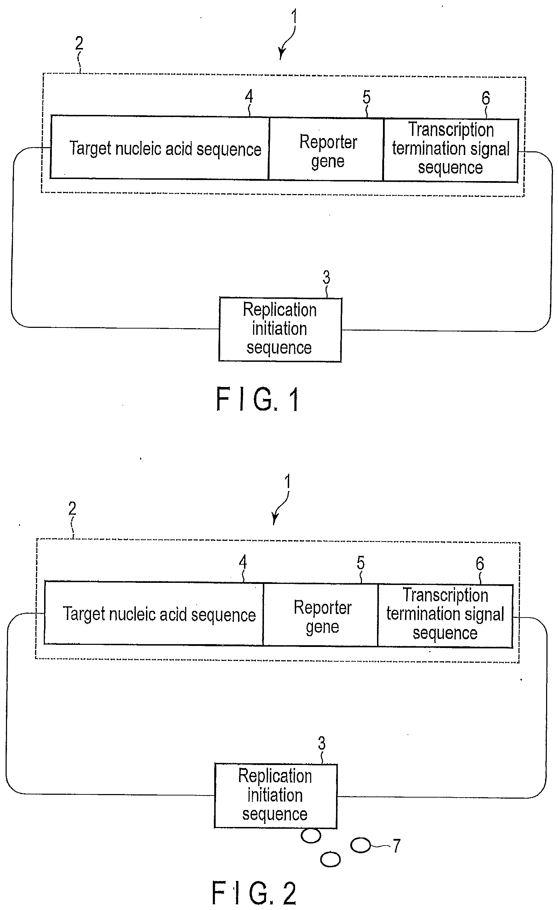

An example of the self-replicating vector will be described with reference to FIG. 1. A self-replicating vector 1 includes a reporter gene expression unit 2 and a replication initiator sequence 3. The replication initiator sequence 3 is located on a nucleic acid identical to that of the reporter gene expression unit 2.

The reporter gene expression unit 2 includes a target nucleic acid sequence 4, a reporter gene 5 coding a reporter protein and a transcription termination signal sequence 6.

The target nucleic acid sequence 4 is a nucleic acid sequence from which epigenetic information could be acquired. The target nucleic acid sequence 4 is homologous to a specific nucleic acid sequence of a subject cell. Further, the target nucleic acid sequence 4 includes a modified base (modifiable base). The self-replicating vector 1 (for example, target nucleic acid sequence 4) has the promoter activity which depends on the degree of the modification in this modified base. The activation of the promoter activity of the self-replicating vector 1 (for example, target nucleic acid sequence 4) is controlled by the modification of the modified base on the target nucleic acid sequence 4. Such a modified base serves as a target for modification. The target nucleic acid sequence 4 may be selected in advance in accordance with a necessity. Epigenetic information of a specific nucleic acid sequence in the subject cell is acquired from the epigenetic information acquired regarding the target nucleic acid sequence 4.

As to the target nucleic acid sequence 4, a "homogenous sequence" may be a sequence identical or substantially identical to the specific sequence on the genome. A sequence substantially identical to the specific sequence on the genome is, for example, a sequence in which one or a few bases may be substituted with, deleted from or added to the specific sequence. Further, the "specific sequence" may be a sequence from which epigenetic information could be acquired. For example, expressions such as "homology", "homologous" and "homologous to" may be used when a sequence is 90% or more or 95% or more identical to the genome sequence. The "sequence identical to the sequence on the genome" may be a sequence identical to a specific continuous sequence on the genome, or a sequence which can be formed when sequences identical to two or more specific non-continuous sequences on the genome are integrated with each other.

For example, the target nucleic acid sequence 4 may be a sequence which can be formed when sequences identical to two or more specific non-continuous sequences located different parts of the genome from each other are integrated with each other, or may contain such an integrated sequence. In other words, supposing that two or more sequences present in positions on the genome form one unit respectively, the target nucleic acid sequence 4 may be a sequence in which sequences homologous respective to these units are integrated with each other, or a sequence containing such an integrated sequence. Further, in other words, the target nucleic acid sequence 4 may consist of two or more units on a genome to correspond to units of homologous genes on the genome. The target nucleic acid sequence 4 may be in such a configuration in which, for example, one unit is homologous to the part of a specific promoter sequence on a genome and another unit is homologous to a sequence containing a modified base which can control activation of the promoter sequence.

For example, the part of the specific promoter sequence on a genome may not necessarily be present continuous to the sequence containing the modified base which can control the activation of the promoter sequence, on the genome. That is, a further sequence may be included therebetween. If such a further sequence is of the type which does not affect the promoter sequence or its activity, it is not necessarily required to include such a further sequence in the target nucleic acid sequence 4. Further, as long as the target nucleic acid sequence 4 contains a modified base and a promoter activity which depends on the degree of the modification in the modified base, the target nucleic acid sequence 4 may include any further sequence in addition to the sequence homologous to the sequence on the genome of the subject cell. The further sequence does not obstruct the sequence from including a modified base on the target nucleic acid sequence 4 or having a promoter activity which depends on the degree of the modification in the modified base. For example, the further sequence may be included between bases which constitute a sequence homologous to the sequence on the genome of the subject cell, or may be included to be adjacent to the sequence homologous to the sequence on the genome of the subject cell. The above-provided descriptions are made in connection with the target nucleic acid sequence 4 with regard to a "sequence homologous" to a sequence on a genome, but as to a subject cell, a "sequence homologous" to the target nucleic acid sequence 4 should be comprehended in a similar way.

The self-replicating vector 1 is a vector which replicates itself after being introduced into a subject cell. The target nucleic acid sequence 4 is a sequence homologous to a specific nucleic acid sequence included in the genome of the subject cell. Because of such a configuration, it is possible to transcribe the modification state of the homologous sequence on the genome of the subject cell to the target nucleic acid sequence 4 in the self-replicating vector 1 by utilizing the self-replicating ability of the self-replicating vector 1.

Examples of the target nucleic acid sequence 4 are a SOX2 gene-promoter sequence, a GFAP gene-promoter sequence, a CK19 gene-promoter sequence, a COX2 gene-promoter sequence, but not limited to these.

The modification of the target nucleic acid sequence 4 may be, for example, methylation or alkylation. Here, the state of the modification of the target nucleic acid sequence 4 indicates whether or not a methyl group and/or an alkyl group, which are functional groups, have joined to a base of the target nucleic acid sequence 4. The "epigenetic information" of a subject cell may be whether such a functional group is present or not, or may be the information as to the quantity of presence of functional groups. Or the change in the state of the modification, that is, the presence/absence of substitution of a functional group, or the degree thereof may as well be such information. Moreover, it may be combination of any of these. The modification of the target nucleic acid sequence 4 may be modification to cytosine and/or guanine in the main chain of the target nucleic acid sequence 4, for example. When two or more cytosine and/or guanine are present in the target nucleic acid sequence 4, the modification may occur on one or more of them. For example, the modification of the target nucleic acid sequence 4 may be the methylation to the cytosine and/or guanine in the target nucleic acid sequence 4.

The promoter activity means induction of the transcription of the gene functionally linked downstream of the promoter sequence. When the target nucleic acid sequence 4 has a promoter activity, the promoter activity may be derived from the entire sequence of the target nucleic acid sequence 4, or a part of target nucleic acid sequence 4. Further, for example, the target nucleic acid sequence 4 may also contain the minimum promoter. Furthermore, modified bases may be continuously or intermittently present over an entire sequence which can exhibit the promoter activity in the target nucleic acid sequence 4, or may be present in a portion of the target nucleic acid sequences 4 except for that which can exhibit the promoter activity, or may be a combination of these. The promoter activation of the target nucleic acid sequence 4 is controlled by the state of the modification of the modified base on the target nucleic acid sequence 4. In other words, the promoter activity of the target nucleic acid sequence 4 is activated depending on the degree of the modification of the base within the sequence of the target nucleic acid sequence 4.

The promoter activity of the target nucleic acid sequence 4 may be activated in any situations of the followings, that is, for example, when the degree of the modification in the modified base of the target nucleic acid sequence 4 is low, or when modification occurs, or when modification does not occur. Examples of the characteristics of the target nucleic acid sequence 4 regarding the promoter activity are "being activated depending on the degree of modification of the bases within the sequence of the target nucleic acid sequence 4" and "being activated depending on the degree of substitution of the functional groups in a base".

Examples of the sequence having a promoter activity which depends on the degree of the modification in the modified base within the sequence of the target nucleic acid sequence 4 may be those derived from the cell to be tested, or those synthesized artificially. An example of the sequence may be a 5' upstream region (-1651 to +32bp) (SEQ ID NO: 1) of a glial fibrillary acidic protein (GFAP) gene. The 5' upstream region sequence of GFAP gene is activated when there is no methylation of a target nucleic acid sequence. The sequence thereof is shown in TABLE 2. Further examples thereof may be a 5' upstream region (-617 to +61bp) (SEQ ID NO: 2) of cytokeratin 19 (CK19) gene and a 5' upstream region (-540 to +115bp) (SEQ ID NO: 3) of cyclooxygenase 2 (COX2) gene. The sequences thereof are shown in TABLES 2, 3 and 4.

**TABLE 2**

| <SEQ ID NO: 1> 5' upstream region (-1651 to +32bp) of mouse GFAP gene |
|---|
| |
| |

**TABLE 3**

| <SEQ ID NO: 2> 5' upstream region (-617 to +61bp) of human CK19 gene |
|---|
| |

**TABLE 4**

| <SEQ ID NO: 3> 5' upstream region (-540 to +115bp) of human COX2 gene |
|---|
| |

The reporter gene 5 is functionally linked downstream of the target nucleic acid sequence 4. That is, the target nucleic acid sequence 4 is functionally linked upstream of the reporter gene 5. The reporter gene 5 is transcribed when the target nucleic acid sequence 4 is activated. By this transcription, a reporter protein is expressed. Detection of a reporter protein may be performed by detection of fluorescence, photogenesis, coloration, or the like, or may be detected with a protein detection means well-known in itself.

The reporter protein may be, for example, luciferase, β-galactosidase, nitric monoxide synthase, xanthine oxidase, blue fluorescent protein, green fluorescent protein, red fluorescent protein and a heavy metal binding protein. In such cases, the reporter gene 5 may be a gene which encodes a respective protein. Examples of the reporter gene 5 are a luciferase gene, a β-galactosidase gene, a nitric-monoxide-synthase gene, a xanthine-oxidase gene, a blue fluorescent protein gene, a green fluorescent protein gene, a red fluorescent protein gene and a heavy metal binding protein gene.

The transcription termination signal sequence 6 may be a sequence which terminates the transcription of the upstream gene, and may be, for example, a poly (A) addition signal.

The poly (A) addition signal may be such a type which functions to terminate the transcription of a mammalian gene. Examples thereof are SV40 virus late poly (A) addition signal (SEQ ID NO: 4) and bovine growth hormone gene poly (A) addition signal (SEQ ID NO: 5). Note that the poly (A) addition signals usable in the embodiment are not limited to those mentioned, but as long as the functions as a poly (A) addition signal are not lost, those having re-modified gene sequence may be used. Examples of such poly(A) addition signals will be provided together with their sequences in TABLE 5 below.

**TABLE 5**

| <SEQ ID NO: 4> SV40 virus late poly (A) |
|---|
| |

| <SEQ ID NO: 5> bovine growth hormone transcription termination signal sequence |
|---|
| |

The expression "functional" used for linkage of two or more sequences refers to such a state that they are linked so that each gene is able to exhibit its object function. For example, in the case of a sequence which encodes a protein, the state indicates that amino acid sequences encoded by base sequences are properly linked. In other words, it indicates that a peptide by which an object activity functions, is synthesized in a cell into which the base sequences are introduced without displacement of the frames of codons of the amino acid. Moreover, here, the expression "linking functionally" may be used exchangeably with the expression "linking to be operable".

The transcription termination signal sequence 6 is functionally linked downstream of the reporter gene 5.

The self-replicating vector 1 can self-replicate within a subject cell under a condition that the replication initiator protein has been expressed in the subject cell. The replication initiator sequence 3 is a sequence which initiates the self-replication by binding of the replication initiator protein.

The replication initiator sequence 3 may be a sequence which starts the self-replication in a mammalian cell, for example, by being activated by binding of a replication initiator protein and be a replication initiator sequence known in itself. The replication initiator sequence 3 may be a replication initiator sequence derived from, for example, simian virus 40 (SEQ ID NO: 6), Epstein-Barr virus, or mouse polyomavirus, but not limited to these. An example of the replication initiator sequence will be provided in TABLE 6 below.

**TABLE 6**

| <SEQ ID NO: 6> simian virus 40 replication initiator sequence |
|---|
| |

The self-replicating vector 1 may be a self-replicative vector, such as a plasmid vector, for example. Here, for example, a plasmid vector well-known in itself may be used. Examples of the plasmid vector of this self-replicating vector are provided in JP 2013-42721 A.

The replication of the self-replicating vector 1 is initiated when a replication initiator protein is bound to a replication initiator sequence 3.

FIG. 2 shows schematically a state that a replication initiator protein 7 is bound to the replication initiator sequence 3 of the self-replicating vector 1. The replication initiator protein 7 is bound to the replication initiator sequence 3, and further two or more proteins relating to DNA replication are bound. With these bound proteins, the self-replicating vector 1 replicates in the subject cell.

The replication initiator protein 7 is expressed under a "condition that replication initiator proteins are expressed in a cell". The "condition that replication initiator proteins are expressed in a cell" may be such a condition that the replication initiator protein which initiates the replication of the self-replicating vector 1 is expressed in a cell into which the vector has been introduced. That is, it suffices if the replication initiator protein unit is present in the subject cell into which the self-replicating vector 1 should be introduced. The "replication initiator protein unit" is a unit for expressing a replication initiator protein, and may be referred to as a "replication initiator protein expression unit".

The replication initiator protein unit may include a sequence which can express the replication initiator protein to initiate the replication of the self-replicating vector 1. Or the replication initiator protein unit may consist of a sequence(s) which can express the replication initiator protein to initiate the replication of the self-replicating vector 1. For example, the replication initiator protein unit includes a promoter which controls the expression of a sequence which encodes a replication initiator protein, a sequence which encodes a replication initiator protein functionally linked downstream thereof and a transcription termination signal sequence functionally linked downstream thereof. Or, for example, the replication initiator protein unit may consist of a promoter which controls the expression of a sequence which encodes a replication initiator protein, a sequence which encodes a replication initiator protein functionally linked downstream thereof and a transcription termination signal sequence functionally linked downstream thereof.

Examples of the sequence bound to the replication initiator sequence 3 and encoding the replication initiator protein to initiate the replication of the self-replicating vector 1 are a large T-antigen gene of simian virus 40, EBNA-1 gene of Epstein-Barr virus and a large T-antigen gene of mouse polyomavirus.

Preferable examples of the large T-antigen gene are shown in TABLES 7, 8 and 9, respectively. That is, they may be nucleic acids represented by SEQ ID NO: 7, SEQ ID NO: 8 and SEQ ID NO: 9, respectively. Or they may be such nucleic acids that one or a few bases are deleted, substituted or added in the sequence of SEQ ID NO: 7, SEQ ID NO: 8 or SEQ ID NO: 9, and that has a DNA replication initiating function. Here, the DNA replication initiating function means that it encodes a protein which can initiate replication.

**TABLE 7**

| <SEQ ID NO: 7> large T-antigen gene of simian virus 40 (wild type) |
|---|
| |

**TABLE 8**

| <SEQ ID NO: 8> large T-antigen gene of simian virus 40 (variant type 1) |
|---|
| |
| |

**TABLE 9**

| <SEQ ID NO: 9> large T-antigen gene of simian virus 40 (variant type 2) |
|---|
| |
| |

A replication initiator protein unit may be present on a nucleic acid of the self-replicating vector 1, or on a nucleic acid different from the nucleic acid of the self-replicating vector 1. When a replication initiator protein unit is present on a nucleic acid different from that in the self-replicating vector 1, the replication initiator protein unit may be present on a chromosome of a subject cell, or on another nucleic acid contained in the subject cell.

When a replication initiator protein unit is present on a nucleic acid different from that of the self-replicating vector 1, the replication initiator protein unit has the following structure. That is, it comprises sequences including a promoter constitutively expressed, a sequence linked downstream thereof and encoding a replication initiator protein and a transcription termination signal sequence functionally linked downstream thereof, or it consists of these sequences themselves. It suffices if, with the above-described structure, a sequence encoding a replication initiator protein is transcribed and translated to produce a replication initiator protein.

When a replication initiator protein unit is included in the self-replicating vector 1, the replication initiator sequence 3 may be included in the self-replicating vector 1. Expressions of the reporter gene expression unit 2 and the replication initiator protein unit in the self-replicating vector 1 may be controlled by one promoter, or by two promoters of the same type independent from each other, or by two promoters independent and different from each other.

When the expression is controlled by different promoters, for example, the replication initiator protein unit is present on a nucleic acid of the self-replicating vector 1 independently of the reporter gene expression unit 2. The replication initiator protein unit may only comprise a promoter constitutively expressed and a sequence linked downstream thereof and encoding a replication initiator protein. Further, a transcription termination signal sequence may be functionally linked downstream of the sequence encoding the replication initiator protein.

When the reporter gene expression unit 2 and the replication initiator protein unit are controlled by one promoter in the self-replicating vector 1, for example, the following structure may be taken. That is, an example of the self-replicating vector 1 may comprise an internal ribosomal entry site (IRES) sequence (SEQ ID NO: 10) functionally linked downstream of the reporter gene expression unit, a sequence functionally linked downstream of the IRES sequence and encoding the replication initiator protein and a transcription termination signal sequence functionally linked downstream thereof. The IRES sequence is provided in TABLE 10 below.

**TABLE 10**

| <SEQ ID NO: 10>internal ribosomal entry site (IRES) sequence of encephalomyocarditis virus |
|---|
| |

The replication initiator protein unit includes a sequence which encodes the replication initiator protein, a promoter functionally linked upstream thereof and constitutively expressed, a sequence functionally linked downstream thereof and encoding the replication initiator protein, and a transcription termination signal sequence functionally linked down stream thereof, or it may be consists of these sequences themselves. The sequence encoding the replication initiator protein is transcribed and translated to produce the replication initiator protein. Note that the self-replicating vector 1 includes a replication initiator sequence in the same vector. The produced replication initiator protein is bound to the replication initiator sequence in the vector. Such a self-replicating vector will be described as an example of plasmid vector in the second embodiment.

When the replication initiator protein unit is present on the genome of a subject cell, the replication initiator protein is produced in the following manner, for example. That is, a replication initiator protein gene expression vector is introduced into a subject cell by a introducion method or using a viral vector, followed by culturing, thus the replication initiator protein gene expression vector is integrated in the chromosome of the cell. The introducion method may be performed by the biochemical process or the physicochemical process. Examples of the biochemical process may be a lipofection method using cation lipid, a magnetofection method using magnetic particles, and a method which uses calcium chloride. An example of the physicochemical procedure may be an electroporation method. The biochemical process or the physicochemical process may be used solely or in combination. For example, a lipofection method and a magneto-fection method, which are biochemical processes, may be combined, or a lipofection method, which is a biochemical process, and an electroporation method, which is a physicochemical process, may be combined.

When a replication initiator protein gene expression vector is integrated in a chromosome stably, the replication initiator protein gene can be transcribed and translated stably within the subject cell, thus producing a replication initiator sequence. Examples of the cell line which stably expresses a replication initiator sequence are a HEK293 T cell and a COS cell.

When the replication initiator sequence unit is present on a nucleic acid chain different from the nucleic acid of the self-replicating vector 1 or different from the chromosome of the subject cell, the replication initiator sequence unit may be present within a second vector other than self-replicating vector 1. For example, the second vector may be a vector which expresses a replication initiator sequence within a subject cell, and a vector well-known in itself may be used. The second vector may also be called a "replication initiator protein gene expression vector".

Thus, for example, a replication initiator sequence unit may be contained in a self-replicating vector. Moreover, for example, a subject cell may include a further sequence different from the self-replicating vector or different from the genome of the subject cell, and the replication initiator sequence unit may be contained in such a further sequence. Such a further sequence may be included in the chromosome of the subject cell or in a further nucleic acid chain, such as the second vector.

The combination of the gene which encodes the replication initiator sequence and the replication initiator sequence may be such as to be able to initiate replication. Examples of such a combination are as follows:
a combination in which the gene which encodes the replication initiator sequence is a large T-antigen gene of simian virus 40 and the replication initiator sequence is that from simian virus 40;
a combination in which the gene which encodes the replication initiator protein is EBNA-1 gene of Epstein-Barr virus and the replication initiator sequence is that from Epstein-Barr virus; and
a combination in which the gene which encodes the replication initiator protein is large T-antigens gene of mouse polyomavirus and the replication initiator sequence is that from mouse polyomavirus. Although these combinations are preferable, the embodiments are not limit to these.

The subject cell may be, for example, a group of cells and/or a single cell. The subject cell may be a cell contained in blood, urine, feces, sperm, saliva, tissue obtained form a biopsy, mucosa in the oral cavity, coelomic fluid, expectoration, etc., or may be a cultured cell or the like.

Introducion of the self-replicating vector 1 to a subject cell may be performed in vitro, or in vivo.

### Second Example Vector

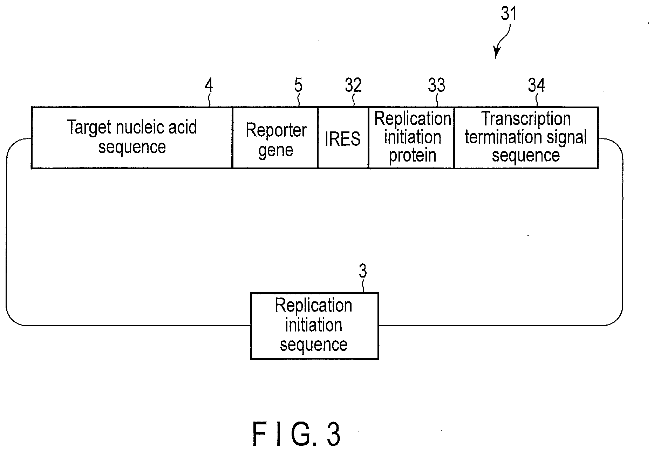

Next, an example of the plasmid vector will be described with reference to FIG. 3.

A self-replicating vector 31, which is a plasmid vector, comprises, as shown in FIG. 3, a target nucleic acid sequence 4, a reporter gene 5 functionally linked downstream thereof, an IRES 32 functionally linked downstream thereof, a sequence 33 functionally linked downstream thereof and encoding a replication initiator protein, a transcription termination signal sequence 34 functionally linked downstream thereof, and a replication initiator sequence 3 functionally linked downstream thereof. In this example, the components of a reporter gene expression unit 2 are the target nucleic acid sequence 4, the reporter gene 5 and the transcription termination signal sequence 34. Further, the components of the replication initiator protein unit are the target nucleic acid sequence 4, the IRES 32, the sequence 33 that encodes the replication initiator protein and the transcription termination signal sequence 34.

The term "IRES" is the abbreviation for "internal ribosomal entry site" and it is also called an "internal ribosome junction sequence". The IRES may be a sequence derived from, for example, encephalomyocarditis-virus. In the presence of IRES 32, the sequence of the reporter gene 5 is transcribed by the target nucleic acid sequence 4 activated depending on the degree of modification of the base within the sequence of the target nucleic acid sequence 4, and then the sequence 33 which encodes a replication initiator protein transcribed. After the sequence of the sequence 33 which encodes replication initiator protein is read, the transcription is stopped by the presence of the transcription termination signal sequence 34. A replication initiator protein thus produced by being transcribed and translated from the sequence 33 which encodes the replication initiator protein is linked to the replication initiator sequence 3, and thus the replication of the self-replicating vector 31 is initiated.

In the self-replicating vector 31 including the target nucleic acid sequence 4 introduced into the subject cell, a modifiable base of the target nucleic acid sequence 4 to be modified may have been or not have been modified at the time of introducion of a vector.

When the modifiable base(base to be modified) of the target nucleic acid sequence 4 included in the self-replicating vector 31 has not been modified, for example, it behaves as follows after being introduced into a subject cell. First, when the modification state of the target nucleic acid present in the subject cell is high, the modifiable base of the target nucleic acid sequence 4 is highly modified. Thus, the target nucleic acid sequence 4 is not activated and a reporter gene and a replication initiator protein gene are not expressed. Therefore, the replication of the self-replicating vector 31 stops. By contrast, when the modification state of the target nucleic acid present in a subject cell is low or there is no modification, the modification state of the modifiable base of the target nucleic acid sequence 4 is maintained low. Thus, the target nucleic acid sequence 4 is activated. When the target nucleic acid sequence 4 is in an activated state, the reporter gene 5 linked by the IRES 32 and the sequence 33 which encodes a replication initiator protein are transcribed by bicistronic mRNA and translated. As the IRES 32 is integrated between two genes, these two genes are transcribed as a single bicistronic mRNA. Ribosome not only translates the reporter gene 5 from the bicistronic mRNA, but also links to the position of IRES and translates the sequence which encodes a replication initiator protein. The transcription termination signal sequence 34 is a base sequence which encodes a signal for terminating transcription of the reporter gene 5 and the sequence 33 which encodes replication initiator protein. The replication initiator protein transcribed from the sequence 33 which encodes a replication initiator protein and translated by components of the subject cell recognizes the replication initiator sequence 3, and binds thereto. Further, two or more proteins relating to replication of the DNA contained in a subject cell bind thereto. The replication of the self-replicating vector 31 is performed in the subject cell by these bound proteins.

When the modifiable base of the target nucleic acid sequence 4 has been modified, it behaves as follows after being introduced into the subject cell. First, when the modification state of the nucleic acid present in the subject cell is high, the initial degree of the modification is maintained in the modified base of the target nucleic acid sequence 4, and the target nucleic acid sequence 4 is not activated. Therefore, a reporter gene and a replication initiator protein gene are not expressed. Thus, the replication of the self-replicating vector 31 stops. By contrast, when the modification state of the nucleic acid present in the subject cell is low or there is no modification, the modification state in the modified base of the target nucleic acid sequence 4 decreases or is lost, and therefore the target nucleic acid sequence 4 is activated. When the target nucleic acid sequence 4 is in an activated state, the reporter gene 5 linked by the IRES 32 and the sequence 33 which encodes a replication initiator protein are transcribed to bicistronic mRNA and translated. As the IRES 32 is integrated between two genes, these two genes are transcribed as a single bicistronic mRNA. Ribosome not only translates the reporter gene 5 from the bicistronic mRNA, but also links to the position of IRES and translates the sequence which encodes a replication initiator protein. The transcription termination signal sequence 34 is a base sequence which encodes a signal for terminating transcription of the reporter gene 5 and the sequence 33 which encodes replication initiator protein. The replication initiator protein transcribed from the sequence 33 which encodes a replication initiator protein and translated by components of the subject cell recognizes the replication initiator sequence 3, and binds thereto. Further, two or more proteins relating to replication of the DNA contained in a subject cell bind thereto. The replication of the self-replicating vector 31 is performed in the subject cell by these bound proteins.

When the degree of the modification in the modifiable base of the target nucleic acid sequence 4 is low, the reporter gene 5 is expressed. Furthermore, when the sequence present further downstream is read, the replication of the self-replicating vector 31 is initiated. The replication product produced by the replication of the self-replicating vector 31 has the same sequence as the self-replicating vector 31. But, the modification state of the target nucleic acid present in the subject cell reflects the modification state of the modifiable or modified base of the target nucleic acid sequence 4. As a result, if the degree of the modification of the modified base of the target nucleic acid sequence 4 becomes higher, the target nucleic acid sequence 4 is not activated. Consequently, the reporter gene 5 is not expressed and the sequence further downstream is not read. Therefore, the replication of the self-replicating vector 31 is stopped.

### Third Example vector

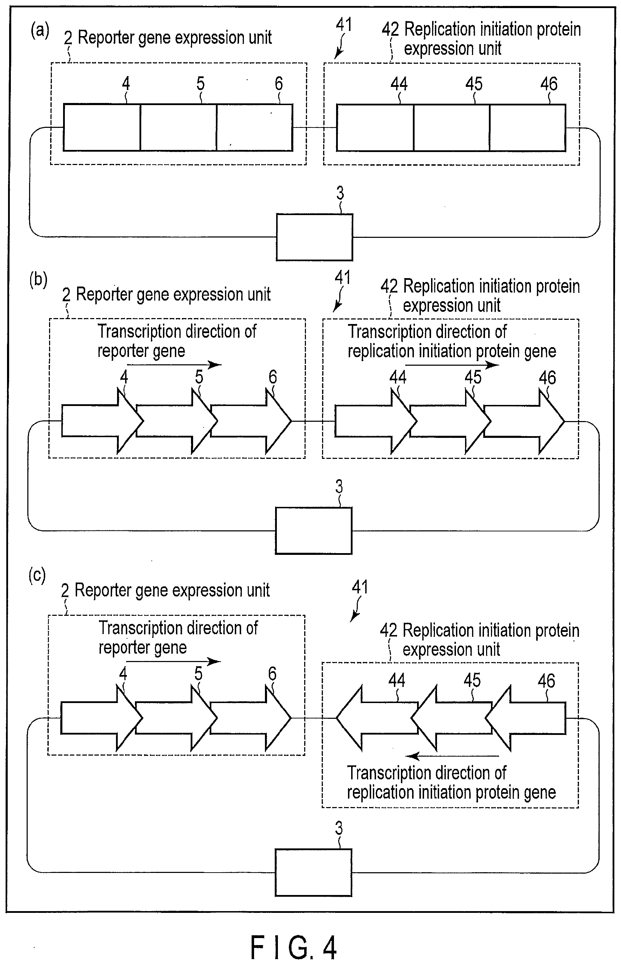

Another example of the plasmid vector will be described with reference to FIG. 4 (a), (b) and (c).

First, the basic structure will be described with reference to FIG. 4 (a). A self-replicating vector 41, which is a plasmid vector, includes a reporter gene expression unit 2, a replication initiator protein unit 42 linked thereto and a replication initiator sequence 3 linked thereto. The reporter gene expression unit 2 includes a target nucleic acid sequence 4, a reporter gene 5 functionally liked downstream thereof and a transcription termination signal sequence 6 functionally linked thereto. The replication initiator protein unit 42 is present on the same nucleic acid as that of the reporter gene expression unit 2 and includes a constitutively expressed promoter 44, a sequence 45 that encodes a replication initiator protein functionally linked downstream thereof, and a transcription termination signal sequence 46 functionally linked downstream thereof. The replication initiator sequence 3 is linked downstream of the transcription termination signal sequence 46 of the replication initiator protein unit 42.

The base to be modifiable of the target nucleic acid sequence 4 of the self-replicating vector 41 introduced to a subject cell may have been or may not have been modified at the time of introducion of the vector 41. Examples of the sequence which has the promoter activity depending on the degree of modification of the modified base of the target nucleic acid sequence 4 are the same as those mentioned above.

The self-replicating vector 41 behaves as follows, after being introduced into the subject cell. That is, the replication product produced by the replication of the self-replicating vector 41 has the same sequence as the self-replicating vector 41. But, the modification state of the modifiable base of the target nucleic acid sequence 4 of self-replicating vector reflects the modification state of the target nucleic acid present in the subject cell.

For example, when the modifiable base of the target nucleic acid sequence 4 has been modified and the modification state of the nucleic acid present in the subject cell is high, the target nucleic acid sequence 4 is not activated. Therefore, the reporter gene 5 is not expressed. By contrast, the following is the case where the modifiable base of the original target nucleic acid sequence 4 has been modified and the modification state of the target nucleic acid present in the subject cell is low, or there is no modification. The modification state of the modified base of the target nucleic acid sequence 4 of the self-replicating vector 41 reflects the modification state of the target nucleic acid present in a subject cell. As a result, the degree of the modification in the modified base of the target nucleic acid sequence 4 becomes lower, and the target nucleic acid sequence 4 is activated. Therefore, the reporter gene 5 is expressed.

When the modifiable base of the target nucleic acid sequence 4 has not been modified, and the modification state of the target nucleic acid present in the subject cell is high, the modifiable base of the target nucleic acid sequence 4 is modified. Therefore, the target nucleic acid sequence 4 is not activated. Consequently, a reporter gene is not expressed. By contrast, when the modification state of the nucleic acid present in the subject cell is low or there is no modification, the initial degree of the modification is maintained, and therefore the target nucleic acid sequence 4 is activated. Consequently, the reporter gene 5 is expressed.

The transcription of the sequence 45 which encodes a replication initiator protein is controlled by the promoter 44 constitutively expressed. Since the constitutively expressed promoter 44 is in the activated state at all times, and repeatedly activated. The transcription of the sequence 45 which encodes a replication initiator protein is stopped by the transcription termination signal sequence 46. The replication initiator protein is repeatedly produced because of the activity of the constitutively expressed promoter 44 regardless of the state of modification of the sequence having a promoter activity which depends on the degree of modification of the base within the sequence of the target nucleic acid sequence 4.

Examples of the constitutively expressed promoter 44 may be a cytomegalovirus promoter, a Rous-sarcoma-virus promoter, a simian virus initial promoter and a simian-virus late promoter.

As for the self-replicating vector 41 shown in FIG. 4 (a), the replication initiator protein unit 42 is linked downstream of the reporter gene expression unit 2. Further, the replication initiator sequence 3 is linked downstream thereof. But the structure of the self-replicating vector 41 is not limited to the example shown in FIG. 4. For example, the replication initiator sequence 3 may be linked downstream of the reporter gene expression unit 2 and the replication initiator protein unit 42 may be further linked downstream thereof. Or, the reporter gene expression unit 2 may be linked downstream of the replication initiator protein unit 42 and the replication initiator sequence 3 may be further linked downstream thereof. Or, the replication initiator sequence 3 may be linked downstream of the replication initiator protein unit 42 and the reporter gene expression unit 2 may be further connected downstream thereof.

Further, the direction of each of the reporter gene expression unit 2 and the replication initiator protein unit 42 is not limited to that of the example shown in FIG. 4 (a). The direction in which the sequence is read in the self-replicating vector 41 shown in FIG. 4 (a) is indicated by an arrow (FIG. 4(b)). In this case, the reporter gene expression unit 2 is read from left to right as viewed. Similarly, the replication initiator protein expression unit 42 is also read from left to right as viewed. Furthermore, an example designed and structured, for example, so that the reporter gene expression unit 2 and the replication initiator protein unit 42 are directed in opposite directions is shown in FIG. 4 (c). In the case of the example of FIG. 4 (c), the reporter gene expression unit 2 is read from left to right as viewed, whereas the replication initiator protein expression unit 42 is read from right to left. But, the directions of the reporter gene expression unit 2 and the replication initiator protein unit 42 are not limited to those of this example. The replication initiator protein unit 42 may be, for example, arranged in an upper or lower stream of the reporter gene expression unit 2. In any case, the replication initiator protein unit 42 may be linked so that replication protein gene may be transcribed in an opposite direction to the transcribe direction of the reporter gene. Or even any case of these, the replication initiator protein unit 42 may be linked so that a replication protein gene may be transcribed in the same direction as the transcribed direction of the reporter gene.

### Fourth Example Vector

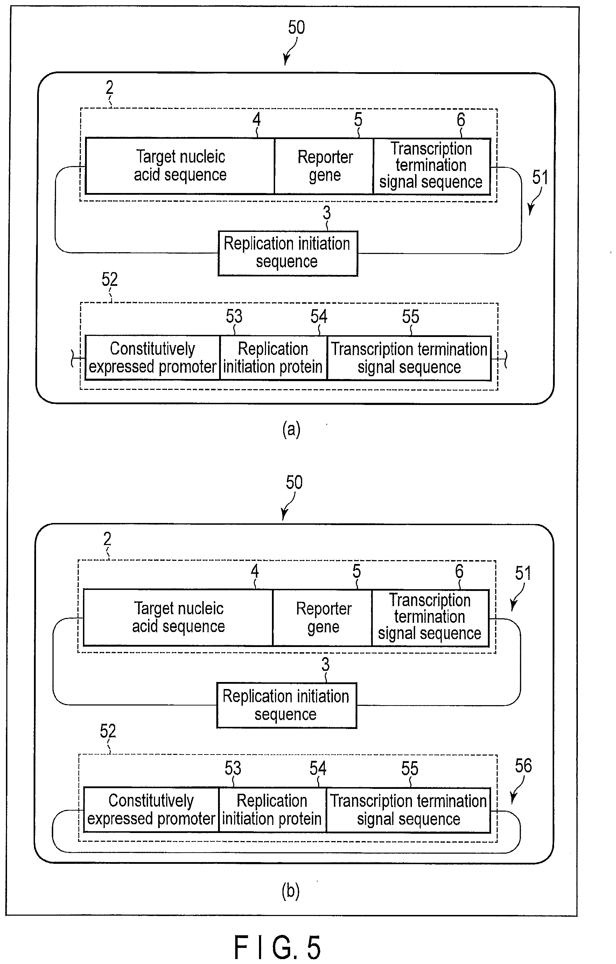

A further example of the plasmid vector will be described with reference to FIG. 5. FIG. 5 (a) shows a self-replicating vector 51 introduced to a subject cell 50. The self-replicating vector 51 includes a reporter gene expression unit 2 and a replication initiator sequence 3. The reporter gene expression unit 2 includes a target nucleic acid sequence 4, a reporter gene 5 functionally linked downstream thereof and a transcription termination signal sequence 6 functionally linked downstream thereof. In the subject cell 50, a replication initiator protein unit 52 is present independent from the self-replicating vector 51. The replication initiator protein unit 52 includes a constitutively expressed promoter 53, a sequence 54 that encodes a replication initiator protein functionally linked downstream thereof and a transcription termination signal sequence 55 functionally linked downstream thereof.

FIG. 5 (b) shows an example in which the replication initiator protein unit 52 is present on a further vector. In this case, the self-replicating vector 51 and a vector 56 containing the replication initiator protein unit 52 are both introduced into a subject cell 50 as the first vector 51 and the second vector 56, respectively. This introducion may be through a biochemical or physicochemical method mentioned above. When the second vector 56 is introduced into the subject cell, the sequence 54 which encodes a replication initiator protein is expressed, which is transcribed and translated, and the replication initiator protein is generated. The replication initiator protein recognizes the replication initiator sequence 3 and binds thereto. Further, two or more proteins relating to the replication of DNA contained in the subject cell bind thereto. The self-replicating vector 51 is replicated in the subject cell by these bound proteins.

The second vector 56 here may be called a replication initiator protein gene expression vector. This vector may contain a replication initiator protein unit to express a replication initiator protein. Further, the replication initiator protein gene expression vector may be a vector which can express a replication initiator protein in a cell into which a self-replicating vector is to be introduced. An example of the vector may be a vector well-known in itself, such as a plasmid vector or a viral vector or a vector which has a self-replicating function in itself.

The self-replicating vector and the replication initiator protein gene expression vector may be introduced to the subject cell at the same time. Or the replication initiator protein gene expression vector may be introduced into the subject cell prior to the introducion of the self-replicating vector, or the self-replicating vector may be introduced into the subject cell prior to the introducion of the replication initiator protein gene expression vector.

Thus, with use of a self-replicating vector and a replication initiator protein gene expression vector together in combination, the reporter protein can be effectively expressed and the self-replicating vector can effectively self-replicate even if, for example, the promoter activity is low or the subject cell is of a low proliferative property type.

### First Embodiment

In the first to fourth example vectors described above, one reporter gene expression unit is contained in one self-replicating vector. But, the number of reporter gene expression units contained in one self-replicating vector is not limited to one. In other words, a self-replicating vector may include at least one reporter gene expression unit and replication initiator sequence. Here, an example in which two reporter gene expression units are contained will be described as an embodiment in which two or more reporter gene expression units are contained.

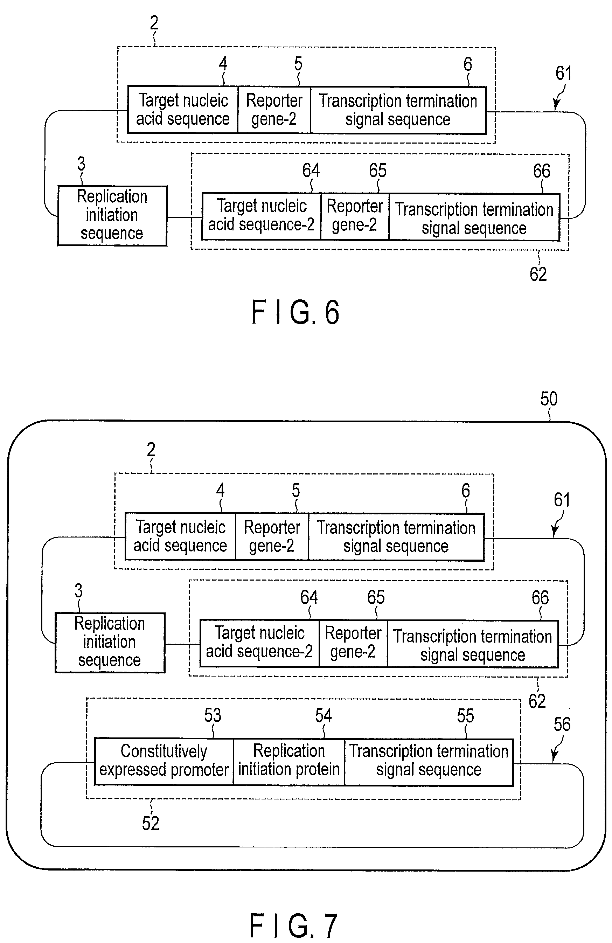

One further example of the self-replicating vector will be described with reference to FIG. 6. A self-replicating vector 61 includes a reporter gene expression unit 2, which is the first reporter gene expression unit, a reporter gene expression unit 62, which is the second reporter gene expression unit, and a replication initiator sequence 3. The replication initiator sequence 3 is present on the same nucleic acid as that of the reporter gene expression units 2 and 62.

The reporter gene expression unit 62 includes a target nucleic acid sequence 64, a reporter gene 65 which encodes a reporter protein and a transcription termination signal sequence 66.

The target nucleic acid sequences 4 and 64 may be of the same nucleic acid sequences, or may be of different sequences from each other. The reporter genes 5 and 65 contained in the reporter gene expression unit are of different reporter genes from each other. As also disclosed herein, the reporter genes contained in the reporter gene expression unit may be of the same reporter genes.

The transcription termination signal sequences 6 and 66 may be of the same sequences, or may be of different sequences from each other.

As for the self-replicating vector 61 shown in FIG. 6, the reporter gene expression unit 62 is linked downstream of the reporter gene expression unit 2, and the replication initiator sequence 3 is linked downstream thereof. But, the structure of the self-replicating vector 61 is not limited to that of the embodiment shown in FIG. 6. For example, the replication initiator sequence 3 may be linked downstream of the reporter gene expression unit 2 and the reporter gene expression unit 62 may be further linked downstream thereof. Or the reporter gene expression unit 2 may be linked downstream of the reporter gene expression unit 62, and the replication initiator sequence 3 may be further linked downstream thereof. Or the replication initiator sequence 3 may be linked downstream of the reporter gene expression unit 62, and the reporter gene expression unit 2 may be further linked downstream thereof.

Moreover, the directions of the reporter gene expression unit and the replication initiator sequence are not limited to those of the embodiment shown in FIG. 6. For example, the reporter gene expression unit 2 and the reporter gene expression unit 62 may be directed in opposite directions. That is, the linkage may be made so that the reporter gene of the reporter gene expression unit 62 is transcribed in a direction opposite to that of the reporter gene of the reporter gene expression unit 2. Or the linkage may be made so that the reporter gene of the reporter gene expression unit 62 is transcribed in the same direction as that of the reporter gene of the reporter gene expression unit 2. Further, the transcribed direction of the replication initiator sequence 3 may be the same or opposite direction as or to that of the reporter gene expression unit 2 and/or the reporter gene expression unit 62.

In all of the reporter gene expression unit 2, the reporter gene expression unit 62 and the replication initiator protein unit 52 used at the same time in the example shown in FIG. 6, the expressions may be controlled by one promoter. Or the expressions may be controlled by three promoters independent of each other. Or two of the three configurations may be controlled by one promoter, and the other one may be controlled by a further promoter independent from the other.

A preferable example thereof will be described. That is, the self-replicating vector 61 is designed so that the target nucleic acid sequences 4 and 64 may include the same sequence. Here, the states of the functional groups in the target nucleic acid sequences 4 and 64 are made to differ from each other. For example, in the reporter gene expression unit 2, a target nucleic acid sequence 4 in which the functional groups of nucleic acid are not substituted is located, and in the reporter gene expression unit 62, the target nucleic acid sequence 64 in which the functional groups of nucleic acid have been substituted is located. Further, the reporter gene expression units 2 and 62 are designed to comprise, respectively, reporter genes which encode different reporter proteins from each other.

In the example discussed above, the difference between the reporter gene expression units 2 and 62 is whether or not there is substitution of the functional group of the nucleic acid contained in the target nucleic acid sequence 4 or the degree of substitution, and the type of reporter gene. More specifically, in the self-replicating vector 61, the functional group of the nucleic acid contained in the target nucleic acid sequence 4 is not substituted, but the functional group of the nucleic acid contained in the target nucleic acid sequence 64 is substituted beforehand. By using such a self-replicating vector, it is possible to acquire two different kinds of detection signals from the reporter gene expression units 2 and 62, respectively. With this structure, it becomes possible to identify false-positive and false-negative signals. Thus, a highly precise assay can be realized.

As for the details of each element contained in the reporter gene expression unit 62, the explanation made for each element contained in the reporter gene expression unit 2 may referred to.

Moreover, the second reporter gene expression unit may be contained in the first to fourth examples of the self-replicating vector disclosed above.

A further example of the plasmid vector will be described with reference to FIG. 7. FIG. 7 shows an example in which the replication initiator protein gene expression vector discussed in the fourth example vector and the technique of the first embodiment are used together in combination. The details of these are described in the fourth example vector and first embodiment provided above.

### 2. Method of Obtaining Epigenetic Genetic Information of Subject Cell

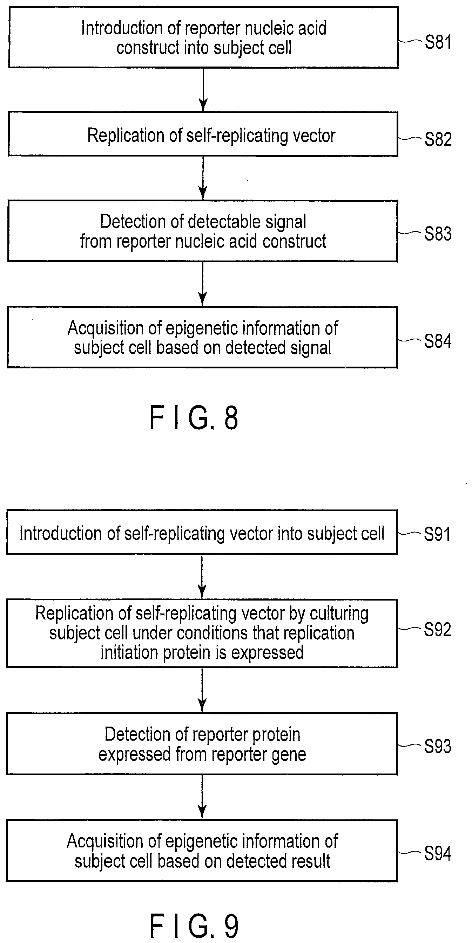

A method of obtaining epigenetic genetic information of a cell is described. How to obtain epigenetic genetic information of a subject cell will be described with reference to FIG. 8.

The method shown in FIG. 8 uses a self-replicative reporter nucleic acid construct. As mentioned above, this reporter nucleic acid construct copies the state of modification in a specific sequence on the genome of a subject cell onto the sequence of itself during its self-replication in the nuclear of the cell by substitution of a functional group. Then, a detectable signal is produced depending on the state of presence of the functional group copied by the reporter nucleic acid construct. According to this method, first, such a reporter nucleic acid construct is introduced into a subject cell (S81). Then, the reporter nucleic acid construct is allowed to self-replicate (S82). Next, the presence of a signal produced in the subject cell and/or the size thereof are detected (S83). Based on the obtained result, the epigenetic information on the subject cell is acquired (S84). In this manner, epigenetic information of a cell can be acquired simply.

A further method will be described with reference to FIG. 9. FIG. 9 shows an example in which the self-replicating vector is used as the reporter nucleic acid construct. First, a self-replicating vector is introduced into, for example, the nuclear of a subject cell (S91). Next, the replication of the self-replicating vector is carried out by culturing a subject cell under the conditions which replication initiator protein has expressed (S92). After a predetermined time elapses, the reporter protein expressed from the reporter gene is detected (S93). This detection may be based on the presence/absence of the reporter protein and/or the amount of expression thereof. Further, the "predetermined time" may be, for example, the time required for the subject cell to divide. The self-replication of self-replicating vector occurs when a subject cell is divided. Therefore, a subject cell may divide at least once during the predetermined time. From this detection result, the epigenetic information of the subject cell is acquired (S94). With this method, it is also possible to determine in what sort of state the subject cell is by comparing with a table of association provided in advance. Such a table may be one that indicates, for example, association between the status of a cell or the pathology thereof, and the presence/absence of expression of a reporter protein. Or what sort of concrete epigenetic information the epigenetic information of the subject cell is may be determined from the obtained detection result. These procedures may be performed in vitro or in vivo.

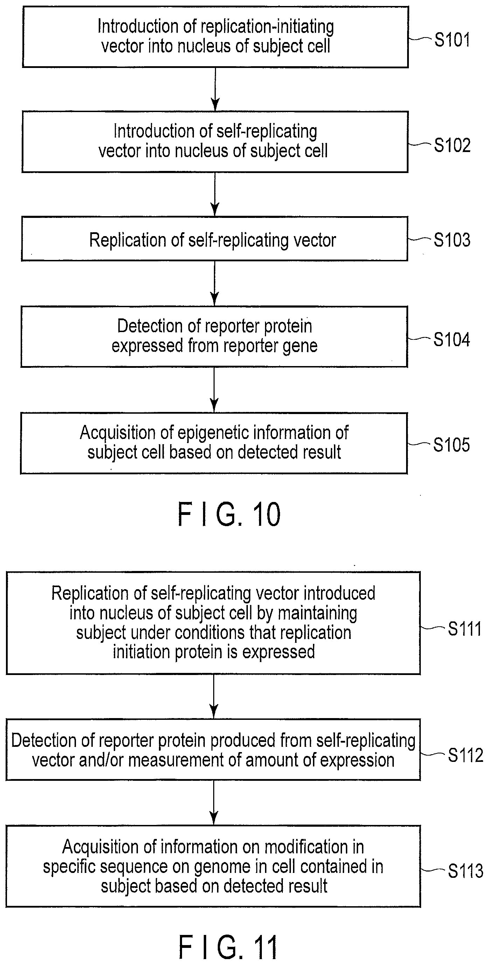

A further example will be described with reference to FIG. 10. In this method, first, a replication initiator protein gene expression vector is introduced in the nuclear of a subject cell (S101). Next, a self-replicating vector is introduced in the nuclear of the subject cell (S102). Then, the subject cell is incubated, and thus the self-replicating vector is allowed to self-replicate (S103). After a predetermined time elapses, the reporter protein expressed from the reporter gene is detected (S104). From this detection result, the epigenetic information of the subject cell is acquired (S105).

A further example will be described with reference to FIG. 11. In this method, by maintaining a self-replicating vector in a subject into whose cell's nuclear the self-replicating vector is introduced under conditions that a replication initiator protein is expressed, the introduced self-replicating vector discussed above is allowed to self-replicate (Sill). Next, the subject is examined in term of detection of the reporter protein produced from the self-replicating vector and/or measurement of the amount of expression thereof (S112). Based on the result, the information on the modification in a specific sequence on the genome in the cell contained in the subject is acquired (S113).

Examples of the reporter protein may be luciferase, β-galactosidase, nitric-monoxide-synthase, xanthine oxidase, blue fluorescent protein, green fluorescent protein, red fluorescence protein and a heavy metal binding protein. Here, appropriate detection and/or measuring methods may be selected according to the type of the reporter protein selected.

The detection of a reporter protein will now be described in detail. As mentioned above, a photoprotein gene, a fluorescent protein gene, a coloring protein gene, an active-oxygen producing enzyme gene, a heavy-metal-binding protein gene and the like can be selected as a reporter gene.

The photoprotein gene is a gene which encodes an enzyme protein which catalyzes the photogenic reaction. An example of the photoprotein gene is a luciferase gene. Luciferase, which is a product of translation of a luciferase gene, carries out a photogenic reaction using luciferin, which is a type of substrate.

The fluorescence protein gene is a gene which encodes a fluorescent protein. Examples of the fluorescent protein gene are a blue fluorescent protein gene, a green fluorescent protein gene and a red fluorescent protein gene.

The coloring protein gene is a gene which encodes a enzyme protein which catalyzes a coloring reaction. An example of the coloring protein gene is a β-galactosidase gene. β-galactosidase, which is a product of translation of a β-galactosidase gene, carries out a color reaction using a substrate, such as 5-bromo-4-chloro-3-indolyl-β-D-galactopyranoside (X-Gal) or o-nitrophenyl-β-D-galactopyranoside (ONPG).

Translation products of a photoprotein gene, a fluorescent protein gene and a coloring protein gene are detectable with an optical detector. More specifically, in the case where the reporter gene is a luciferase gene, a luciferase protein may be extracted from a subject cell and a substrate be added, and then the luminescent intensity of the solution may be measured using a luminometer. When the reporter gene is a fluorescent protein gene, for example, a subject cell is irradiated with the light of a specific wavelength and the intensity of the fluorescence produced by the fluorescence protein in the subject cell may be measured with a fluorometry device. Or when the reporter gene is a fluorescent protein gene, the extract containing the fluorescence protein extracted from the subject cell is irradiated with the light of a specific wavelength, and the intensity of the fluorescence produced by the fluorescent protein in the extract may be measured with a fluorometry device. When the reporter gene is a β-galactosidase gene, a β-galactosidase protein is extracted and a substrate is added, and then the light absorbance of the solution is measured using a spectrometry device.

The active oxygen producing enzyme gene is a gene which encodes an enzyme which produces active oxygen. Examples of the active oxygen generation enzyme gene are a nitric-oxide-synthase gene and a xanthine-oxidase gene. Each of nitric-oxide-synthase, which is a product of translation of a nitric-oxide-synthase gene, and xanthine oxidase, which is a product of translation of a xanthine-oxidase gene, generates active oxygen.

Active oxygen is detectable with an electron spin resonance (ESR) device or the like. With the method using an ESR device, the amount of production of active oxygen may be measured directly or by using a specific spin trap agent. When using a spin trap agent, first, the active oxygen generation enzyme is trapped with the spin trap agent, and then the amount of production of the active oxygen generated from the trapped active oxygen generation enzyme is measured.

The heavy-metal-binding protein gene is a gene which encodes a protein binds to a heavy metal specifically. The amount of generation of protein bonded to the heavy metal may be measured by a magnetic resonance imaging system, a nuclear-medicine-diagnosing device or a computerized transverse axial tomography.

The amount of production of heavy-metal-binding protein is measured, for example, as follows. First, a measurable heavy metal is added to a culture medium of a subject cell in advance. Next, when needed, the subject cell is washed, and thereafter the heavy-metal-binding protein is extracted from the subject cell. Subsequently, an image of the extracted heavy metal binding protein is picked up with a diagnostic imaging device compatible for the heavy metal added to the medium, to measure the quantity of the heavy-metal-binding protein. Note that a heavy metal binding protein may be expressed inside a subject cell or on an outer surface of a subject cell.

Examples of the heavy-metal-binding protein gene may be base sequences which encode metallic-compound-binding peptides. For example, the metallic-compound-binding protein may be a peptide, oligopeptide, polypeptide and/or protein which specifically bind to a specific metallic compound.

For example, as the sequence which encodes a peptide which binds to a metallic compound, a base sequence of an antibody gene or a single strand antibody gene, known to bind to a desired metallic compound, may be used, and the sequence may only be designed based on such a base sequence. Such a base sequence may be designed by modification and/or alteration such as substitution, deletion and addition, of one or some bases in the range in which the binding of the metallic compound is maintained, or according to the object to which such modification and/or alteration is applied.

The gene which encodes a single-strand antibody peptide can be designed from the amino acid sequence of the antibody binding to the metallic compound.

For example, in an MRI imaging, a gadolinium compound is used preferably because of its high contrasting effect. Examples of the gadolinium compound may be gadolinium, gadolinium ion, a gadolinium complex, a salt and a derivative of any of these, a derivative containing any of these, and a metallic compound formed of an analogue or the like of the gadolinium compound.

Further, a reporter gene which exhibits a metallic-compound-binding capability to the outside of a cell may be used. Such a reporter gene may be a base sequence which encodes a metallic-compound-binding peptide exhibited extracellularly. Such a base sequence may be of the following nature. That is, for example, the sequence may be constructed such that it may be transcribed and translated in a cell and produces a metallic-compound-binding peptide, and then, the produced metallic-compound-binding peptide migrates to the cell membrane and exhibits a metallic-compound-binding capability extracellularly. An example of such a reporter gene is disclosed in, for example, JPA 2012-200245. As indicated in this document, such a reporter gene may only comprise a base sequence which encodes a metallic-compound-binding peptide, a base sequence which encodes a signal peptide which conveys the metallic-compound-binding peptide to a cell membrane, and a base sequence which encodes the anchor peptide which immobilizes the metallic-compound-binding peptide conveyed to the cell membrane by the signal peptide to the cell membrane.

### 3. Method of determing characteristics of subject cell

It is also possible to determine the characteristics of a subject cell using a method of obtaining epigenetic genetic information of the procedure subject cell. The method of determining the characteristics of a subject cell includes determining the characteristics of a subject cell based on the epigenetic genetic information of a subject cell.

The characteristics of a subject cell may be, for example, drug sensitivity, drug adaptability.

Further, determination of the characteristics of a subject cell may be determining whether or not the subject cell is in a specific state. The determination as to whether a subject cell is in a specific state may be performed in vitro or in vivo.

For example, the method of determining the characteristics of a cell may be a method of determining the drug sensitivity regarding a subject from which the subject cell is derived, a method of selecting the kind of drug or immunotherapy agent which should be used after a surgical operation, regarding a subject from which the subject cell is derived, or a method of determining whether or not a patient's prognosis is good. Further, for example, the method of determining the characteristics of a cell may be that of obtaining information for making these judgments.

Here, the "specific state" may be a specific health condition, for example, the state of being affected by a specific disease. The "specific disease" may be cancer, a psychiatric disorder, a lifestyle-related disease, a neurological disorder, an autoimmune disease, a cardiovascular disease, for example. Or the method may be that of checking whether or not the methylation state of an organ induced to be differentiated from a stem cell in regenerative medicine is normal.

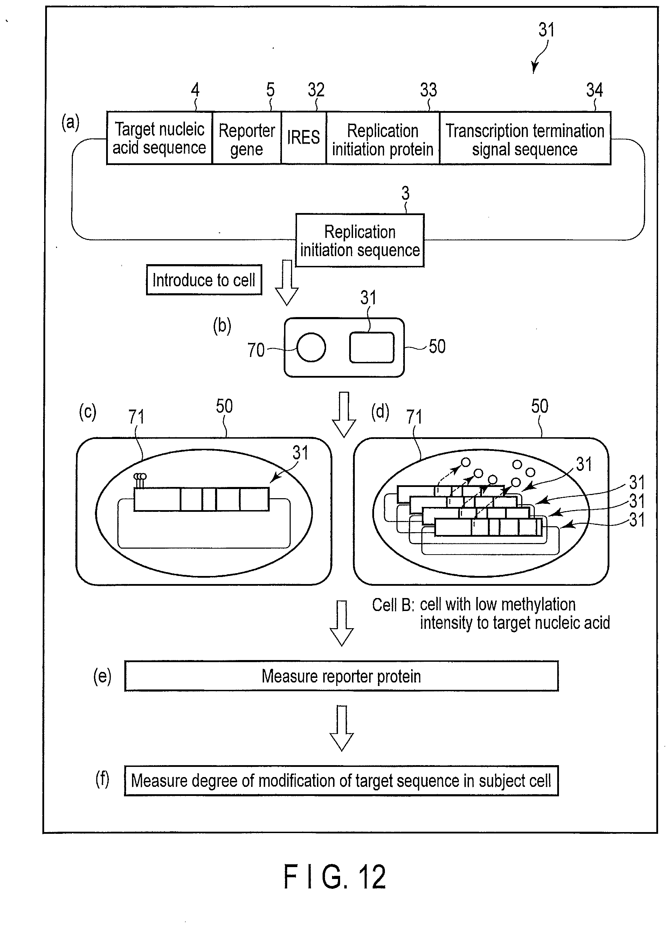

An example of the method of determining the characteristics of a cell based on epigenetic genetic information using the second example vector will now be described with reference to FIG. 12. First, a self-replicating vector 31 comprising a desired target nucleic acid sequence 4 is prepared (a). Next, the self-replicating vector 31 is introduced into a living subject cell 50 containing a nuclear 71 (b). Then, the state is maintained so that the self-replicating vector 31 is self-replicative in the subject cell 50 according to a condition (b). For example, under such a culture condition that a subject cell can divide and proliferate in the state that a replication initiator protein is present, the subject cell is cultured for an arbitrary period.

In the self-replicating vector 31 of the nuclear 71, the modification state of a target nucleic acid sequence in the nuclear 71 of the subject cell 50 is reflected in the state of the modification of the modified base of the target nucleic acid sequence 4. As a result, for example, when the degree of the methylation of the target nucleic acid sequence is high, the degree of the replication of the self-replicating vector 31 becomes lower (c). On the other hand, the degree of the replication of the self-replicating vector 31 becomes high when the degree of the methylation of the target nucleic acid sequence becomes higher (d).

Next, the amount of the reporter protein is measured (e). When the result indicates that the amount of the reporter protein is large, the base to be modified in a gene sequence homologous to the target nucleic acid sequence 4 is not methylated or modified in the subject cell 50, and thus the subject cell 50 is judged to have a low magnitude in methylation (f). Or when the result indicates that the amount of the reporter protein is low, the subject cell 50 is judged as a cell in which the modifiable base in the gene sequence homologous to the target nucleic acid sequence 4 is methylated (f).

In the self-replicating vector, the modification state thereof is reflected in the modification state of the modified (or modifiable) base of the target nucleic acid sequence 4 according to the modification state of the target nucleic acid sequence present in the subject cell during the self-replication. The modification state of the target nucleic acid sequence 4 can be determined by detecting or quantifying the reporter protein.

As a further example of the method of determining the characteristics of a cell based on epigenetic genetic information, the method which uses the self-replicating vector 61, shown in FIG. 6, will be described. First, a self-replicating vector 61 containing desired target nucleic acid sequences 4 and 64 is prepared. Next, the self-replicating vector 61 is introduced into the subject cell 50. Then, the state is maintained so that the self-replicating vector 61 is self-replicative in the subject cell 50 according to a condition (b). For example, under such a culture condition that a subject cell can divide and proliferate in the state that a replication initiator protein is present, the subject cell is cultured for an arbitrary period.

When the self-replicating vector 61 self-replicates in the subject cell, the modification state of the nucleic acid sequence on the genome of the subject cell 50 is reflected in the state of the modification of the modified (or modifiable) bases of the target nucleic acid sequences 4 and 64. As a result, for example, when the degree of the methylation of the target nucleic acid sequence is high, the amount of expression of reporter protein (reporter gene 5) from the reporter gene expression unit 2 containing the target nucleic acid sequence 4 which is not methylated decreases gradually. On the other hand, the amount of expression of the reporter protein (reporter gene 65) from the reporter gene expression unit 62 containing the methylated target nucleic acid sequence 64 does not change.

Next, the amounts of the reporter proteins are measured to obtain a ratio in amount of the reporter proteins from the reporter genes 5 and 65. When the ratio is small, it is judged that in the subject cell 50, the base to be modified in a nucleic acid sequence analogous to the target nucleic acid sequence 4 or 64 is methylated or modified. Therefore, the target cell is further judged as a cell having a high magnitude in methylation.

In the self-replicating vector 61, the modification state thereof is reflected in the modification states of the modified bases of the target nucleic acid sequences 4 and 64 according to the modification state of the target nucleic acid sequence present in the subject cell during the self-replication. The modification states of the target nucleic acid sequences can be determined by detecting or quantifying the reporter proteins from the reporter genes 5 and 65 and obtaining the ratio therebetween.

Here, it is also possible to provide a method of determining whether the possibility that a subject from which a cell is derived, will be affected by a specific disease is high or low by further using the epigenetic genetic information of an cell obtained as described. Moreover, information for final diagnosis may be collected based on the epigenetic genetic information of a cell obtained as above.

### 4. Diagnostic Method

A specific disease in a subject may be diagnosed based on epigenetic genetic information of a cell obtained as described above. In general, such diagnosis may finally include a judgment made by a specialist such as a medical practitioner or veterinarian.

Here, the term "subject" may be an individual of a mammal including a human being, livestock, a pet or an industrial animal, or may be an organ, an internal organ, tissue, a cell group and/or a single cell obtained from the subject.

In the diagnostic method, a self-replicating vector is introduced into an individual, an organ, an internal organ, a tissue, a cell group and/or a single cell, etc., which are the subjects for detection. Next, a reporter protein may be detected or quantified.

In the diagnostic method, a self-replicating vector which contains a photoprotein gene as a reporter gene may be judged, for example, as follows. That is, a self-replicating vector is introduced by a biochemical or physicochemical process to an individual, an organ, an internal organ, a tissue, a cell group, a single cell or the like, which is a subject for detection. At the same time as or before and after the introducion, luciferase is detected, which is a translation product of a luciferase gene depending on the state of modification of the modified base. Then, after extracting luciferase protein from the subjedt for detection and adding luciferin, which is a type of substrate, the luminescence intensity of the solution may be measured using a luminometer. That is, according to this detection method, it is possible to specifically detect a cell in a specific situation using the amount of translation of luciferase as an index. Such a diagnostic method is of a low invasive type.

Here, the detection of luciferase may be performed at one time, a plurality of times, continuously or occasionally over time. The diagnostic method is not limited to such a process which utilizes detection of luciferase, but may be performed using a reporter nucleic acid construct which produces other detectable signal.

A method of determining whether or not a subject of detection is affected by a specific disease using an organ, an internal organ, a tissue, a cell group, a single cell or the like extracted from an individual will now be described.

Such a method may comprise, for example:
(1) incorporating to a subject of detection containing a cell, a self-replicating vector containing a gene which produces a detectable signal as a reporter gene, under a conditions that a replication initiator protein can be expressed;
(2) culturing the subject of detection over an arbitrary time period under a condition that the cell as the subject of detection in itself or the cell contained in the object of detection can divide and proliferate;
(3) detecting the signal produced from the subject of detection, and
(4) determining whether the subject of detection is affected by a specific disease based on the signal detected.

Moreover, such a method may comprise, for example:
(1) incorporating to a subject of detection containing a cell, a self-replicating vector containing a luciferase gene as a reporter gene, under a conditions that a replication initiator protein can be expressed;
(2) culturing the object of detection over an arbitrary time period under a condition that the cell as the subject of detection in itself or the cell contained in the object of detection can divide and proliferate;
(3) extracting luciferase protein from the subject of detection; and
(4) contacting the luciferase protein with luciferin, which is a substrate thereof, thereby detecting luminescence with a luminometer;
(5) determining whether the subject of detection is affected by a specific disease based on the photogenesis detected.

Before the extraction of the luciferase protein in (3) above, the cell may be washed, for example. With such embodiment method, it becomes possible to detect the luciferase protein translated according to the situation of the cell.

The determination regarding the subject based on the detection of luciferase may be made based on such information as whether or not luciferase has been detected, or whether the detection value of luciferase is larger or smaller than a predetermined threshold, or increase/decrease in detection value. Based on such information, for example, whether a subject of detection is affected by a disease under a specific condition set as an index, the severity of a disease, etc., should only be judged.

In the diagnostic method, a self-replicating vector which contain a reporter gene exhibiting a metallic compound binding capability extracellularly as a reporter gene, may be judged as follows, for example. A self-replicating vector is introduced by a biochemical or physicochemical process to an individual, an organ, an internal organ, a tissue, a cell group, and/or a single cell, etc. which are the subjects of detection. At the same time as and/or before and after the introducion, a metallic compound is contacted, which forms a binding pair with the reporter protein extracellularly exhibited depending on the state of the modification of the target nucleic acid sequence concerned. At the same time as and/or before and after the contact of the metallic compound, the metallic compound specifically bound to the reporter protein is detected. That is, according to this detection method, it is possible to specifically detect a cell in a specific situation based on the presence of a metallic compound as an index.

Here, the detection of a metallic compound may be performed at one time, a plurality of times and/or continuously and/or occasionally over time.

Further, the detection of a metallic compound may be performed according to the kind of metal atom contained in the metallic compound using any method conventionally known in itself. For example, a chemical, physical, physicochemical and/or biochemical method of detecting a metal element based on the chemical and/or physical properties of the metal atom may be used.

Moreover, for example, the metallic compound for specifically forming a bonding pair with a reporter protein may be selected from those of the measurable types with diagnostic imaging devices such as MRI, PET, SPECT and CT. In this manner, the self-replicating vector can be utilized in combination with the diagnostic imaging device. Thus, it becomes possible to detect a cell with higher sensitivity and specificity.

The method of determining whether a subject of detection is affected by a specific disease, may comprise:
(1) incorporating a self-replicating vector which contain a reporter gene extracellularly exhibiting a metallic-compound-binding capability as a reporter gene into a cell as a subject of detection or a subject of detection containing a cell;
(2) exhibiting a metallic-compound-binding peptide extracellularly;
(3) contacting a metal which can form a binding pair with metallic-compound-binding peptide, to the metallic-compound-binding peptide;
(4) detecting the metal bound to the metallic-compound-binding peptide; and
(5) determining whether the subject of detection is affected by the specific disease based on a result of the detection.

At the same time as and/or before and after the contact of the metallic compound in (3), such proceeding as to remove excessive metal elements which have not been bound, that is, for example, washing, rinsing, dilution and/or circulating current elimination may be performed.

With this method, it becomes possible to bind a desired metal on a cell surface by the metallic compound-binding capability extracellularly exhibited according to the situation of the cell. Here, it is possible to detect a cell in a specific condition by using the metallic compound-binding capability extracellularly exhibited and binding and/or accumulating metallic compounds thereto.

The method of determining whether the subject of detection is affected by a specific disease based on the result of the detection of metal is, for example, as follows. That is, the determination may be based on such information as to whether the presence of metal is detected, whether the detection value of the metal is larger or smaller than a predetermined threshold or increase/decrease in detection value. Based on the information, further, whether the subject of detection is affected by a disease of a specific condition set as an index, the severity of the disease, etc., should only be determined.

### 5. Assay Kit

As a further embodiment, an assay kit is provided. An assay kit may include at least one kind of reporter nucleic acid construct, for example, at least one kind of self-replicating vector as described above. The assay kit may include one kind of self-replicating vector, or a combination of two or more kinds of self-replicating vectors. The assay kit may further contain a carrier well-known in itself supporting a self-replicating vector and/or a container well-known in itself including a self-vector replicating vector, and a replication initiator protein gene expression vector. Furthermore, the assay kit may also include any reagent required to carry out the method of obtaining epigenetic information of a cell, or the method of determining the characteristics of a cell.

### 6. Composition

Moreover, the reporter nucleic acid construct may be provided as a composition. An example of the composition contains at least one kind of self-replicating vector according to embodiment, for example.

One example of the composition may also contain one kind of self-replicating vector, or may also contain a combination of two or more kinds of self-replicating vectors. Further, the composition may also contain a carrier well-known in itself which supports a self-replicating vector, a replication initiator protein gene expression vector, an excipient, a stabilizer and/or an ingredient of other desired effect. The composition may be contained in a container when provided.

### 7. Device

Also disclosed herein is an analytic device for carrying out the above-described methods, for example, the method of obtaining epigenetic information, and the method of determining the characteristics of a cell, which uses it, etc. will be provided.

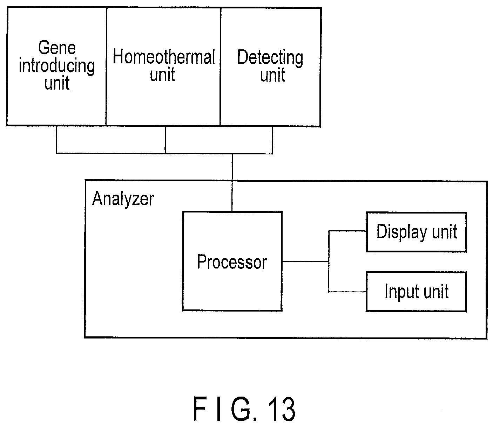

One example of the analytic device will be described with reference to FIG. 13. The analytic device contains a gene introducion unit, a homeothermal unit adjacent thereto, a detection unit adjacent thereto and an analyzer electrically connected to these. In the gene introducion unit, a self-replicating vector is introduced into the nuclear of a subject cell. In the thermo regulating unit, the self-replicating vector is allowed to self-replicate within the nuclear of the subject cell. In the detection unit, a reporter protein produced in the cell is detected. Information obtained by the detection unit is sent to the analyzer. The analyzer may comprises a processor, a display unit and an input unit. In the analyzer, the information obtained in the detection unit may be subjected to data processing by the processor and displayed on the display unit when needed. That is, the result obtained by the detection unit may be sent to the processor of the analyzer, to be analyzed, calculated and processed according to the predetermined procedure, and indicated on the display unit.

The inside of the gene introducion unit, the thermo regulating unit and the detection unit are communicated to each other via connection windows so that processing can be continuously carried out. After the subject cell is passed to one unit to the following unit, the respective connection window is closed by a closure shield.

The procedure of the method of obtaining epigenetic information with the analytic device is as follows. First, an experimenter places a container containing a subject cell and a self-replicating vector into the gene introducion unit. Then, the instruction to start the analysis is entered from the input unit. The instruction from the input unit is sent to the processor and with an instruction from the processor, a treatment to enabling the introducion of the self-replicating vector to the subject cell of performed in the gene introducion unit. After a predetermined time elapses, the introducion process is finished and the subject cell accommodated in the container is sent to the thermo regulating unit. In the thermo regulating unit, the subject cell is maintained under predetermined conditions for a predetermined time. Thus, the self-replicating vector self-replicates. After the predetermined time elapses, the subject cell accommodated in the container is sent to the detection unit from the thermo regulating unit. In the detection unit, the reporter protein expressed within the subject cell is detected. The detected information is sent to the analyzer and subjected to data processing by the processor. The information thus obtained is shown in the display unit. The container is moved to the gene introducion unit, the thermo regulating unit and the detection unit by a transportation means such as a conveyor belt, a movable arm and/or a movable tray, etc. All of these movements are performed under control of the processor according to the directions of the processor based on a preset program. The gene introducion unit has a configuration required for the gene introducion by a selected method. The thermo regulating unit has a configuration required in order to fulfill conditions required for the self-replicating vector to self-replicate. The detection unit has a configuration required for detection of a reporter protein according to the signal detected.

Such an analytic device is useful to acquire epigenetic information of a cell in a simple way.

### [Example]

### Reference Example 1

### Production of plasmid type self-replicating vector

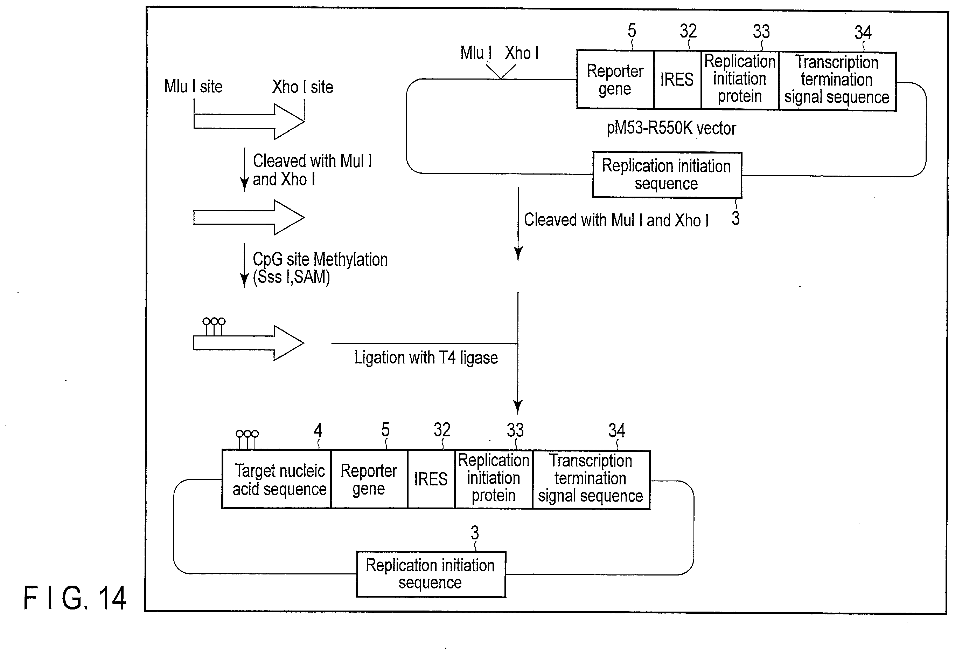

A plasmid type self-replicating vector including a methylated target nucleic acid sequence such as shown in FIG. 14 was produced. A GFAP gene-promoter sequence was selected as a target nucleic acid sequence. The GFAP gene-promoter sequence was prepared as a target nucleic acid sequence by carrying out amplification of template nucleic acid and cleaving it with a restriction enzyme. The obtained GFAP gene-promoter sequence is a nucleic acid sequence which contained a modified base and has promoter activity. Then, the cleaving with restriction enzyme was carried out and then this target nucleic acid sequence was methylated with methylation enzyme SssI. The obtained methylation target nucleic acid sequence was ligated with ligase to be incorporated to a pM53-R550K vector. The pM53-R550K vector is a vector which comprises a reporter gene, IRES, a sequence that encodes a replication initiator protein, a transcription termination signal sequence and a replication initiator sequence in this order from upstream to downstream. Thus obtained vector was a plasmid type self-replicating vector containing a methylated target nucleic acid sequence. The details of each processing step will be described.

### (1) Production of GFAP gene-promoter sequence which is a target nucleic acid sequence

Using mouse genomic DNA as a template, PCR was carried out. The sequence amplified in mouse genomic DNA is SEQ ID NO: 1 provided in TABLE 2.

Further, the base sequences of the primers used in the PCR were SEQ ID NOS: 11 and 12 as follow;
Forward primer:
   5'-CGACGCGTGTCTGTAAGCTGAAGACCTGGC-3' (SEQ ID NO: 11)
Reverse primer:
   5'-AAAAGTACTCCTGCCCTGCCTCTGCTGGCTC-3' (SEQ ID NO: 12).

Using the forward primer and reverse primer with mouse genomic DNA as the template, PCR was carried out, and a GFAP gene-promoter sequence represented by SEQ ID NO: 1 was obtained. The obtained GFAP gene-promoter sequence contains a modified base in its target nucleic acid sequence, and has a promoter activity which depends on the degree of the modification. The GFAP gene-promoter sequence is 5' upstream region (-1651bp to +32bp) of the mouse GFAP gene. Next, the cloning of GFAP gene-promoter sequence was performed with PGV-B2 (TOYO B-Net) vector.

The PGV-B2 (TOYO B-Net) vector was cleaved with restriction enzyme Sma I, which was then dephosphorylated. A phosphorylated polynucleotide represented by SEQ ID NO: 1 was ligated with T4 ligase to this vector. Thus, a PGV-B2-GFAPp vector was produced.

The obtained PGV-B2-GFAPp vector was to be used as a material for obtaining a GFAP gene-promoter sequence in the following process, and at the same time, it is to be used in a detection examination later mentioned for comparison as PGV-B2-GFAPp vector. Further, a methylated PGV-B2-GFAPp vector is also produced by methylating a GFAP gene-promoter sequence contained in the PGV-B2-GFAPp vector by a process mentioned later, which would be used in the detection examination for comparison. Both of methylated or non-methylated PGV-B2-GFAPp vector does not self-replicate.

In order to obtain a GFAP gene-promoter sequence, the PGV-B2-GFAPp vector was cleaved with restriction enzyme Mlu I and restriction enzyme Xho I. Thus, the GFAP gene-promoter sequence to which restriction enzyme Mlu I and restriction enzyme Xho I recognition sequence were added, was prepared. This sequence was used in the following process as a target nucleic acid sequence.

### (2) Methylation of base to be modified

The GFAP gene-promoter sequence was methylated and the modifiable base contained therein was methylated.

To 40 µm of a solution of the GFAP gene-promoter sequence nucleic acid produced in (1) above, 16 µL of 10xNE Buffer2, 8 µL of methylation enzyme SssI (New England Bio-lab Japan, Inc.), 8 µL of S-adenosylmethionine (SAM) (New England bio-lab Japan, Inc.) and 88 µL of ddH₂O were added, to prepare a reaction mixture. The mixture was subjected to an enzyme reaction at 37°C for 6 hours. After the methylation reaction, the resultant was incubated using a heat block at 65°C for 20 minutes, and thus Sss I was deactivated. Further, the resultant was purified using QIA quick PCR purification kit (Qiagen, Inc.), and thus a purified GFAP gene-promoter sequence was obtained. The base to methylated in the obtained sequence was methylated.

On the other hand, a purified GFAP gene-promoter sequence in which the modifiable base was not methylated was produced by a method similar to the above except that Sss I was not added to the reaction mixture, as a control sequence of non-methylation.

Further, methylation of the GFAP gene-promoter sequence contained in the PGV-B2-GFAPp vector obtained in (1)above was similarly carried out, and thus a methylated PGV-B2-GFAPp vector was obtained.

### (3) Functional linkage between plasmid type self-replicating vector pM53-R550K and GFAP gene-promoter sequence, a purified target nucleic acid sequences

A self-replicating vector pM53-R550K was cleaved with restriction enzyme Mlu I and restriction enzyme Xho I. Here, the purified GFAP gene-promoter sequence obtained in (2) above was ligated by T4 ligase. Thus, a pM53-R550 K-GFAPp vector was obtained. The pM53-R550 K-GFAPp vector included a target nucleic acid sequence. The target nucleic acid sequence was methylated.

By a similar method, the non-methylated control sequence obtained in (2) above was incorporated into the vector. Thus, a non-methylated pM53-R550 K-GFAPp vector was obtained as a control vector.

### Reference Example 2

Methylation of target nucleic acid sequence and detection of demethylation

A test was carried out to confirm that the degree of promoter activation varies depending on the state of modification of the base of the target nucleic acid sequence contained in the pM53-R550 K-GFAPp vector.

### (1) Vector used in the test

As an example, a methylated plasmid type self-replicating vector pM53-R550 K-GFAPp vector obtained by the above method and a non-methylated plasmid type self-replicating vector pM53-R550 K-GFAPp vector were used. These are self-replicative plasmid vectors.

As a comparative example, a methylated PGV-B2-GFAPp vector obtained by the above method and a non-methylated PGV-B2-GFAPp vector were used. These are non-self-replicative plasmid vectors.

As an internal control vector, β-galactosidase expression vector pcDNA4/V5-His/lacZ vector (Life Technologies, Inc.) was used.

### (2) Introducion of vector to glioma C6 cell

### (a) Introducion of vector to cell

PcDNA4/V5-His/lacZ vector, methylated or non-methylated PGV-B2-GFAPp vector, methylated or non-methylated pM53-R550 K-GFAPp vector were introduced into glioma C6 cell, respectively. The introducion was performed by the lipofection method. Lipofectamine 2000 (Life Technologies, Inc.) was used for the introducion of each of these vectors as a introducoin reagent. The lipofection was carried out according to a manual made by the manufacturer of the reagent. An outline is as follows. 0.45 µL of cation lipid (lipofectamine 2000) suspended in 25 µL of Opti-MEM and 25 µL of Opti-MEM containing each vector were mixed in a micro-tube, and thus a complex of lipofectamine/vector was formed. Here, the quantity of each vector contained in 25 µL of Opti-MEM was as follows. The pM53-R550 K-GFAPp or PGV-B-2-GFAPp contained 0.066 µg of DNA in 25 µL of Opti-MEM. The pcDNA4/V5-His/lacZ contained 0.033 µg of DNA in 25 µL of Opti-MEM.

### (b) Dissemination

After forming a lipofectamine/vector complex for each vector, each complex was added to the culture plate (96-well plate) in 50pL per well. Further, a suspension of glioma C6 cell solution to a RPMI1640 culture medium (containing immobilized FCS at 10%)(5 × 10⁵ cells/mL) was disseminated in 100µL per well.

### (c) Culture

Then, the plate was quietly agitated for 1 minute to mix the reaction mixture and cell solution together. After that, the mixture was subjected to adhesion culture under a 5% CO₂ atmosphere at 37°C for 24 hours, a 5% CO₂ atmosphere at 37°C.

### (2) Demethylation of modified base by 5-aza-2-deoxycytidine

After introducing each vector into the glioma C6 cell, the target nucleic acid sequence contained in each vector was subjected to demethylation reaction. The demethylation reaction was carried out using 5-aza-2-deoxycytidine (5-aza-dC) (Funakoshi Co., Ltd.), which is a inhibitor of a methyltransferase.

The 5-aza-dC reagent was prepared as follows. 5-aza-dC was added to PBS, and thus a 5-aza-dC diluent having a concentration of 500 µM was prepared. The 5-aza-dC diluent was added to a fresh RPMI1640 culture medium to prepare a solution containing 5 µM 5-aza-dC. The obtained solution was used as a 5-aza-dC reagent.

Demethylation was carried out as follows. First, the culture medium of each well was removed after culture of (c). Then, the 5-aza-dC reagent was added in 200 µL each. As a control, which is without a demethylation reaction, 200 µL of a culture media, RPMI1640L, in which PBS was added in place of 5-aza-dC reagent, were added. The resultant was further subjected to adhesion culture in a 5% CO₂ atmosphere at 37°C for 48 hours.

### (3) Reporter gene assay (luciferase assay)

The culture medium was removed from the culture plate 48 hours after the addition of 5-aza-dC. Then, the cells were washed twice with PBS and luciferase (reporter protein) was extracted from these each cells. More specifically, the extraction was carried out as follows. That is, a cytolysis liquid (PicaGene Cell lysis buffer LCβ, TOYO B-Net Co. Ltd.), which is an extracting reagent, was added and the suspension of the cells and cytolysis liquid was incubated for 15 minutes at room temperature. Then, this suspension was centrifuged with a centrifugal separator at 15,000 rpm for 5 minutes. Thus, the cell residue was removed from the suspension, and a supernatant was obtained. A luciferase substrate solution (PicaGene LT2.0, TOYO B-Net Co. Ltd.) was added to the supernatant. Next, the luciferase substrate solution (luciferin solution), which is a detector reagent was added. The intensity of luminescence generated was measured with a luminometer (Mithras LB940, Berthold Co. Ltd.).

### (4) Results

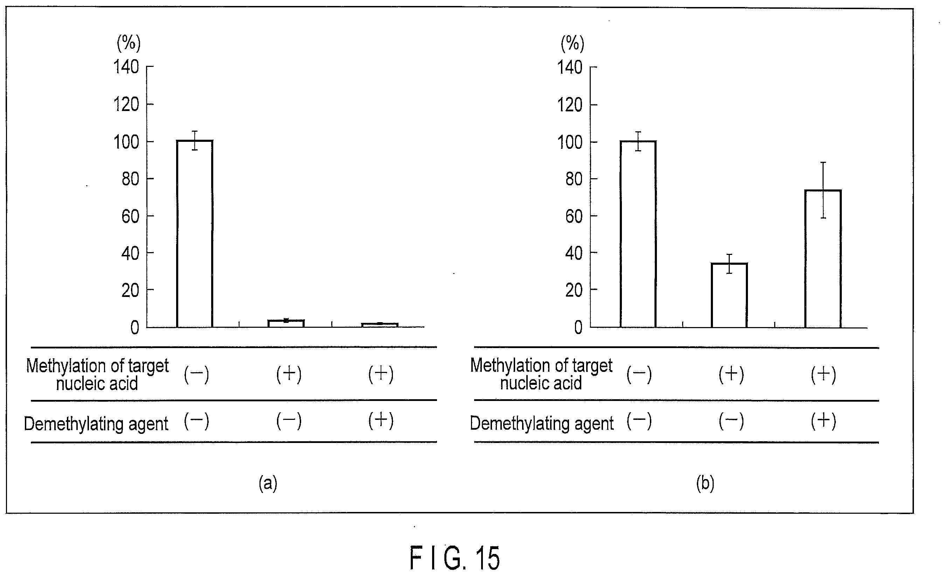

The results are shown in FIG. 15 (a) and (b).

These graphs indicate changes caused by the demethylation reaction in the signal strength of reporter protein from the vector-introduced cell.

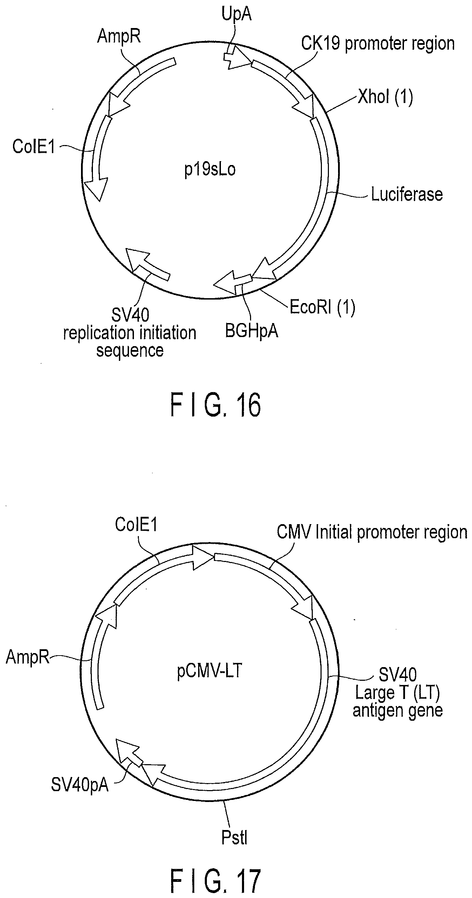

FIG. 16(a) shows the data measured with regard to the cell introduced with the PGV-B2-GFAPp vector. In the left end of FIG. 16(a), the signal strength obtained from a cell which was introduced with a PGV-B2-GFAPp vector containing a target nucleic acid sequence to be non-methylated and not subjected to a demethylation treatment was indicated as 100%. In the center, the relative signal strength obtained from the cell which was introduced with a PGV-B2-GFAPp vector containing a nucleic acid sequence to be methylated and not subjected to a demethylation treatment was indicated. The relative signal strength was 3.4%. In the right end, the relative signal strength obtained from the cell which was introduced with a PGV-B2-GFAPp vector containing a methylated nucleic acid sequence and subjected to a demethylation treatment was indicated. The relative signal strength was 2.4%.

FIG. 15 (b) shows the data measured with regard to the cell introduced with the pM53-R550 K-GFAPp vector. At the left end of FIG. 15 (b), the signal strength obtained from a cell which was introduced with a pM53-R550 K-GFAPp vector containing a non-methylated base to be modified and not subjected to a demethylation treatment was indicated as 100%. In the center, the relative signal strength obtained from the cell which was introduced with a pM53-R550 K-GFAPp vector containing a methylated base to be modified and not subjected to a demethylation treatment was indicated. The relative signal strength was 34.1%. At the right end, the relative signal strength obtained from the cell which was introduced with a pM53-R550 K-GFAPp vector containing a methylated base to be modified and subjected to a demethylation treatment was indicated. The relative signal strength was 74.6%.

As described above, with the PGV-B2-GFAPp vector, which is a non-replicative plasmid vector, a demethylation reaction of the target nucleic acid sequence occurring in the cell was not detected. On the other hand, with use of the pM53-R550 K-GFAPp vector, which is a self-replicative plasmid vector, the demethylation reaction of the target nucleic acid sequence occurring in the cell could be detected. Note that the homology between the GFAP gene-promoter sequence, which is a target nucleic acid sequence, and the corresponding sequence in the glioma C6 cell, which is the subject cell, was 90.26%.

From these results, it was made clear that with use of the pM53-R550 K-GFAPp vector, which is a plasmid type self-replicating vector and one example of the embodiments, the followings are possible. That is, it is now clear that it is possible with use thereof to detect the demethylation in a base to be modified by referring to the signal strength of reporter protein as an index. That is, these results indicate that the degree of promoter activation varies depending on the state of modification of the modified base contained in the pM53-R550 K-GFAPp vector. The difference in the degree of activation could be detected with reference to the signal strength of reporter protein as the index. Thus, by using a self-replicating vector which has a target nucleic acid sequence homologous to a specific sequence of a cell, it is now possible to acquire epigenetic information of the specific sequence. Further, when a pM53-R550 K-GFAPp vector which includes a sequence homologous to a specific sequence of a subject cell, and is a plasmid type self-replicating vector, self-replicates in the subject cell, the followings occur. That is, the state of the modification in the modified base within the specific sequence of the subject cell is reflected in the state of the modification in the modified base in the target nucleic acid sequence on the pM53-R550 K-GFAPp vector.

From these results, it is now clear that by using the self-replicating vector, the epigenetic information in a specific sequence of a subject cell can be obtained by referring to the signal strength of reporter protein as an index.

Conventionally, in order to acquire the epigenetic information on a genome with foreign nucleic acid, it is essential that the foreign nucleic acid be replicated simultaneously with the replication of the genome to undergo an epigenetic modification reaction. For this reason, it is difficult to determine the characteristics of a cell using the reporter vector which is not incorporated in the genome. By contrast, according to the embodiments, it is possible to acquire epigenetic information, including the state and the like of the modification of a target nucleic acid, without incorporating foreign nucleic acid on the genome.

### Reference Example 3

### Comparison in methylation ratio of CK19 gene-promoter region (target nucleic acid sequence) between genomic DNA and reporter vector

A reporter vector (self-replicating vector 51) shown in FIG. 5 and a replication initiator protein gene expression vector (vector containing a replication initiator protein unit 52), were constructed. A promoter region (-617 to +61 bp, SEQ ID NO:2) of CK19 gene was incorporated in the reporter vector as a target nucleic acid sequence, the genomic DNA of the subject cell in which the vector was introduced and the target nucleic acid sequence of the reporter vector were compared in terms of methylation ratio.

### (1) Construction of reporter vector

The promoter region (-617 to +61, SEQ ID NO: 2) of CK19 gene, which was a target nucleic acid sequence, was amplified by PCR by using human genomic DNA as a template. The PCR was carried out using PrimeSTAR GLX DNA polymerase (TaKaRa BIO). The base sequences of the primers used for the PCR are indicated below.
Forward primer,
   5'-GCCTGGTCAACATGGTGAAAC-3' (SEQ ID NO: 13)
Reverse primer,
   5'-TGGGCTAGCCCAAGAAGCTGGTTCTGAGAGG-3' (SEQ ID NO: 14).

The target nucleic acid sequence obtained by the PCR was incorporated to upstream of the luciferase gene of the reporter vector which has the replication initiator sequence of the simian virus 40, and thus a reporter vector p19sLo shown in FIG. 16 was produced.

### (2) Construction of replication initiator protein gene expression vector

The replication initiator protein gene was the large T-antigens gene (SV40LT) of the simian virus 40. After amplifying the gene by the PCR using PrimeSTAR GLX DNA polymerase (TaKaRa BIO), the gene was incorporated downstream of the initial promoter of cytomegalovirus of a gene expression vector. Thus, a replication initiator protein (SV40LT) gene expression vector pCMV-LT shown in FIG. 17 was produced. The base sequences of the primers used for the PCR are indicated below.
Forward primer,
   5'-GGGGTACCAGATGGATAAAGTTTTAAACAGAGAGGAA-3' (SEQ ID NO: 15)
Reverse primer,
   5'-GGGAATTCTTATGTTTCAGGTTCAGGTTCAGGGGGAG-3' (SEQ ID NO: 16) .

### (3) Introducion

To 100µL of Opti-MEM (Life Technologies), 0.5 µg of the reporter vector was solely added, or 0.4 µg of the reporter vector and 0.1 µg of replication initiator protein expression vector pCMV-LT were added together. Then, 0.5 µL of a enhancer reagent (Plus reagent, Life Technologies) was added. The mixture was incubated for 5 minutes at room temperature. To this solution, 1.25 µL of Lipofectamine LTX was added to be well suspended. Then, the solution was incubated for 25 minutes at room temperature. The solution was transferred to a 24-well plate, and 200 µL of a suspension of Huh-7 cell was added thereto, to be mixed gently. After that, the cell was cultured within an incubator of a 5% CO₂ atmosphere at 37°C.

### (4) Preparation of genomic DNA and reporter vector from cell

96 hours after the introducion, the medium was removed from the 24-well plate and the cells were washed with phosphate buffered saline (PBS). Then, 200 µL of Trypsin-EDTA was added and the resultant was let stand for 5 minutes at room temperature. After that, 300 µL of PBS was added and the cells were collected in a 1.5-ml tube. Subsequently, the cells were precipitated by centrifuge. Then, the cells were suspended in 200 µL of PBS and using DNeasy Blood & Tissue Kit (Qiagen Inc.), a solution containing genomic DNA and reporter vector was obtained.

### (5) Detection of methylation ratio of genomic DNA

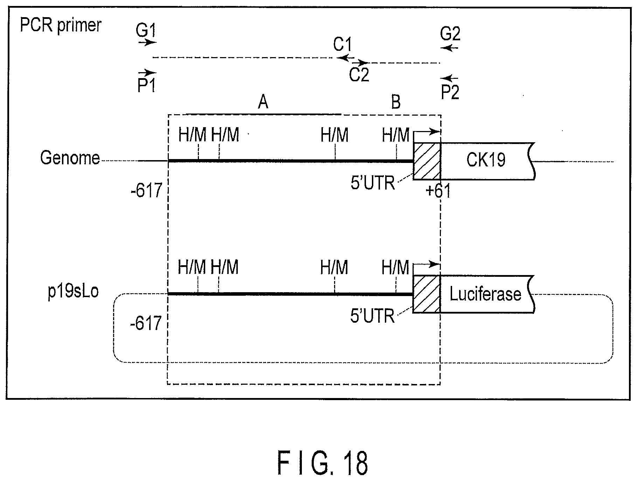

The CCGG sequence methylation ratio (presence/absence of 5-methylation of the 2nd cytosine) contained in the promoter region (-617 to +61 bp, SEQ ID NO: 2) of CK19 gene of the genomic DNA was examined as follows. That is, it was examined by the methylation detection method with a methylation sensitive restriction enzyme HpaII and a methylation non-sensitive restriction enzyme MspI. HpaII and MspI are restriction enzymes which recognize a common CCGG sequence. HpaII cannot cleave a CCGG sequence (CmCGG) containing methylated cytosine, whereas MspI cleaves a CCGG sequence regardless of methylated cytosine. The solution containing the genomic DNA and reporter vector prepared in (4) above was subjected to cleaving with restriction enzymes HpaII and MspI. Then, the resultant was purified with a QIAquick PCR purification kit (Qiagen Inc.). The resultant was a restriction-enzyme-treated DNA. Then, PCR was performed using the obtained DNA and restriction-enzyme-untreated DNA as templates with the primers indicated below. Thus, a region including the CCGG sequence of COX2 gene-promoter region of the genomic DNA was amplified (FIG. 18).
Forward primer 1 '(G1), 5'-TCAGAGGGGACAAAAGGGGAGTTGG-3' (SEQ ID NO: 17)
Reverse Primer 1 (C1), 5'-AGAGGCCCCTGCCCTCCAGAGGT-3' (SEQ ID NO: 18)
Forward Primer 2 (C2), 5'-GCAAATTCCTCAGGGCTCAGATA-3' (SEQ ID NO: 19)
Reverse Primer 2' (G2), 5'-CCAGGCCTCCGAAGGACGACGTGGC-3' (SEQ ID NO: 20)

The PCR reaction mixture was subjected to electrophoresis with agarose gel (containing ethidium bromide). Then, the PCR amplification product in the gel was visualized by UV irradiation, to be photographed. Then, the band strength of each section of the photograph obtained was digitized with an image-analysis software, Image J. Further, the rate of the CCGG sequence methylation to all the DNAs of A and B was calculated from the obtained numerical values and the following formulas. $Methylation ratio = \frac{\left(PCR after treated with HpaII \left(signal strength\right)-PCR after treated with MspI \left(signal strength\right)\right)}{\left(PCR of DNA untreated with restriction enzyme \left(signal strength\right)-PCR after treated with MspI \left(signal strength\right)\right)}$

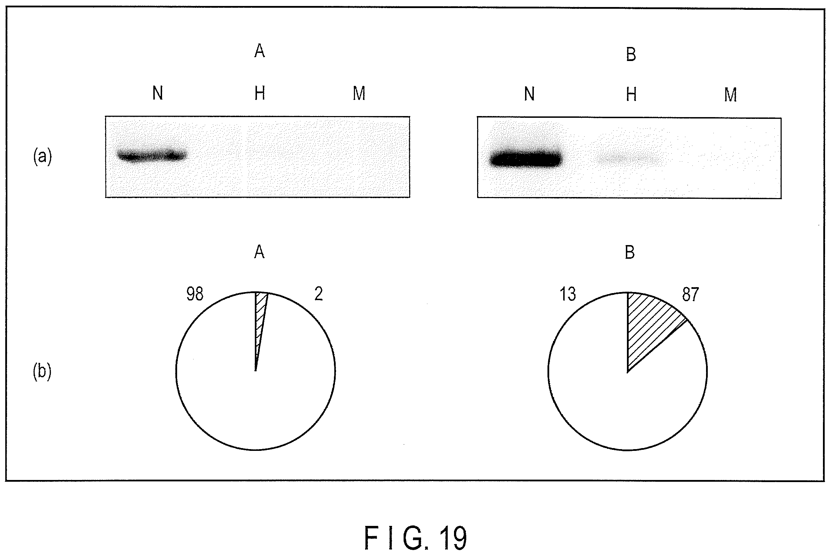

The results are shown in FIG. 19a and FIG. 19(b). Here, in each photograph obtained, the background is black and the bands are shown as white-out portions. The photograph was subjected to digitization for band strength with image-analysis software as it was. But, if the image is shown as it is in a diagram, the bands do not clearly appear. Under the circumstance, in order to make a band more visible, the photograph obtained was imported to the computer to be processed with image processing software and thus, an image as shown in FIG. 19 (a) was obtained, in which black and white were reversed so that the relativity between the band strength and other data might not be impaired. The other photographs taken were subjected to reversal of black and white in a similar manner.

In FIG. 19a, a lane N is the band of a PCR amplification product of restriction-enzyme untreated DNA and indicates the total amount of DNA subjected to the PCR. A lane H is the band of a PCR amplification product of DNA treated with HpaII, and indicates the amount of the DNA in which the 2nd cytosine of CCGG was methylated among all DNA subjected to the PCR. A lane M is a PCR amplification product of DNA treated with MspI, and indicates the background of the detection. The results shown in FIG. 19a indicate that the band of the PCR amplification product was detected on the lane H of CCGG sequences A and B, and the band strength was higher in B than A.

The results related to the ratio in methylation of the CCGG sequences, obtained by the above-described calculation are shown in FIG. 19b. The results of FIG. 19b indicate that the ratio in methylation of CCGG sequence A was 2% and that of B was 87%.

Here, according to the above-described formula, the methylation ratio is calculated from the signal strength regarding the band of a PCR amplification product, but the methylation ratio may be similarly calculated from a signal strength regarding an amplification product obtained by some other amplification method. Moreover, the calculation procedure of the methylation ratio is not limited to that described above with the above formula.

### (6) Detection of methylation ratio of non-replicating reporter vector

The methylation ratio of the non-replicating reporter vector was examined by the method described in (3) above. Together with a reporter vector pC2sLo, or p19sLo solely, the introducion was carried out to human hepatoma cell strain Huh-7. Using the DNA solution extracted 96 hours thereafter, the methylation ratio was detected. For the replication of a reporter vector, it is required that a replication initiator sequence and a replication initiator protein be present in the same cell. Therefore, when p19sLo is solely introduced to a cell, p19sLo is not replicated in the cell. By the same method as that described in (5) above, the methylation ratio of the CCGG sequence (presence/absence of 5-methylation of the 2nd cytosine) contained in the promoter region (-617 to +61bp, SEQ ID NO: 2) of CK19 gene of reporter vector p19sLo was examined. The DNA prepared in (4) above was cleaved with restriction enzymes HpaII and MspI. Then, the resultant was purified with QIAquick PCR purification kit (Qiagen Inc.). Thus, the restriction enzyme-treated DNA was obtained. Then, PCR was performed using the obtained DNA and restriction-enzyme-untreated DNA as templates with the primers indicated below. Thus, the region which includes the CCGG sequence of the CK19 gene promoter region of p19sLo was amplified (FIG. 18).
Forward Primer 1 '(PI), 5'-TAGGCTGTCCCCAGTGCAAGT-3' (SEQ ID NO: 21)
Reverse Primer 1(C1), 5'-AGAGGCCCCTGCCCTCCAGAGGT-3' (SEQ ID NO: 18)
Forward Primer 2(C2), 5'-GCAAATTCCTCAGGGCTCAGATA-3' (SEQ ID NO: 19)
Reverse Primer 2'(P2), 5'-AATGCCAAGCTTACTTAGATCG-3' (SEQ ID NO: 22).

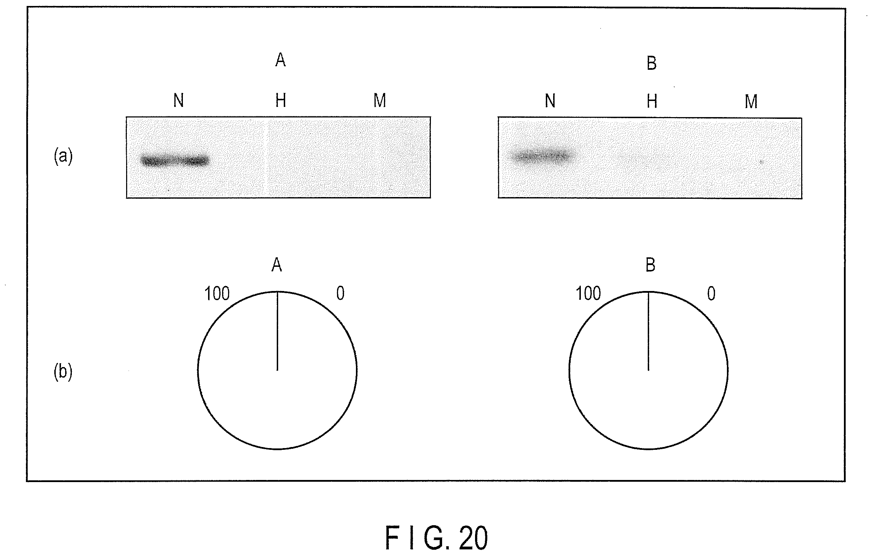

The results of the electrophoresis of the PCR reaction mixture in the agarose gel (containing ethidium bromide) are shown in FIG. 20a. The results of FIG. 20a indicate that a PCR amplification product was not detected on a lane H in either A or B. Thus, the ratio of methylation of each of the CCGG sequences A and B was 0% (FIG. 20b). Further, in the non-replicating reporter vector, methylation of A or B did not occur.

### (7) Detection of methylation ratio of replication reporter vector

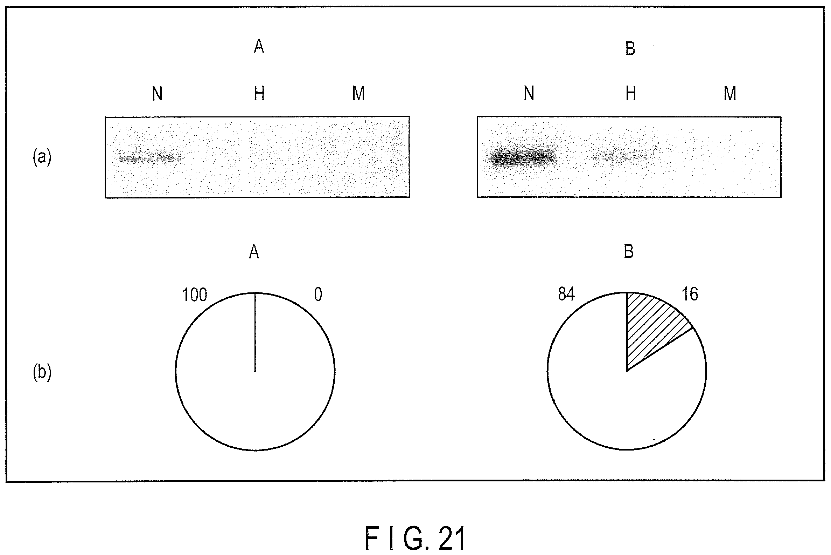

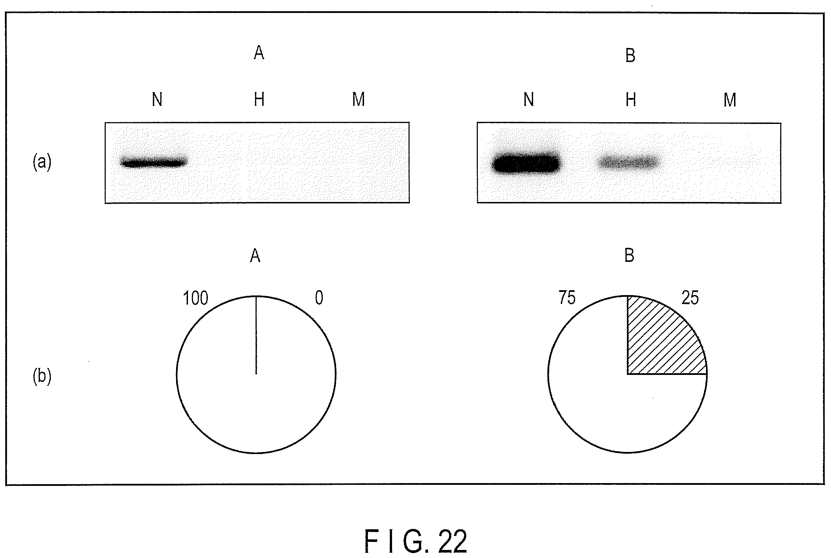

The methylation ratio of the replication reporter vector was detected using DNA obtained by introducing both reporter vectors p19sLo and pCMV-LT to Huh-7 and extracting DNA 96 hours thererafter. When P19sLo and pCMV-LT are introduced together into a cell, p19sLo replicates in the cell since a replication initiator sequence and a replication initiator protein are co-present in the cell. PCR was performed with the same primers as those of (6) above using DNA treated with a restriction enzyme HpaII or MspI, and restriction-enzyme-untreated DNA as templates. Thus, the region which includes the CCGG sequence of CK19 gene-promoter region of p19sLo was amplified. The results of electrophoresis of the PCR reaction mixture in agarose gel (containing ethidium bromide) are shown in FIG. 21a. The results indicate that a PCR amplification product was detected on the lane H of each of A and B. The ratio of methylation of the CCGG sequence A was 0% and that of B was 16% (FIG. 21b). Further, only for the reporter vector replicated in the cell, the methylation ratio of the CCGG sequence of the CK19 gene-promoter region was examined with a combination of a DpnI method (method of detecting a vector replicated in a cell) and a methylation detection method using HpaII and MspI (FIG. 22a). The results indicate that a PCR amplification product was detected on the lane H of each of A and B. The ratio of methylation of CCGG sequence A was 0% and that of B was 24% (FIG. 22b). The CK19 gene-promoter region of the reporter vector was methylated at approximately the same ratio as that of the CK19 gene-promoter region (target nucleic acid sequence) of the genomic DNA.

### Reference Example 4

### Comparison in methylation ratio of COX2 gene-promoter region (target nucleic acid sequence) between genomic DNA and reporter vector

A reporter vector (self-replicating vector 51) shown in FIG. 5 and a replication initiator protein gene expression vector (vector containing a replication initiator protein unit 52) were constructed. A promoter region (-540 to +115bp, SEQ ID NO: 3) of COX2 gene was incorporated in the reporter vector as a target nucleic acid sequence, the genomic DNA of the subject cell in which the vector was introduced and the target nucleic acid sequence of the reporter vector were compared in terms of methylation ratio.

### (1) Construction of reporter vector

The promoter region (-540 to +115bp, SEQ ID NO: 3) of COX2 gene, which was a target nucleic acid sequence, was amplified by PCR by using human genomic DNA as a template. The PCR was carried out using PrimeSTAR GLX DNA polymerase (TaKaRa BIO). The base sequences of the primers used for the PCR are indicated below.
Forward primer, 5'-CTTAACCTTACTCGCCCCAGTCT-3' (SEQ ID NO: 23)
Reverse primer, 5'-AGGCTCGAGCGCGGGGGTAGGCTTTGCTGTCTGAG-3' (SEQ ID NO: 24).

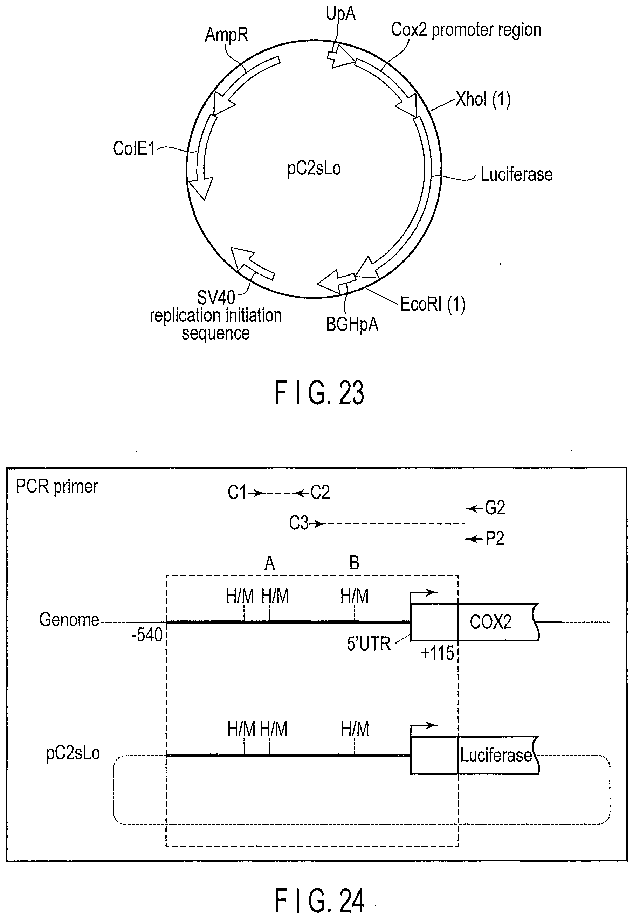

The target nucleic acid sequence obtained by the PCR was incorporated to upstream of luciferase gene of the reporter vector containing a replication initiator sequence of simian virus 40. Thus, a reporter vector pC2sLo shown in FIG. 23 was produced.

### (2) Replication initiator protein gene expression vector

The same vector as that of (2) in Example 3, vector pCMV-LT for expressing the replication initiator protein (SV40LT) gene, was used.

### (3) Methylation of reporter vector

A reaction solution (50mM-NaCl, 10mM-Tris-HCl, 10mM-MgCl₂, 1mM-DTT and 160pM-S-adenosylmethioine) containing a reporter vector and a reaction buffer was prepared. To the solution, a DNA methylation enzyme: SssI CpG Methyltransferase (New England Biolabs) was added and the solution was allowed to react at 37°C overnight. Thus, cytosine of CG sequence of the reporter vector was methylated.

### (4) Introducion

The introducion was performed by the same method as that of (3) in Example 3.

### (5) Preparation of genomic DNA and reporter vector from cell

The preparation of genomic DNA and a reporter vector from a cell was performed by the same method as that of (4) in Example 3 described above.

### (6) Detection of methylation ratio of genomic DNA

The methylation ratio of CCGG sequence (presence/absence of 5-methylation of the 2nd cytosine) contained in the COX2 gene promoter (-540 to +115bp, SEQ ID NO: 3) of the genomic DNA was examined as follows. That is, it was examined by the methylation detection method with a methylation sensitive restriction enzyme HpaII and a methylation non-sensitive restriction enzyme MspI. The solution containing the genomic DNA and reporter vector prepared in (5) above was subjected to cleaving with a restriction enzyme HpaII or MspI. Then, the resultant was purified with QIAquick PCR purification kit (Qiagen Inc.). The resultant was a restriction-enzyme-treated DNA. Then, PCR was performed using the obtained DNA and restriction-enzyme-untreated DNA as templates with the primers indicated below. Thus, a region including the CCGG sequence of COX2 gene-promoter region of the genomic DNA was amplified (FIG. 24).
Forward Primer 1 (C1), 5'-GGAAGCCAAGTGTCCTTCTGC-3' (SEQ ID NO: 25)
Reverse Primer 1(C2), 5'-GGGCAGGGTTTTTTACCCAC-3' (SEQ ID NO: 26)
Forward Primer 2(C3), 5'-AGCTTCCTGGGTTTCCGATTT-3' (SEQ ID NO: 27)
Reverse Primer 2' (G2), 5'-GCCAGGTACTCACCTGTATGGCTG-3' (SEQ ID NO: 28).

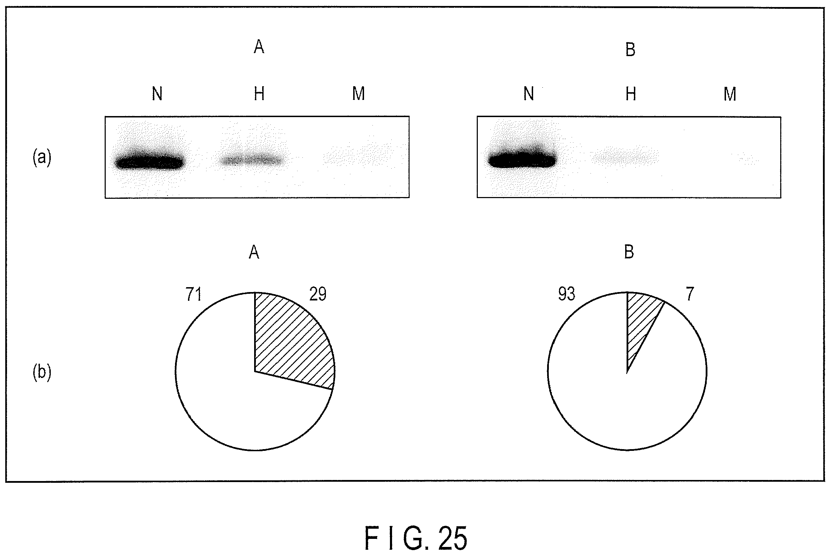

The PCR reaction mixture was subjected to electrophoresis with agarose gel (containing ethidium bromide). Then, the PCR amplification product in the gel was visualized by UV irradiation, to be photographed. The results are shown in FIG. 25a. The result of FIG. 25a indicate that the band of a PCR amplification product was detected in lane H of each of the CCGG sequences A and B. The band strength was higher in A than B. The ratio of methylation in CCGG sequence, calculated by the same method as that of (5) in Example 3, was 29% in A and 7% in B (FIG. 25b).

### (7) Detection of methylation ratio of non-replicating and non-methylated reporter vector

The methylation ratio of the non-replicating reporter vector was examined by the method described in (3) above. Together with a reporter vector pC2sLo, or solely, the introducion was carried out to human hepatoma cell strain Huh-7. Using the DNA solution extracted 96 hours thereafter, the methylation ratio was detected. By the same method as that described in (5) above, the methylation ratio of the CCGG sequence contained in the promoter region (-540 to +115bp, SEQ ID NO: 3) of COX2 gene of reporter vector pC2sLo was examined. The DNA prepared in (5) above was cleaved with a restriction enzyme HpaII or MspI. Then, the resultant was purified with QIAquick PCR purification kit (Qiagen Inc.). Thus, the restriction enzyme-treated DNA was obtained. Then, PCR was performed using the obtained DNA and restriction-enzyme-untreated DNA as templates with the primers indicated below. Thus, the region which includes the CCGG sequence of the COX2 gene-promoter region of pC2sLo was amplified (FIG. 24).
Forward Primer 1 (C1), 5'-GGAAGCCAAGTGTCCTTCTGC-3' (SEQ ID NO: 25)
Reverse Primer 1 (C2), 5'-GGGCAGGGTTTTTTACCCAC-3' (SEQ ID NO: 26)
Forward Primer 2 (C3), 5'-AGCTTCCTGGGTTTCCGATTT-3' (SEQ ID NO: 27)
Reverse Primer 2' (P2), 5'-AATGCCAAGCTTACTTAGATCG-3' (SEQ ID NO: 29).

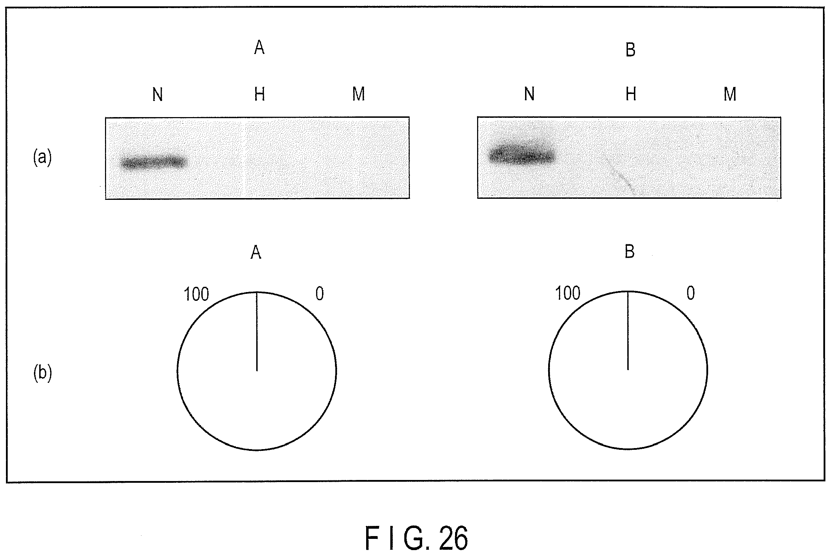

The results of the electrophoresis of the PCR reaction mixture in the agarose gel (containing ethidium bromide) are shown in FIG. 26a. The results of FIG. 26a indicate that a PCR amplification product was not detected on a lane H in either A or B. Thus, the ratio of methylation of each of the CCGG sequences A and B was 0% (FIG. 20b). Further, in the non-replicating reporter vector, methylation of A or B did not occur (FIG. 26b).

### (8) Detection of methylation ratio of replication non-methylated reporter vector

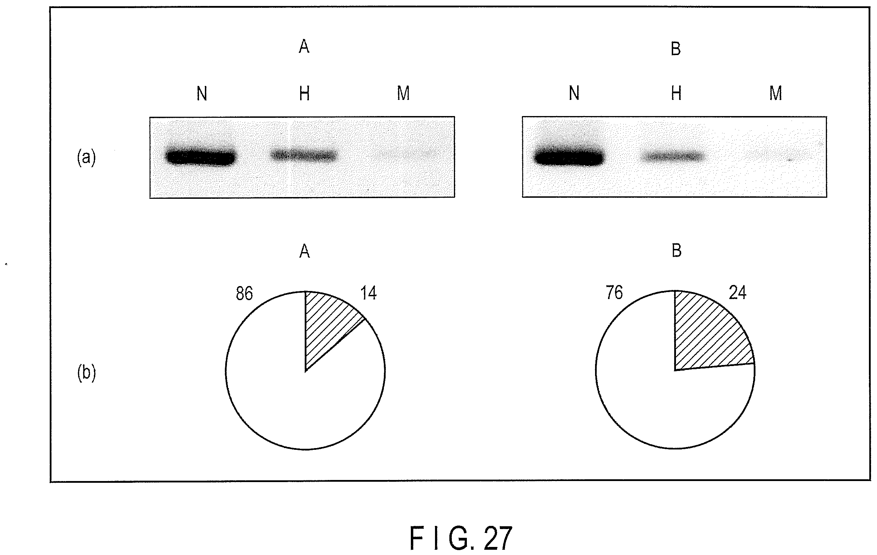

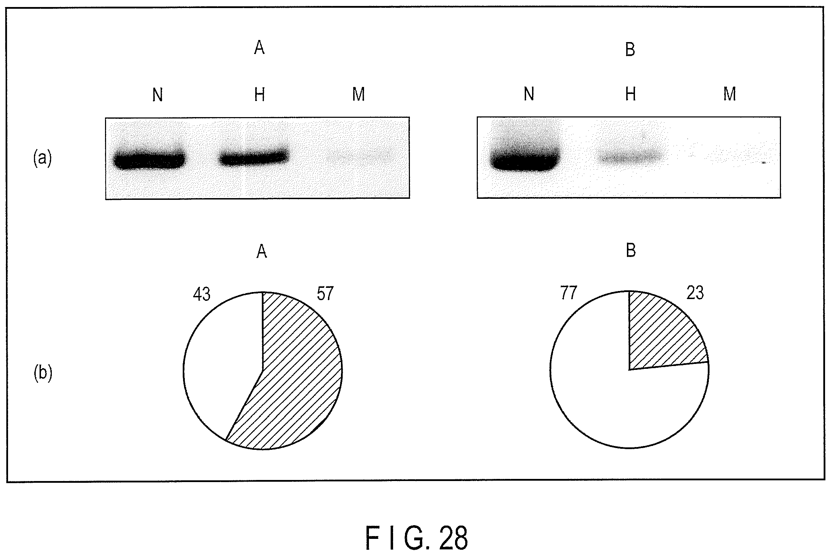

The methylation ratio of the replication reporter vector was examined as follows. A reporter vector pC2sLo and replication initiator protein gene expression vector pCMV-LT were introduced together to Huh-7. Using the DNA solution extracted 96 hours thereafter, the methylation ratio was detected. PCR was performed with the same primers as those of (7) above using DNA treated with a restriction enzyme HpaII or MspI, and restriction-enzyme-untreated DNA as templates. Thus, the region which includes the CCGG sequence of COX2 gene-promoter region of pC2sLo was amplified. The results of electrophoresis of the PCR reaction mixture in agarose gel (containing ethidium bromide) are shown in FIG. 27a. The results indicate that a PCR amplification product was detected on the lane H of each of A and B. The ratio of methylation of the CCGG sequence A was 14% and that of B was 24% (FIG. 27b). Further, only for the reporter vector replicated in the cell, the methylation ratio of the CCGG sequence of the COX2 gene-promoter region was examined with a combination of a DpnI method (method of detecting a vector replicated in a cell) and a methylation detection method using HpaII and MspI (FIG. 28a). The results indicate that a PCR amplification product was detected on the lane H of each of A and B. The ratio of methylation of CCGG sequence A was 57% and that of B was 23% (FIG. 28b). The COX2 gene-promoter region of pC2sLo of the replicated reporter vector was methylated at substantially the same ratio as that of the COX2 gene-promoter region (target nucleic acid sequence) of the genomic DNA.

### (9) Detection of methylation ratio of non-replicating methylated reporter vector

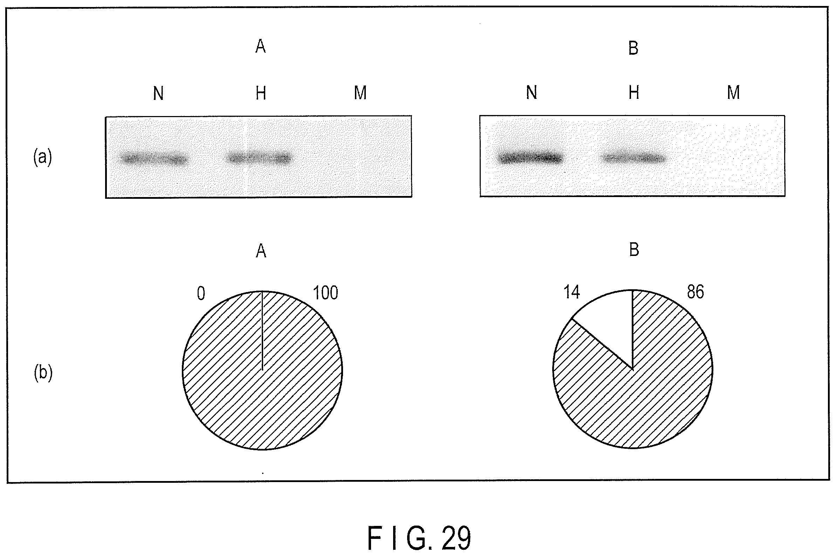

In order to detect the methylation ratio of the non-replicating methylated reporter vector, a methylated reporter vector pC2sLo was introduced solely to human hepatoma cell strain Huh-7. The DNA solution extracted 96 hours thereafter by the same method described in (5) above was used. The DNA was cleaved with a restriction enzyme HpaII or MspI. And then, it was purified with QIAquick PCR purification kit (Qiagen Inc.), thus, restriction-enzyme-treated DNA was obtained. PCR was performed with the primers listed below using the DNA and restriction-enzyme-untreated DNA as templates. Thus, the region which includes the CCGG sequence of COX2 gene-promoter region of pC2sLo was amplified. The results of electrophoresis of the PCR reaction mixture in agarose gel (containing ethidium bromide) are shown in FIG. 29a. The results of FIG. 29a indicate that a PCR amplification product was detected on the lane H of each of A and B. The ratio of methylation of the CCGG sequence A was 100% and that of B was 86% (FIG. 29b). The methylation ratio of the non-replicating methylated reporter vector did not match that of the genomic DNA of the host cell.

### (10) Detection of methylation ratio of replicating methylated reporter vector

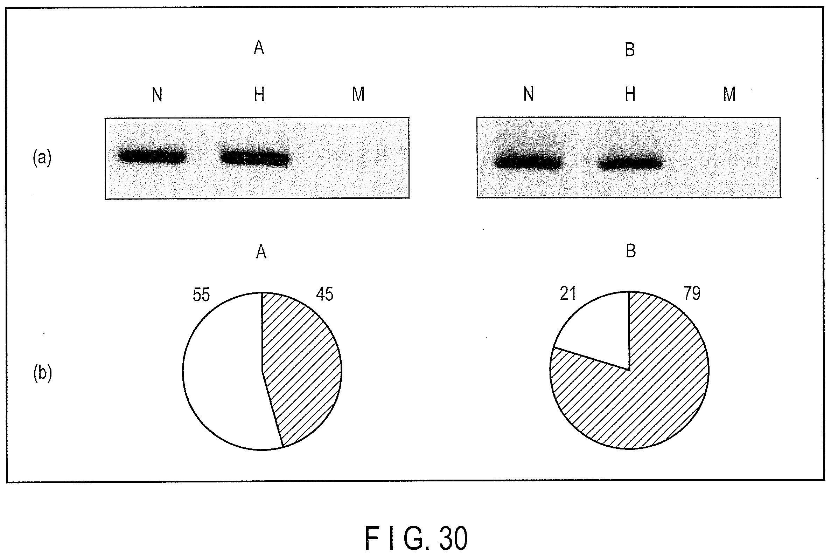

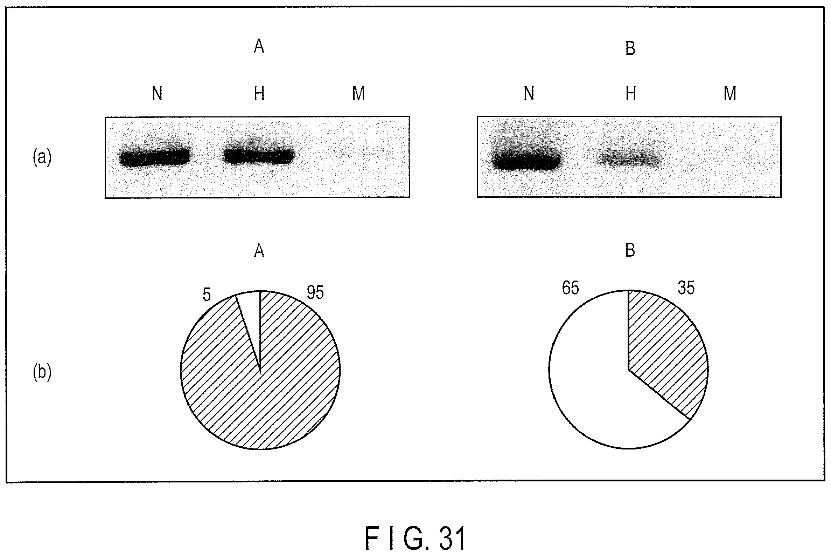

In order to detect the methylation ratio of the replicating methylated reporter vector, a reporter vector pC2sLo and replication initiator protein gene expression vector pCMV-LT were co-introduced to Huh-7, and then using the DNA solution extracted 96 hours thereafter, the methylation ratio was detected. DNA treated with a restriction enzyme HpaII or MspI and restriction-enzyme-untreated DNA were used as templates. PCR was performed with the same primers as those used in (6) above. Thus, the region which includes the CCGG sequence of COX2 gene-promoter region of pC2sLo was amplified. The results of electrophoresis of the PCR reaction mixture in agarose gel (containing ethidium bromide) are shown in FIG. 30a. The results indicate that a PCR amplification product was detected on the lane H of each of A and B. The ratio of methylation of the CCGG sequence A was 45% and that of B was 79% (FIG. 31b). Further, only for the reporter vector replicated in the cell, the methylation ratio of the CCGG sequence of the COX2 gene-promoter region was examined with a combination of a DpnI method (method of detecting a vector replicated in a cell) and a methylation detection method using HpaII and MspI (FIG. 31a). The results indicate that a PCR amplification product was detected on the lane H of each of A and B. The ratio of methylation of each CCGG sequence A was 95% and that of B was 35% (FIG. 31b). When the reporter vector replicated in the cell and the COX2 gene-promoter region of genomic DNA were compared with each other in the methylation ratio of the CCGG sequence, it was found that B was strongly demoethylated, and the methylation ratio was high in A.

### Reference Example 5

### Detection of demethylation of target nucleic acid sequence (COX2 gene-promoter region) by luciferase assay

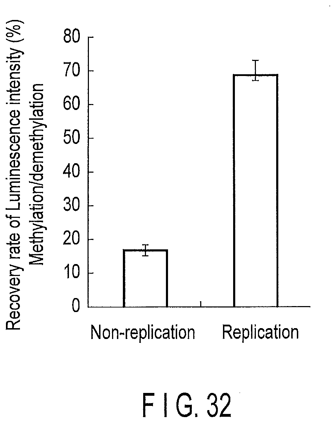

The same methods as those of Example 4 were used here. Methylated or non-methylated reporter vector pC2sLo was solely or together with replication initiator protein expression vector pCMV-LT, introduced to human hepatoma cell strain Huh-7. After 72 hours of culturing, the reporter activity derived from pC2sLo was measured. The culture medium was removed from a 24-well plate and washed with PBS. Then, 150µL of a cytolytic solution (Promega KK) was added and the resultant was let stand at -80°C for 30 minutes or more to be frozen. After thawing the cytolytic solution at room temperature, the solution was collected in the centrifugal tube. The cell residue was precipitated by the centrifuge. Then, 10µL of the supernatant was dispensed to a 96-well plate (Nunc), and 50µL of a Luciferase substrate solution (One-Glo Luciferase Assay System, Promega) was added thereto. Thus, the luminescence intensity of the cytolytic solution was measured. The results are shown in FIG. 32. In FIG. 32, the vertical axis of the graph indicates the rate of recovery of luminescence intensity. The rate of recovery of the luminescence intensity is an index of demethylation. The rate of recovery of luminescence intensity is a rate of the luminescence intensity obtained from the cell into which methylated reporter DNA was introduced with respect to the luminescence intensity obtained from the cell into which non-methylated reporter vector was introduced, being set as 100%. The left end of the graph indicates the rate of recovery of the luminescence intensity of the non-replicating reporter vector, whereas that of the replicating reporter vector is shown at the right end. From this graph, it was understood that the rate of recovery of luminescence intensity of the replicating reporter vector is higher than that of the non-replicating reporter vector. Thus, it becames possible in a replicating reporter vector to detect the demethylation occurring a target nucleic acid sequence with high sensitivity. From this result, it has been confirmed that epigenetic information in a specific sequence of a subject cell can be obtained by using a reporter vector replicated in the cell and referring to the reporter activity as an index.

### Reference Example 6

Self-replicating vector comprising two kinds of reporter gene expression units (a unit containing a target nucleic acid sequence in which a functional group was substituted and a unit containing a target nucleic acid sequence in which a functional group is not substituted) and a replication initiator sequence

### (1) Production of vector

A reporter vector (self-replicating vector 61) shown in FIG. 6, which is an example of the embodiments was constructed. A reporter vector pNL1.1 (Promega KK) in which a luciferase of shrimp-origin was incorporated, was cleaved with restriction enzymes KpnI and BamHI. The DNA end thus obtained was truncated with T4 DNA polymerase. Then, a DNA fragment comprising shrimp luciferase and SV40 transcription termination sequence was prepared. The fragment was ligated to pl9sLo cleaved with restriction enzyme SspI with T4 DNA ligase to construct a self-replicating vector pl9sLo-NL. To pl9sLo-NL, a COX2 gene promoter (SEQ ID NO: 3) in which cytosine of CpG was methylated was incorporated. The COX2 gene promoter was amplified by PCR using human genome as a template. The primer sequences used for the PCR are indicated below.
Forward primer, 5'-GGGGCTAGCCTTAACCTTACTCGCCCCAGTCT-3' (SEQ ID NO: 30)
Reverse primer, 5'-AGGCTCGAGCGCGGGGGTAGGCTTTGCTGTCTGAG-3' (SEQ ID NO: 24).

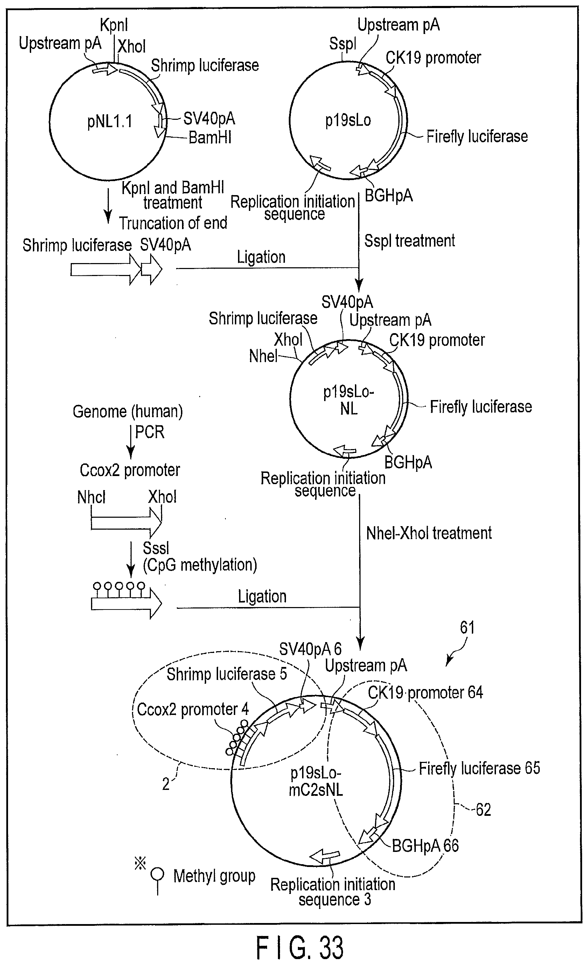

The PCR amplification product (COX2 gene promoter) was purified. Then, a reaction solution containing the amplification product and reaction buffer (50 mM-NaCl, 10 mM-Tris-HCl, 10 mM-MgCl2, 1 mM-DTT, 160 µM-S-adenosylmethioine) was prepared. To the solution, DNA methylation enzyme:SssI CpG Methyltransferase (New England Biolabs) was added and the solution was made to react at 37°C overnight. The cytosine of CG sequence of COX2 gene promoter was methylated. The thus methylated DNA fragment (methylated COX2 gene promoter) was ligated to self-replicating reporter vector pl9sLo-NL treated with restriction enzymes KpnI and XhoI with T4 DNA ligase. Thus, the self-replicating reporter vector p19sLo-mC2sNL containing two kinds of reporter gene expression units (a reporter gene expression unit containing a methylated COX2 gene promoter and a reporter gene expression unit containing a non-methylated CK19 gene promoter) and a replication initiator sequence was constructed (FIG. 33).

### (2) Introducion

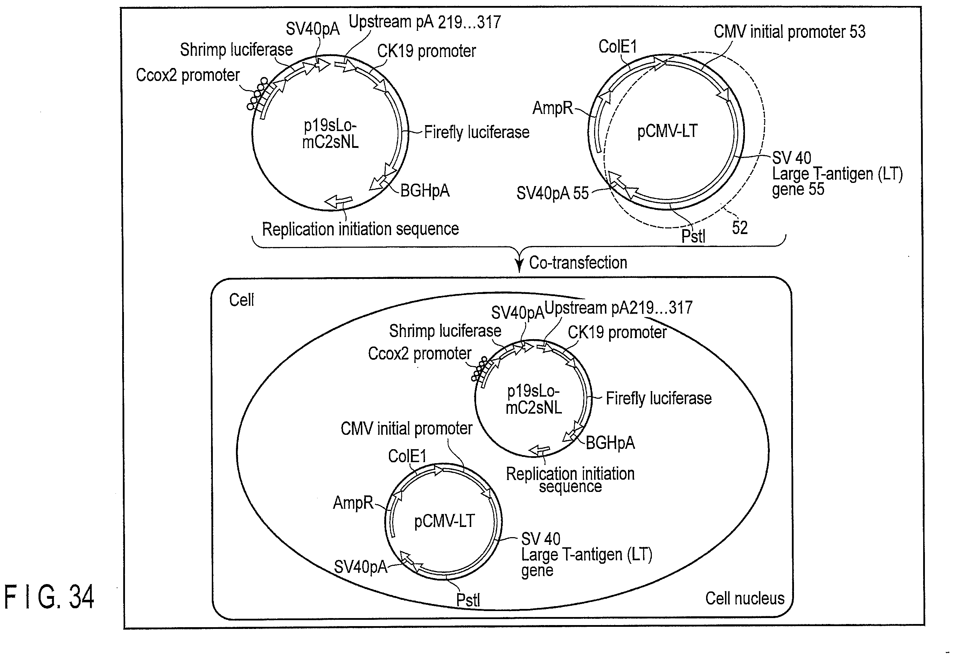

To 100 µL of Opti-MEM (Life Technologies), 0.5µg of the reporter vector was solely added, or 0.4 µg of p19sLo-mC2sNL and 0.1µg of a replication initiator protein expression vector pCMV-LT were added together. Then, 0.5 µL of a enhancer reagent (Plus reagent, Life Technologies) was added, and the mixture was incubated for 5 minutes at room temperature. To this solution, 1.25µL of Lipofectamine LTX was added to be well suspended. Then, the solution was incubated for 25 minutes at room temperature. The solution was transferred to a 24-well plate, and 200 µL of a suspension of Huh-7 cell was added thereto, to be mixed gently. Thus, the vector was introduced to the cell (FIG. 34).

### Reference Example 7

Self-replicating vector containing reporter gene expression unit, replication initiator gene expression unit and replication initiator sequence

### (1) Production of vector

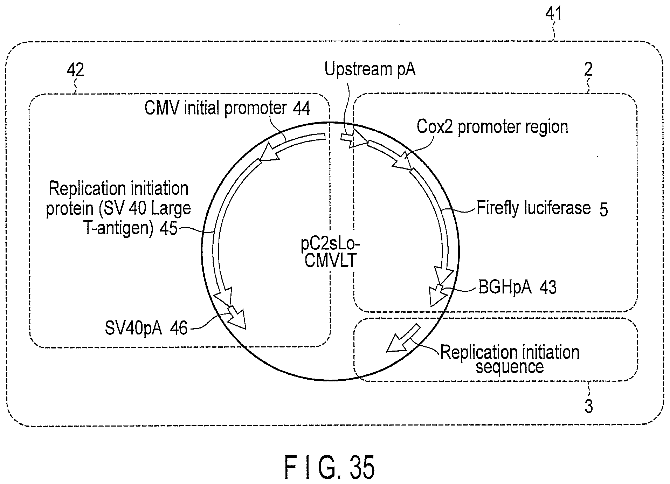

A reporter vector (self-replicating vector 41) shown in FIG. 4 was constructed. A replication initiator protein gene expression unit was cleaved out from pCMV-LT (Example 3 (2))with restriction enzymes BglI and BamHI. The DNA end thus obtained was truncated with T4 DNA polymerase. Then, a DNA fragment of the replication initiator protein gene expression unit was purified. This DNA fragment was ligated to pC2sLo (Example 4 (1)) cleaved with restriction enzyme SspI with T4 DNA ligase to construct a self-replicating vector pC2sLo-CMVLT (FIG. 35).

### (2) Introducion

To 100µL of Opti-MEM (Life Technologies), 0.5µg of pC2sLo-CMVLT was added. Then, 0.5µL of a enhancer reagent (Plus reagent, Life Technologies) was added, and the mixture was incubated for 5 minutes at room temperature. To this solution, 1.25µL of Lipofectamine LTX was added to be well suspended. Then, the solution was incubated for 25 minutes at room temperature. The solution was transferred to a 24-well plate, and 200µL of a suspension of Huh-7 cell was added thereto, to be mixed gently. After that, the cell was cultured within an incubator of a 5%-CO₂ atmosphere at 37°C.

### (3) Preparation of genomic DNA and reporter vector from cell

The preparation of genomic DNA and a reporter vector from a cell was performed by the same method as that of (4) in Example 3 described above.

### (4) Detection of methylation ratio of reporter vector

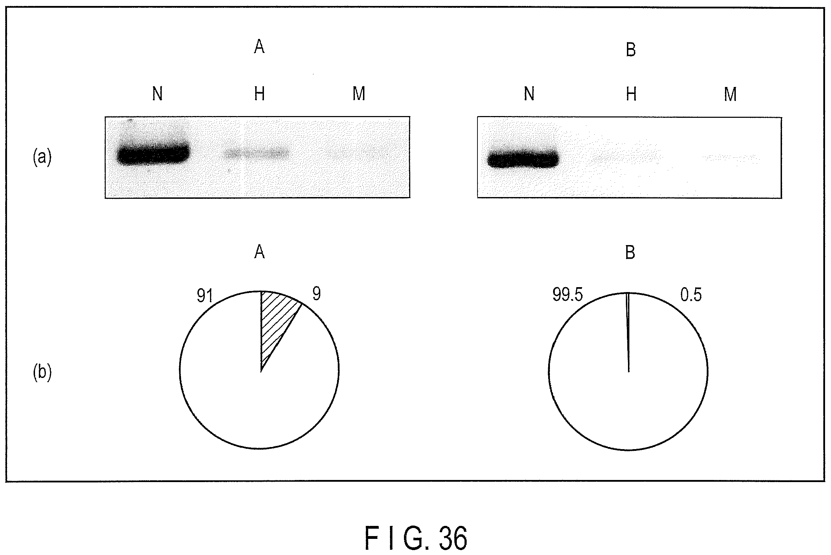

The methylation ratio of the reporter vector was examined by the method described in Example 4 (7) above. That is, a self-replicating reporter vector pC2sLo-CMVLT was introduced into Huh-7. Then, using the DNA solution extracted 72 hours thereafter, the methylation ratio was detected. The solution prepared in (3) above was cleaved with a restriction enzyme HpaII or MspI. Then, the resultant was purified with a QIAquick PCR purification kit (Qiagen Inc.). PCR was performed using the obtained DNA and restriction-enzyme-untreated DNA as templates with the same primers as those of Example 4 (7). Thus, the region which includes the CCGG sequence of the COX2 gene-promoter region (-540 to +115 bp, SEQ ID NO: 3) of pC2sLo-CMVLT was amplified (FIG. 24). The results of electrophoresis of the PCR reaction mixture in agarose gel (containing ethidium bromide) are shown in FIG. 36a. The results of FIG. 36a indicate that a PCR amplification product was detected on the lane H of each of A and B. The ratio of methylation of the CCGG sequence A was 9% and that of B was 0.5% (FIG. 31b).
Thus, in the COX2 gene-promoter region of the self-replicating reporter vector pC2sLo-CMVLT, the methylation ratio of A was high as in the case of the COX2 gene-promoter region of genome DNA (Example 4 (6), FIG. 25).

### Reference Marks List

1, 31, 41, 51, 61 Self-replicating vector
2, 62 Reporter gene expression unit
3 Replication initiator sequence
4, 64 Target nucleic acid sequence
5, 65 Reporter Genes
6, 34, 46, 55, 66 Transcription termination signal sequence
7 Replication initiator protein
32 IRES
33, 45, 54 Sequence encoding replication initiator protein
42 Replication initiator protein expression unit
44, 53 Constitutively expressed promoter
45, 50 Subject cell
51 First vector
56 Second vector

## Claims

1. A method of obtaining epigenetic information about a subject cell containing a specific genome sequence, comprising:
introducing a self-replicating vector into the subject cell, wherein the self-replicating vector comprises:
(a) a first reporter gene expression unit containing:
a first target nucleic acid sequence containing a base with a substitutable methyl group, having a promoter activity depending on a degree of substitution of the methyl group of the base, and corresponding to or having homology with the specific genome sequence of the subject cell, wherein the base is cytosine and/or guanine,
a first reporter gene encoding a first reporter protein functionally linked downstream of the first target nucleic acid sequence and which is expressed by activation of the first target nucleic acid sequence, and
a first transcription termination signal sequence functionally linked downstream of the reporter gene;
(b) a second reporter gene expression unit containing:
a second target nucleic acid sequence containing a base with a substitutable methyl group, having a promoter activity depending on a degree of substitution of the methyl group of the base, and corresponding to or having homology with the specific genome sequence of the subject cell, wherein the base is guanine and/or cytosine,
a second reporter gene encoding a second reporter protein functionally linked downstream of the second target nucleic acid sequence and which is expressed by activation of the second target nucleic acid sequence, wherein the first and second reporter proteins are different from one another, and
a second transcription termination signal sequence functionally linked downstream of the reporter gene,
wherein the second reporter gene expression unit is linked to a downstream side of the first reporter gene expression unit, and methylation states of the first and second target nucleic acid sequences are different from each other; and
(c) a replication initiator sequence present on the same nucleic acid as the first reporter gene expression unit,
wherein the self-replicating vector is capable of transcribing states of methylation of the specific genome sequence onto the first and second target nucleic acid sequences during self-replication of the self-replicating vector, by substitution of the methyl group of the first and second target nucleic acid sequence, and
wherein the self-replicating vector is capable of producing the first and second reporter proteins as first and second signals respectively in the subject cell depending on the states of substitution of the methyl groups of the first and second target nucleic acid sequences, respectively;
culturing the subject cell to promote the self-replicating vector to self-replicate in the subject cell;
detecting the first and second signals and/or measuring the amounts of expression of the first and second reporter proteins; and
obtaining the epigenetic information about the subject cell by comparing the amounts of expression of the first and second reporter proteins.

2. The method of Claim 1, **characterized in that**, during the culturing of the subject cell, a replication initiator protein is expressed in the subject cell, and the replication initiator protein activates the replication initiator sequence.

3. The method of Claim 2, **characterized by** further comprising:
introducing a replication initiator protein gene expression vector into the nucleus of the subject cell,
wherein the replication initiator protein gene expression vector contains a replication initiator protein unit which expresses the replication initiator protein.

4. The method of Claim 2, **characterized in that** the self-replicating vector further comprises a replication initiator protein unit which expresses the replication initiator protein.

5. The method of Claim 2, **characterized in that** the self-replicating vector further comprises
an IRES sequence functionally linked downstream of the first reporter gene expression unit,
a sequence encoding the replication initiator protein, functionally linked downstream of the IRES sequence, and
a replication initiator sequence,
wherein the transcription termination signal sequence is functionally linked downstream of the sequence encoding the replication initiator protein, and the replication initiator sequence is functionally linked downstream of the transcription termination signal sequence.

6. The method of Claim 3 or 4, **characterized in that** the replication initiator protein unit contains a sequence encoding the replication initiator protein, and a constitutively expressed promoter functionally linked upstream of the sequence encoding the replication initiator protein.

7. The method of one of Claims 1 to 6, **characterized in that** the first and second reporter genes are independently selected from the group consisting of a luciferase gene, a β-galactosidase gene, a nitric-monoxide synthase gene, a xanthine oxidase gene, a blue fluorescence protein gene, a green fluorescent protein gene, a red fluorescence protein gene and a heavy metal binding protein gene.

8. The method of one of Claims 6 or 7, **characterized in that** a combination of the gene encoding the replication initiator protein and the replication initiator sequence, is selected from the following (1), (2) and (3):
(1) the gene encoding the replication initiator protein is a large T-antigens gene of simian virus 40, and the replication initiator sequence is a replication initiator sequence from the simian virus 40;
(2) the gene encoding the replication initiator protein is EBNA-1 gene of Epstein-Barr virus and the replication initiator sequence is a replication initiator sequence from Epstein-Barr virus; and
(3) the gene encoding the replication initiator protein is a large T-antigens gene of mouse polyomavirus and the replication initiator sequence is a replication initiator sequence from mouse polyomavirus.

9. The method of Claim 8, wherein the large T-antigens gene is a nucleic acid represented by SEQ ID NO:7, SEQ ID NO: 8 or SEQ ID NO: 9, or a nucleic acid represented by SEQ ID NO:7, SEQ ID NO: 8 or SEQ ID NO: 9 in which one or some bases of its sequence are deleted, substituted or added and having a DNA replication initiating function.

10. The method of one of Claims 1 to 9, wherein the subject cell is a cell contained in blood obtained from a subject.

11. A method of determining characteristics of a cell, comprising:
determining the characteristics of the subject cell based on the epigenetic information of the subject cell obtained by the method of one of Claims 1 to 10.

12. The method of Claim 11, wherein the characteristics are of a drug sensitivity.

13. A method of determining characteristics of a cell, comprising: determining the characteristics of the subject cell based on the epigenetic information of the subject cell obtained by the method of one of Claims 1 to 10,
the determining comprising determining whether the subject cell is a cancerated cell.

14. A self-replicating vector for obtaining epigenetic information about a subject cell containing a specific genome sequence, comprising:
(a) a first reporter gene expression unit containing:
a first target nucleic acid sequence containing a base with a substitutable methyl group, having a promoter activity depending on a degree of substitution of the methyl group in the base and having homology with respect to the specific genome sequence of the subject cell, wherein the base is guanine and/or cytosine,
a first reporter gene encoding a first reporter protein functionally linked downstream of the first target nucleic acid sequence and expressed by activation of the first target nucleic acid sequence, and
a first transcription termination signal sequence functionally linked downstream of the reporter gene;
(b) a second reporter gene expression unit containing:
the second target nucleic acid sequence containing a base with a substitutable methyl group, having a promoter activity depending on a degree of substitution of the methyl group of the base, and corresponding to or having homology with the specific genome sequence of the subject cell, wherein the base is guanine and/or cytosine,
a second reporter gene encoding a second reporter protein functionally linked downstream of the second target nucleic acid sequence and which is expressed by activation of the second target nucleic acid sequence, wherein the first and second reporter proteins are different from one another, and
a second transcription termination signal sequence functionally linked downstream of the reporter gene,
wherein the second reporter gene expression unit is linked to a downstream side of the first reporter gene expression unit, and methylation states of the first and second target nucleic acid sequences are different from each other; and
(c) a replication initiator sequence present on the nucleic acid on which the first reporter gene expression unit is present.

15. The vector of Claim 14, **characterized in that** the first and second reporter genes are independently selected from the group consisting of a luciferase gene, a β-galactosidase gene, a nitric-monoxide synthase gene, a xanthine oxidase gene, a blue fluorescence protein gene, a green fluorescent protein gene, a red fluorescence protein gene and a heavy metal binding protein gene.

16. An assay kit comprising:
a self-replicating vector of Claim 14; and
a container containing the self-replicating vector.

## Patentansprüche

1. Verfahren zum Erhalten epigenetischer Information über eine Zielzelle (subject cell), welche eine spezifische Genomsequenz enthält, umfassend:
Einbringen eines selbst-replizierenden Vektors in die Zielzelle, wobei der selbst-replizierende Vektor umfasst:
(a) eine erste Reportergen-Expressionseinheit, beinhaltend:
eine erste Zielnukleinsäuresequenz, enthaltend eine Base mit einer substituierbaren Methylgruppe,
welche eine von einem Grad der Substitution der Methylgruppe der Base abhängende Promotoraktivität aufweist, und der spezifischen Genomsequenz der Zielzelle entspricht oder Homologie dazu aufweist,
wobei die Base Cytosin und/oder Guanin ist,
ein downstream der ersten Zielnukleinsäuresequenz funktionell verknüpftes erstes Reportergen, welches ein erstes Reporterprotein codiert, und welches durch Aktivierung der ersten
Zielnukleinsäuresequenz exprimiert wird, und
eine downstream des Reportergens funktionell verknüpfte erste Transkriptionsterminations-Signalsequenz;
(b) eine zweite Reportergen-Expressionseinheit, beinhaltend:
eine zweite Zielnukleinsäuresequenz, enthaltend eine Base mit einer substituierbaren Methylgruppe,
welche eine von einem Grad der Substitution der Methylgruppe der Base abhängende Promotoraktivität aufweist, und der spezifischen Genomsequenz der Zielzelle entspricht oder Homologie dazu aufweist,
wobei die Base Guanin und/oder Cytosin ist,
ein downstream der zweiten Zielnukleinsäuresequenz funktionell verknüpftes zweites Reportergen,
welches ein zweites Reporterprotein codiert, und
welches durch Aktivierung der zweiten Zielnukleinsäuresequenz exprimiert wird,
wobei das erste und das zweite Reporterprotein unterschiedlich voneinander sind, und
eine downstream des Reportergens funktionell verknüpfte zweite Transkriptionsterminations-Signalsequenz,
wobei die zweite Reportergen-Expressionseinheit mit einem Downstream-Abschnitt (downstream side) der ersten Reportergen-Expressionseinheit verknüpft ist, und Methylierungszustände der ersten und der zweiten Zielnukleinsäuresequenz unterschiedlich voneinander sind; und
(c) eine Replikationsinitiatorsequenz, welche auf derselben Nukleinsäure wie die erste Reportergen-Expressionseinheit vorliegt,
wobei der selbst-replizierende Vektor die Fähigkeit hat, Methylierungszustände der spezifischen Genomsequenz auf die erste und die zweite Zielnukleinsäuresequenz während selbst-Replikation des selbst-replizierenden Vektors zu transkribieren, durch Substitution der Methylgruppe der ersten und zweiten Zielnukleinsäuresequenz, und wobei der selbst-replizierende Vektor die Fähigkeit hat, das erste und das zweite Reporterprotein jeweils als erstes und zweites Signal in der Zielzelle zu produzieren, jeweils abhängig von den Substitutionszuständen der Methylgruppen der ersten und zweiten Zielnukleinsäuren;
Kultivieren der Zielzelle, um den selbst-replizierenden Vektor zum Selbst-Replizieren in der Zielzelle zu bringen;
Detektieren des ersten und des zweiten Signals und/oder Messen der Expressionsmengen des ersten und des zweiten Reporterproteins; und
Erhalten der epigenetischen Information über die Zielzelle durch Vergleichen der Expressionsmengen des ersten und des zweiten Reporterproteins.

2. Verfahren gemäß Anspruch 1, dadurch charakterisiert, dass während des Kultivierens der Zielzelle ein Replikationsinitiatorprotein in der Zielzelle exprimiert wird, und das Replikationsinitiatorprotein die Replikationsinitiatorsequenz aktiviert.

3. Verfahren gemäß Anspruch 2, charakterisiert durch weitergehendes Umfassen von:
Einbringen eines Replikationsinitiatorproteingen-Expressionsvektors in den Zellkern der Zielzelle,
wobei der Replikationsinitiatorproteingen-Expressionsvektor eine Replikationsinitiatorprotein-Einheit enthält, welche das Replikationsinitiatorprotein exprimiert.

4. Verfahren gemäß Anspruch 2, dadurch charakterisiert, dass der selbst-replizierende Vektor weitergehend eine Replikationsinitiatorprotein-Einheit umfasst, welche das Replikationsinitiatorprotein exprimiert.

5. Verfahren gemäß Anspruch 2, dadurch charakterisiert, dass der selbst-replizierende Vektor weitergehend eine downstream der ersten Reportergen-Expressionseinheit funktionell verknüpfte IRES-Sequenz umfasst,
eine downstream der IRES-Sequenz funktionell verknüpfte Sequenz umfasst, welche das Replikationsinitiatorprotein codiert, und
eine Replikationsinitiatorsequenz umfasst,
wobei die Transkriptionsterminations-Signalsequenz downstream der Sequenz funktionell verknüpft ist, welche das Replikationsinitiatorprotein codiert, und die Replikationsinitiatorsequenz downstream der Transkriptionterminations-Signalsequenz funktionell verknüpft ist.

6. Verfahren gemäß Anspruch 3 oder 4, dadurch charakterisiert, dass die Replikationsinitiatorprotein-Einheit eine Sequenz enthält, welche das Replikationsinitiatorprotein codiert, und einen konstitutiv exprimierten Promotor, welcher upstream der Sequenz funktionell verknüpft ist, welche das Replikationsinitiatorprotein codiert.

7. Verfahren gemäß einem der Ansprüche 1 bis 6, dadurch charakterisiert, dass das erste und das zweite Reportergen unabhängig voneinander ausgewählt werden aus der Gruppe bestehend aus einem Luciferase-Gen, einem β-Galactosidase-Gen, einem Stickstoffmonoxid-Synthase-Gen, einem Xanthin-Oxidase-Gen, einem blau-fluoreszierendes-Protein-Gen, einem grün-fluoreszierendes-Protein-Gen, einem rot-fluoreszierendes-Protein-Gen und einem Schwermetall-bindendes-Protein-Gen.

8. Verfahren gemäß einem der Ansprüche 6 oder 7, dadurch charakterisiert, dass eine Kombination des Gens, welches das Replikationsinitiatorprotein und die Replikationsinitiatorsequenz codiert, ausgewählt wird aus den folgenden (1), (2) und (3):
(1) das Gen, welches das Replikationsinitiatorprotein codiert, ist ein großes-T-Antigen-Gen des Simian-Virus 40, und die Replikationsinitiatorsequenz ist eine Replikationsinitiatorsequenz des Simian-Virus 40;
(2) das Gen, welches das Replikationsinitiatorprotein codiert, ist EBNA-1-Gen des Epstein-Barr-Virus und die Replikationsinitiatorsequenz ist eine Replikationsinitiatorsequenz des Epstein-Barr-Virus; und
(3) das Gen, welches das Replikationsinitiatorprotein codiert, ist ein großes-T-Antigen-Gen des murinen Polyomavirus, und die Replikationsinitiatorsequenz ist eine Replikationsinitiatorsequenz des murinen Polyomavirus.

9. Verfahren gemäß Anspruch 8, wobei das große-T-Antigen-Gen eine durch SEQ ID NO:7, SEQ ID NO: 8 oder SEQ ID NO: 9 dargestellte Nukleinsäure ist, oder eine durch SEQ ID NO:7, SEQ ID NO: 8 oder SEQ ID NO: 9 dargestellte Nukleinsäure ist, in welcher eine oder einige Basen ihrer Sequenz deletiert, substitutiert oder hinzugefügt sind und welche eine DNA-Replikation-initiierende Funktion aufweist.

10. Verfahren gemäß einem der Ansprüche 1 bis 9, wobei die Zielzelle eine in von einem Subjekt erhaltenem Blut enthaltene Zelle ist.

11. Verfahren zum Bestimmen von Charakteristika einer Zelle, umfassend:
Bestimmen der Charakteristika der Zielzelle basierend auf der mittels des Verfahren gemäß einem der Ansprüche 1 bis 10 erhaltenen epigenetischen Information der Zielzelle.

12. Verfahren gemäß Anspruch 11, wobei die Charakteristika die einer Arzneistoffempfindlichkeit sind.

13. Verfahren zum Bestimmen von Charakteristika einer Zelle, umfassend:
Bestimmen der Charakteristika der Zielzelle basierend auf der mittels des Verfahrens gemäß einem der Ansprüche 1 bis 10 erhaltenen epigenetischen Information der Zielzelle,
das Bestimmen umfassend Bestimmen, ob die Zielzelle eine krebsartige (cancerated) Zelle ist.

14. Selbst-replizierender Vektor zum Erhalten epigenetischer Information über eine Zielzelle, welche eine spezifische Genomsequenz enthält, umfassend:
(a) eine erste Reportergen-Expressionseinheit, beinhaltend:
eine erste Zielnukleinsäuresequenz, enthaltend eine Base mit einer substituierbaren Methylgruppe,
welche eine von einem Grad der Substitution der Methylgruppe in der Base abhängende Promotoraktivität aufweist, und Homologie bezüglich der spezifischen Genomsequenz der Zielzelle aufweist, wobei die Base Guanin und/oder Cytosin ist,
ein downstream der ersten Zielnukleinsäuresequenz funktionell verknüpftes erstes Reportergen, welches ein erstes Reporterprotein codiert, und welches durch Aktivierung der ersten
Zielnukleinsäuresequenz exprimiert wird, und
eine downstream des Reportergens funktionell verknüpfte erste Transkriptionsterminations-Signalsequenz;
(b) eine zweite Reportergen-Expressionseinheit, beinhaltend:
die zweite Zielnukleinsäuresequenz, enthaltend eine Base mit einer substituierbaren Methylgruppe,
welche eine von einem Grad der Substitution der Methylgruppe der Base abhängende Promotoraktivität aufweist, und der spezifischen Genomsequenz der Zielzelle entspricht oder Homologie dazu aufweist,
wobei die Base Guanin und/oder Cytosin ist,
ein downstream der zweiten Zielnukleinsäuresequenz funktionell verknüpftes zweites Reportergen,
welches ein zweites Reporterprotein codiert, und
welches durch Aktivierung der zweiten Zielnukleinsäuresequenz exprimiert wird,
wobei das erste und das zweite Reporterprotein unterschiedlich voneinander sind, und
eine downstream des Reportergens funktionell verknüpfte zweite Transkriptionsterminations-Signalsequenz,
wobei die zweite Reportergen-Expressionseinheit mit einem Downstream-Abschnitt der ersten Reportergen-Expressionseinheit verknüpft ist, und
Methylierungszustände der ersten und der zweiten Zielnukleinsäuresequenzen unterschiedlich voneinander sind; und
(c) eine Replikationsinitiatorsequenz, welche auf der Nukleinsäure vorliegt, auf welcher die erste Reportergen-Expressionseinheit vorliegt.

15. Vektor gemäß Anspruch 14, dadurch charakterisiert, dass das erste und das zweite Reportergen unabhängig voneinander ausgewählt werden aus der Gruppe bestehend aus einem Luciferase-Gen, einem β-Galactosidase-Gen, einem Stickstoffmonoxid-Synthase-Gen, einem Xanthin-Oxidase-Gen, einem blau-fluoreszierendes-Protein-Gen, einem grün-fluoreszierendes-Protein-Gen, einem rot-fluoreszierendes-Protein-Gen und einem Schwermetall-bindendes-Protein-Gen.

16. Assay-Kit, umfassend:
einen selbst-replizierenden Vektor gemäß Anspruch 14;
und einen den selbst-replizierenden Vektor enthaltenden Behälter.

## Revendications

1. Procédé d'obtention d'informations épigénétiques à propos d'une cellule sujet contenant une séquence de génome spécifique, comprenant:
l'introduction d'un vecteur pouvant se répliquer dans la cellule sujet, où le vecteur pouvant se répliquer comprend:
(a) une première unité d'expression de gène rapporteur contenant:
une première séquence d'acide nucléique cible contenant une base avec un groupe méthyle substituable, ayant une activité de promoteur dépendant d'un degré de substitution du groupe méthyle de la base, et correspondant à ou ayant une homologie avec la séquence de génome spécifique de la cellule sujet, où la base est une cytosine et/ou une guanine,
un premier gène rapporteur codant pour une première protéine de rapporteur lié de manière fonctionnelle en aval de la première séquence d'acide nucléique cible et qui est exprimé par l'activation de la première séquence d'acide nucléique cible, et
une première séquence signal de terminaison de transcription liée de manière fonctionnelle en aval du gène rapporteur;
(b) une deuxième unité d'expression de gène rapporteur contenant:
une deuxième séquence d'acide nucléique cible contenant une base avec un groupe méthyle substituable, ayant une activité de promoteur dépendant d'un degré de substitution du groupe méthyle de la base, et correspondant à ou ayant une homologie avec la séquence de génome spécifique de la cellule sujet, où la base est une guanine et/ou une cytosine,
un deuxième gène rapporteur codant pour une deuxième protéine de rapporteur lié de manière fonctionnelle en aval de la deuxième séquence d'acide nucléique cible et qui est exprimé par l'activation de la deuxième séquence d'acide nucléique cible, où les première et deuxième protéines de rapporteurs sont différentes l'une de l'autre, et
une deuxième séquence signal de terminaison de transcription liée de manière fonctionnelle en aval du gène rapporteur,
où la deuxième unité d'expression de gène rapporteur est liée à un côté en aval de la première unité d'expression de gène rapporteur et les états de méthylation des première et deuxième séquences d'acides nucléiques cibles sont différents l'un de l'autre; et
(c) une séquence d'initiateur de réplication présente sur le même acide nucléique que la première unité d'expression de gène rapporteur,
où le vecteur pouvant se répliquer est capable de transcrire les états de méthylation de la séquence de génome spécifique sur les première et deuxième séquences d'acides nucléiques cibles pendant l'autoréplication du vecteur pouvant se répliquer, par la substitution du groupe méthyle des première et deuxième séquences d'acides nucléiques cibles, et
où le vecteur pouvant se répliquer est capable de produire les première et deuxième protéines de rapporteurs en tant que premier et deuxième signaux respectivement dans la cellule sujet dépendant des états de substitution des groupes méthyles des première et deuxième séquences d'acides nucléiques cibles, respectivement;
la culture de la cellule sujet pour promouvoir l'autoréplication du vecteur pouvant se répliquer dans la cellule sujet;
la détection des premier et deuxième signaux et/ou la mesure des quantités d'expression des première et deuxième protéines de rapporteurs; et
l'obtention des informations épigénétiques à propos de la cellule sujet par la comparaison des quantités d'expression des première et deuxième protéines de rapporteurs.

2. Procédé selon la revendication 1, **caractérisé en ce que**, pendant la culture de la cellule sujet, une protéine d'initiateur de réplication est exprimée dans la cellule sujet et la protéine d'initiateur de réplication active la séquence d'initiateur de réplication.

3. Procédé selon la revendication 2, **caractérisé par** le fait de comprendre en outre:
l'introduction d'un vecteur d'expression de gène de protéine d'initiateur de réplication dans le noyau de la cellule sujet,
où le vecteur d'expression de gène de protéine d'initiateur de réplication contient une unité de protéine d'initiateur de réplication qui exprime la protéine d'initiateur de réplication.

4. Procédé selon la revendication 2, **caractérisé en ce que** le vecteur pouvant se répliquer comprend en outre une unité de protéine d'initiateur de réplication qui exprime la protéine d'initiateur de réplication.

5. Procédé selon la revendication 2, **caractérisé en ce que** le vecteur pouvant se répliquer comprend en outre:
une séquence IRES liée de manière fonctionnelle en aval de la première unité d'expression de gène rapporteur,
une séquence codant pour la protéine d'initiateur de réplication, liée de manière fonctionnelle en aval de la séquence IRES, et
une séquence d'initiateur de réplication,
où la séquence signal de terminaison de transcription est liée de manière fonctionnelle en aval de la séquence codant pour la protéine d'initiateur de réplication et la séquence d'initiateur de réplication est liée de manière fonctionnelle en aval de la séquence signal de terminaison de transcription.

6. Procédé selon la revendication 3 ou 4, **caractérisé en ce que** l'unité de protéine d'initiateur de réplication contient une séquence codant pour la protéine d'initiateur de réplication et un promoteur exprimé constitutivement lié de manière fonctionnelle en amont de la séquence codant pour la protéine d'initiateur de réplication.

7. Procédé selon l'une des revendications 1 à 6, **caractérisé en ce que** les premier et deuxième gènes rapporteurs sont indépendamment choisis dans le groupe constitué de: un gène de luciférase, un gène de β-galactosidase, un gène de monoxyde nitrique synthase, un gène de xanthine oxydase, un gène de protéine de fluorescence bleue, un gène de protéine de fluorescence verte, un gène de protéine de fluorescence rouge et un gène de protéine de liaison de métal lourd.

8. Procédé selon l'une des revendications 6 ou 7, **caractérisé en ce qu'**une combinaison du gène codant pour la protéine d'initiateur de réplication et de la séquence d'initiateur de réplication est choisie parmi les paragraphes (1), (2) et (3) suivants:
(1) le gène codant pour la protéine d'initiateur de réplication est un gène d'antigènes T de grande taille de virus simien 40 et la séquence d'initiateur de réplication est une séquence d'initiateur de réplication provenant du virus simien 40;
(2) le gène codant pour la protéine d'initiateur de réplication est un gène EBNA-1 de virus d'Epstein-Barr et la séquence d'initiateur de réplication est une séquence d'initiateur de réplication provenant du virus d'Epstein-Barr; et
(3) le gène codant pour la protéine d'initiateur de réplication est un gène d'antigènes T de grande taille de virus de polyome de souris et la séquence d'initiateur de réplication est une séquence d'initiateur de réplication provenant du virus de polyome de souris.

9. Procédé selon la revendication 8, dans lequel le gène d'antigènes T de grande taille est un acide nucléique représenté par la SEQ ID NO: 7, la SEQ ID NO: 8 ou la SEQ ID NO: 9, ou un acide nucléique représenté par la SEQ ID NO: 7, la SEQ ID NO: 8 ou la SEQ ID NO: 9 dans lequel une ou plusieurs bases de sa séquence sont délétées, substituées ou ajoutées et ayant une fonction d'initiation de réplication d'ADN.

10. Procédé selon l'une des revendications 1 à 9, dans lequel la cellule sujet est une cellule contenue dans du sang obtenu à partir d'un sujet.

11. Procédé de détermination des caractéristiques d'une cellule, comprenant:
la détermination des caractéristiques de la cellule sujet sur la base des informations épigénétiques de la cellule sujet obtenue par le procédé selon l'une des revendications 1 à 10.

12. Procédé selon la revendication 11, dans lequel les caractéristiques sont parmi une sensibilité à un médicament.

13. Procédé de détermination des caractéristiques d'une cellule, comprenant: la détermination des caractéristiques de la cellule sujet sur la base des informations épigénétiques de la cellule sujet obtenue par le procédé selon l'une des revendications 1 à 10,
la détermination comprenant la détermination si la cellule sujet est une cellule cancéreuse.

14. Vecteur pouvant se répliquer pour l'obtention d'informations épigénétiques à propos d'une cellule sujet contenant une séquence de génome spécifique, comprenant:
(a) une première unité d'expression de gène rapporteur contenant:
une première séquence d'acide nucléique cible contenant une base avec un groupe méthyle substituable, ayant une activité de promoteur dépendant d'un degré de substitution du groupe méthyle dans la base et ayant une homologie par rapport à la séquence de génome spécifique de la cellule sujet, où la base est une guanine et/ou une cytosine,
un premier gène rapporteur codant pour une première protéine de rapporteur lié de manière fonctionnelle en aval de la première séquence d'acide nucléique cible et exprimé par l'activation de la première séquence d'acide nucléique cible, et
une première séquence signal de terminaison de transcription liée de manière fonctionnelle en aval du gène rapporteur;
(b) une deuxième unité d'expression de gène rapporteur contenant:
la deuxième séquence d'acide nucléique cible contenant une base avec un groupe méthyle substituable, ayant une activité de promoteur dépendant d'un degré de substitution du groupe méthyle de la base, et correspondant à ou ayant une homologie avec la séquence de génome spécifique de la cellule sujet, où la base est une guanine et/ou une cytosine,
un deuxième gène rapporteur codant pour une deuxième protéine de rapporteur lié de manière fonctionnelle en aval de la deuxième séquence d'acide nucléique cible et qui est exprimé par l'activation de la deuxième séquence d'acide nucléique cible, où les première et deuxième protéines de rapporteurs sont différentes l'une de l'autre, et
une deuxième séquence signal de terminaison de transcription liée de manière fonctionnelle en aval du gène rapporteur,
où la deuxième unité d'expression de gène rapporteur est liée à un côté en aval de la première unité d'expression de gène rapporteur et les états de méthylation des première et deuxième séquences d'acides nucléiques cibles sont différents l'un de l'autre; et
(c) une séquence d'initiateur de réplication présente sur l'acide nucléique sur lequel la première unité d'expression de gène rapporteur est présente.

15. Vecteur selon la revendication 14, **caractérisé en ce que** les premier et deuxième gènes rapporteurs sont indépendamment choisis dans le groupe constitué de: un gène de luciférase, un gène de β-galactosidase, un gène de monoxyde nitrique synthase, un gène de xanthine oxydase, un gène de protéine de fluorescence bleue, un gène de protéine de fluorescence verte, un gène de protéine de fluorescence rouge et un gène de protéine de liaison de métal lourd.

16. Nécessaire de dosage comprenant:
un vecteur pouvant se répliquer selon la revendication 14; et
un récipient contenant le vecteur pouvant se répliquer.
